# EUROPEAN PATENT SPECIFICATION

(11) **EP 3 866 786 B1**
(45) Date of publication and mention of the grant of the patent: **18.09.2024**
(21) Application number: 19873345.3
(22) Date of filing: 14.10.2019
(51) Int. Cl.: A61K 31/404, A61K 9/107, A61K 47/40, A61P 3/00, A61P 3/04, A61P 3/10, A23L 33/10

(54) **COMPOSITIONS AND METHODS FOR SUPPRESSING AND/OR TREATING METABOLIC DISEASES AND/OR A CLINICAL CONDITION THEREOF**
ZUSAMMENSETZUNGEN UND VERFAHREN ZUR UNTERDRÜCKUNG UND/ODER BEHANDLUNG VON STOFFWECHSELERKRANKUNGEN UND/ODER EINES KLINISCHEN ZUSTANDS DAVON
COMPOSITIONS ET PROCÉDÉS DE SUPPRESSION ET/OU DE TRAITEMENT DE MALADIES MÉTABOLIQUES ET/OU D'UNE AFFECTION CLINIQUE ASSOCIÉE

(30) Priority: 15.10.2018 KR 20180122511; 01.02.2019 US 201962799912 P; 20.09.2019 US 201962903068 P
(43) Date of publication of application: 25.08.2021
(73) Proprietor: CK Regeon Inc., Seodaemoon-gu Seoul 03722 (KR)
(72) Inventor: CHOI, Kang-Yell, Sajik Dong, Jong Ro Gu Seoul Seoul (KR); SEO, Seol Hwa, Sajik Dong, Jong Ro Gu Seoul Seoul (KR); KIM, Eunhwan, Sajik Dong, Jong Ro Gu Seoul Seoul (KR)
(74) Representative: Leonard, Thomas Charles
(86) International application number: PCT/IB2019/058747
(87) International publication number: WO 2020/079569

(56) References cited:
- WO-A1-2005/041954
- KR-A- 20180 119 941
- KR-B1- 101 855 423
- CHOI O M ET AL: "The small molecule indirubin-3'-oxime activates Wnt/[beta]-catenin signaling and inhibits adipocyte differentiation and obesity", INTERNATIONAL JOURNAL OF OBESITY, vol. 38, no. 8, 15 November 2013 (2013-11-15), London, pages 1044 - 1052, XP055928526, ISSN: 0307-0565, Retrieved from the Internet <URL:http://www.nature.com/articles/ijo2013209> DOI: 10.1038/ijo.2013.209
- WAGMAN A S ET AL: "DISCOVERY AND DEVELOPMENT OF GSK3 INHIBITORS FOR THE TREATMENT OF TYPE 2 DIABETES", CURRENT PHARMACEUTICAL DESIGN, BENTHAM SCIENCE PUBLISHERS, NL, vol. 10, no. 10, 1 January 2004 (2004-01-01), pages 1105 - 1137, XP009058198, ISSN: 1381-6128, DOI: 10.2174/1381612043452668
- NISHA CHALUVEELAVEEDU MURLEEDHARAN ET AL: "Docking and ADMET prediction of few GSK-3 inhibitors divulges 6-bromoindirubin-3-oxime as a potential inhibitor", JOURNAL OF MOLECULAR GRAPHICS AND MODELLING, ELSEVIER SCIENCE, NEW YORK, NY, US, vol. 65, 4 March 2016 (2016-03-04), pages 100 - 107, XP029468080, ISSN: 1093-3263, DOI: 10.1016/J.JMGM.2016.03.001
- CHENG XINLAI ET AL: "Identification of a Water-Soluble Indirubin Derivative as Potent Inhibitor of Insulin-like Growth Factor 1 Receptor through Structural Modification of the Parent Natural Molecule", JOURNAL OF MEDICINAL CHEMISTRY, vol. 60, no. 12, 22 June 2017 (2017-06-22), US, pages 4949 - 4962, XP055800179, ISSN: 0022-2623, Retrieved from the Internet <URL:https://pubs.acs.org/doi/pdf/10.1021/acs.jmedchem.7b00324> DOI: 10.1021/acs.jmedchem.7b00324
- KOSUGE YASUHIRO ET AL: "Indirubin derivatives protect against endoplasmic reticulum stress-induced cytotoxicity and down-regulate CHOP levels in HT22 cells", BIOORGANIC & MEDICINAL CHEMISTRY LETTERS, vol. 27, no. 23, 2017, pages 5122 - 5125, XP085261317, ISSN: 0960-894X, DOI: 10.1016/J.BMCL.2017.10.069
- ZHANG N ET AL: "3D QSAR for GSK-3@b inhibition by indirubin analogues", EUROPEAN JOURNAL OF MEDICINAL CHEMISTRY, ELSEVIER, AMSTERDAM, NL, vol. 41, no. 3, 1 March 2006 (2006-03-01), pages 373 - 378, XP024993814, ISSN: 0223-5234, [retrieved on 20060301], DOI: 10.1016/J.EJMECH.2005.10.018
- BEGUM, J. ET AL.: "An evaluation of indirubin analogues as phosphorylase kinase inhibitors", JOURNAL OF MOLECULAR GRAPHICS AND MODELLING, vol. 61, 2015, pages 231 - 242, XP029267772, DOI: 10.1016/j.jmgm.2015.07.010
- POLYCHRONOPOULOS, P. ET AL.: "Structural basis for the synthesis of indirubins as potent and selective inhibitors of glycogen synthase kinase-3 and cyclin-dependent kinases", JOURNAL OF MEDICINAL CHEMISTRY, vol. 47, no. 4, 2004, pages 935 - 946, XP002453468, DOI: 10.1021/jm031016d
- MEIJER, L. ET AL.: "GSK-3-selective inhibitors derived from Tyrian purple in dirubins", CHEMISTRY & BIOLOGY, vol. 10, no. 12, 2003, pages 1255 - 1266, XP002444147, DOI: 10.1016/j.chembiol.2003.11.010

## Description

### FIELD

Various aspects and embodiments disclosed herein relate generally to compositions for use in treating or preventing conditions/diseases associated with a metabolic disease. Embodiments include compositions for use in treating the conditions/diseases, comprising providing to a subject at least one therapeutically effective dose of a composition disclosed herein. Other disclosures herein include methods for altering and/or suppressing the activity of the CXXC5-DVL interface in a subject.

### BACKGROUND

Metabolic diseases possess multiplex pathological status associated with obesity, atherogenic dyslipidemia, insulin resistance, and increased risk of developing type 2 diabetes mellitus (T2DM). Metabolic diseases have long been considered as incurable, chronic conditions that require glycemic control in peripheral insulin target tissues. Metabolic diseases result from a variety of pathological conditions in obese patients with excess abnormal adipose tissues. Dysregulated adipose tissue functions lie at the root of systemic metabolic problems related to the aberrant regulation of various hormones, cytokines, and adipokines, leading to low-grade inflammation and metabolic disorders.

Non-alcoholic steatohepatitis (NASH) is an advanced form of non-alcoholic fatty liver disease (NAFLD). NAFLD is caused by accumulation of fat in the liver. When the accumulation of fat causes inflammation and liver damage, NAFLD develops into NASH, which can further lead to scarring of the liver. These changes can stimulate hepatic stellate cells, resulting in fibrosis. If advanced, NASH can cause cirrhosis and portal hypertension. NASH is diagnosed most often in patients between 40 years and 60 years but can occur in all age groups. Many affected NASH patients had also been reported to have obesity, type 2 diabetes mellitus, glucose intolerance, dyslipidemia, and/or metabolic diseases. While there is no standard for treating NASH, lifestyle changes have been shown to affect its progression. This can include losing weight, maintaining a healthy diet, or addressing underlying conditions such as hypothyroidism and diabetes.

Some studies have reported the involvement of Wnt/β-catenin signaling in obesity, diabetes, and potentially, NASH. CXXC finger protein 5 (CXXC5) is a negative regulator of Wnt/β-catenin signaling, functioning via interaction with PDZ domain of dishevelled (DVL) in the cytosol. Inhibition of the CXXC5-DVL interaction improved several pathophysiological phenotypes involving Wnt/β-catenin signaling including osteoporosis, cutaneous wounds, and hair loss through activation of the Wnt/β-catenin. Due to the complexity of the processes involving the regulation of metabolic diseases, development of a new treatment regimen(s) is much needed.

### SUMMARY

Given CXXC5's role as a negative regulator of Wnt/β-catenin signaling, it is an attractive target for the development of compounds that can interfere with its activity. Some aspects of the instant disclosure include compounds that interfere with CXXC5-DVL interface and compositions for use in methods of using the same to influence and/or treat obesity, diabetes, and/or NASH in a subject.

The invention provides a composition comprising at least one compound and/or a pharmaceutically acceptable hydrate, salt, or carrier thereof, wherein the compound is: 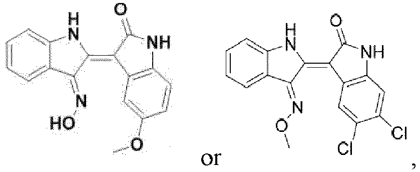 for use in a method of treating or preventing a metabolic disease in a subject.

In some embodiments the subject has upregulated expression of CXXC5. In some embodiments the subject exhibits abnormal lipid profile and/or blood glucose levels. In some embodiments the metabolic disease is obesity, diabetes, non-alcoholic fatty liver disease (NAFLD), and/or non-alcoholic steatohepatitis (NASH). In some embodiments the subject is a human or an animal. In some embodiments the composition is an oral preparation or intravenous preparation.

The technical information set out below may in some respects go beyond the scope of the present invention, which is defined by the appended claims. The additional technical information is provided to place the present invention in a broader technical context and to illustrate possible related technical developments. References herein to methods of treatment of the human or animal body are to be understood as references to medicaments for use in a method of treatment.

Embodiments of the instant application relate to compositions for use in methods for treating a metabolic disease. In certain embodiments, the uses disclosed herein include suppression of one or more side effects of a therapeutic regime. Other embodiments relate to compositions for use in methods for treating a subject diagnosed with a disease or having a condition contributed to obesity, diabetes, and NASH, due at least in part by the accumulation of fat and inflammation in the liver of a subject.

Compositions disclosed herein comprise at least one agent that may act by inhibiting the CXXC5-DVL interface - the interface between CXXC finger protein 5 (CXXC5) and dishevelled (DVL) - in a subject. In some disclosures, at least one agent that inhibits CXXC5-DVL interface comprises at least one agent that binds to the PDZ domain of dishevelled (DVL) and/or the DVL binding motif, and/or at least one GSK3β inhibitor, or a combination thereof.

A first disclosure includes a compound of Formula I, 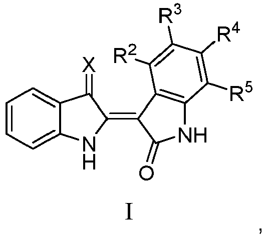
wherein X is O or N optionally substituted with R¹;
R¹ is hydrogen, hydroxy, alkyl, alkenyl, or an alkoxy optionally substituted with alkyl, alkenyl, haloalkyl, aryl, or benzyl; or R¹ is hydrogen, alkyl, alkenyl, or an alkoxy substituted with butyl, alkenyl, haloalkyl, aryl, or benzyl;
R² is hydrogen, nitro, halogen, alkyl, alkenyl, haloalkyl, alkoxy, haloalkoxy, or a carboxy.
R³ is hydrogen, nitro, halogen, alkyl, alkenyl, haloalkyl, alkoxy, haloalkoxy, or a carboxy; or R³ is hydrogen, fluorine, iodine, astatine, alkyl, alkenyl, haloalkyl, OCF₃, ethoxy, propyloxy, butyloxy, haloalkoxy, or a carboxy, and/or wherein when R³ is bromine or chlorine, R⁴ is not hydrogen; and/or wherein when R³ is chlorine, R⁴ is not chlorine.
R⁴ is hydrogen, nitro, halogen, alkyl, alkenyl, haloalkyl, alkoxy, haloalkoxy, or a carboxy; or R⁴ is hydrogen, nitro, fluorine, bromine, iodine, astatine, alkyl, alkenyl, haloalkyl, alkoxy, haloalkoxy, or a carboxy; and/or wherein when R⁴ is chlorine, R³ is not hydrogen or nitro.
R⁵ is hydrogen, nitro, halogen, alkyl, alkenyl, haloalkyl, alkoxy, haloalkoxy, or a carboxy.

A second disclosure includes the compound according to the compound of the first disclosure, wherein Xis O.

A third disclosure includes the compound according to the compound according to the compound of the first disclosure, wherein Xis N and R¹ is hydroxy or alkoxy optionally substituted with alkyl, alkenyl, haloalkyl, aryl, or benzyl, or R¹ is hydrogen, alkyl, alkenyl, or an alkoxy substituted with butyl, alkenyl, haloalkyl, aryl, or benzyl.

A fourth disclosure includes the compound according to the compound according to any one of the first to the third disclosures, wherein R¹ is alkoxy optionally substituted with alkyl, alkenyl, haloalkyl, aryl, or benzyl.

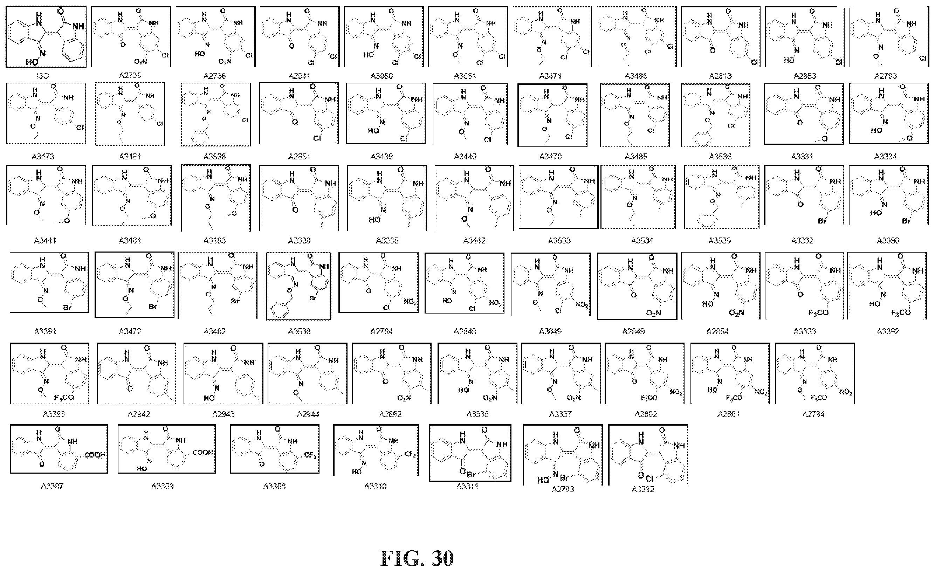

A fifth disclosure includes the compound according to the compound according to any one of the first to the fourth disclosures, wherein the compound is any one of the compounds disclosed in **FIG. 30****,** **FIG. 48****,** **FIG. 49****, Table 4, Table 5, and/or Table 6.**

A sixth disclosure includes the compound according to any one of the first to the fifth disclosures, wherein the compound comprises at least one compound comprising

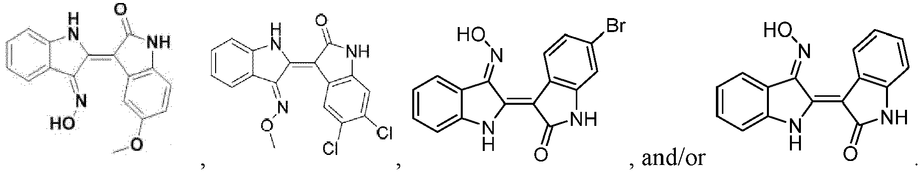

A seventh disclosure includes a compound of Formula II, 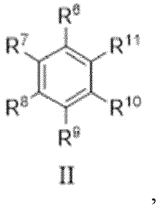
wherein R⁶, R⁷, R⁸, R⁹, and R¹⁰ are independently hydrogen, halogen, hydroxy, alkyl, haloalkyl, alkoxy, or 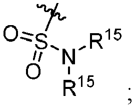
R¹¹ is C₁-C₆ alkyl, C₁-C₆ alkenyl, N, diimide, each substituted with R¹², 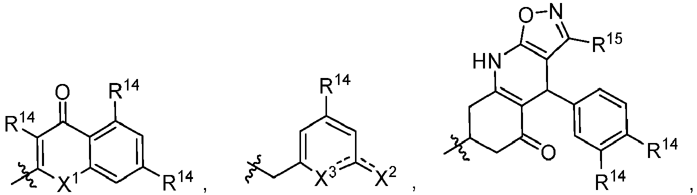 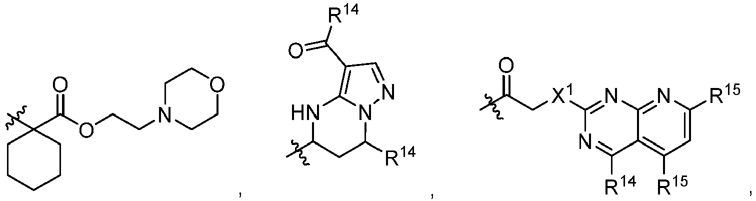 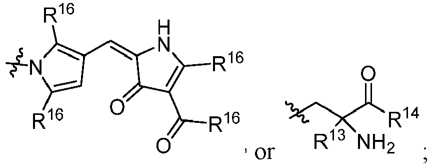 or
R¹¹ is 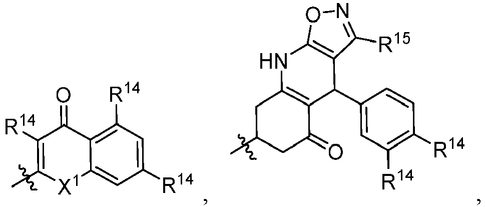 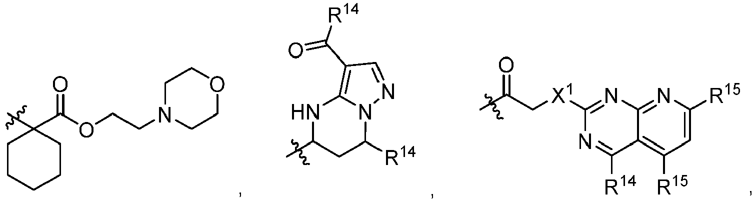 or 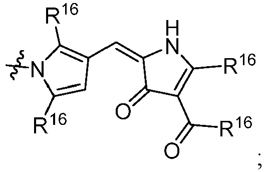
R¹² is 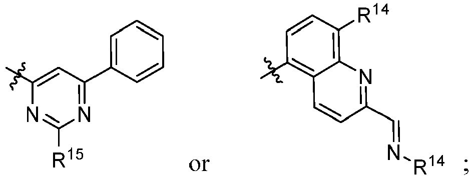
R¹³ is hydrogen or an alkyl optionally substituted with hydrogen, halogen, hydroxy, alkoxy, or haloalkyl;
each R¹⁴, each R¹⁵, and each R¹⁶ are independently hydrogen; halogen; haloalkyl optionally substituted with hydrogen, halogen, hydroxy, or alkoxy; alkyl optionally substituted with hydrogen, halogen, hydroxy, alkoxy, or haloalkyl; alkoxy optionally substituted with hydrogen, halogen, hydroxy, alkoxy, or haloalkyl; alkenyl optionally substituted with hydrogen, halogen, hydroxy, alkoxy, or haloalkyl; or alkynyl optionally substituted with hydrogen, halogen, hydroxy, alkoxy, or haloalkyl; and
X¹, X² and X³ are independently carbon, nitrogen, oxygen, or sulfur.

An eighth disclosure includes the compound according to the seventh disclosure, wherein R¹¹ is N or diimide, each substituted with R¹².

A ninth disclosure includes the compound according to any one of the seventh to the eighth disclosures, wherein R¹¹ is 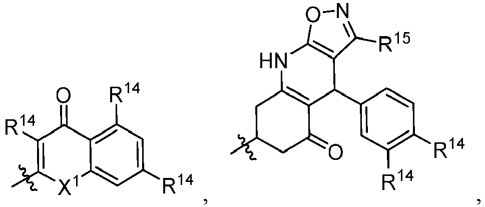 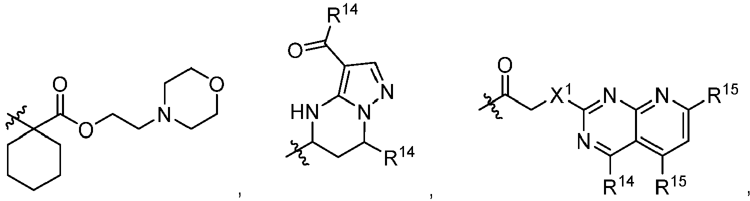 or

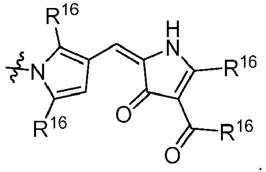

A tenth disclosure includes the compound according to any one of the seventh to the ninth disclosures, wherein the compound is 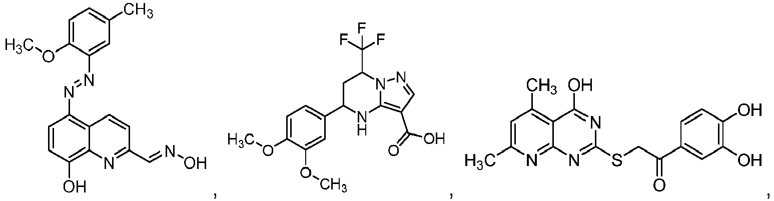 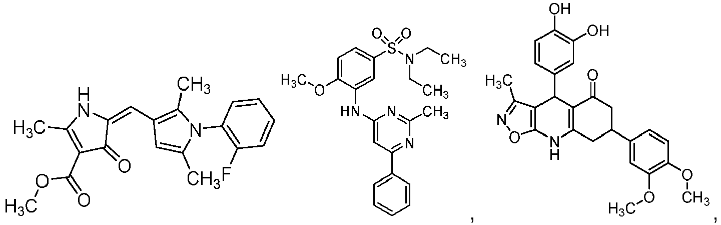 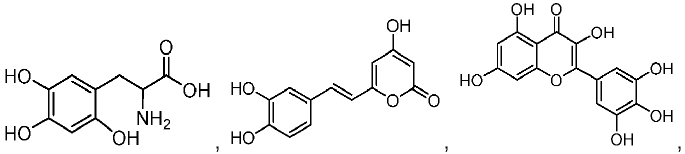 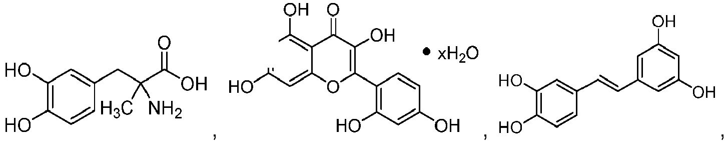 or

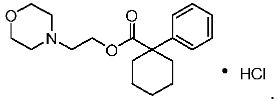

An eleventh disclosure includes a compound of Formula III, 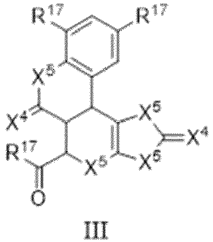
wherein each R¹⁷ is independently hydrogen; halogen; haloalkyl optionally substituted with hydrogen, halogen, hydroxy, or alkoxy; alkyl optionally substituted with hydrogen, halogen, hydroxy, alkoxy, or haloalkyl; alkoxy optionally substituted with hydrogen, halogen, hydroxy, alkoxy, or haloalkyl; alkenyl optionally substituted with hydrogen, halogen, hydroxy, alkoxy, or haloalkyl; or alkynyl optionally substituted with hydrogen, halogen, hydroxy, alkoxy, or haloalkyl;
X⁴ and X⁵ are independently nitrogen, oxygen, or sulfur.

A twelfth disclosure includes the compound according to the eleventh disclosure, wherein each R¹⁷ is independently halogen or hydroxy.

A thirteenth disclosure includes the compound according to any one of the eleventh to the twelfth disclosures, wherein the compound is

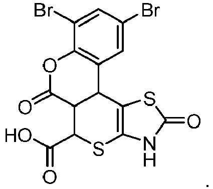

A fourteenth disclosure includes a compound of Formula IV, 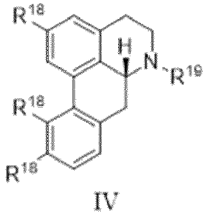 wherein each R¹⁸ and R¹⁹ are independently hydrogen; hydroxy; halogen; haloalkyl optionally substituted with hydrogen, halogen, hydroxy, or alkoxy; alkyl optionally substituted with hydrogen, halogen, hydroxy, alkoxy, or haloalkyl; alkoxy optionally substituted with hydrogen, halogen, hydroxy, alkoxy, or haloalkyl; alkenyl optionally substituted with hydrogen, halogen, hydroxy, alkoxy, or haloalkyl; or alkynyl optionally substituted with hydrogen, halogen, hydroxy, alkoxy, or haloalkyl.

A fifteenth disclosure includes the compound according to the fourteenth disclosure, wherein R¹⁹ is alkyl optionally substituted with hydrogen, halogen, hydroxy, alkoxy, or haloalkyl.

A sixteenth disclosure includes the compound according to any one of fourteenth to the fifteenth disclosures, wherein each R¹⁸ is independently hydrogen, hydroxy, halogen, alkoxy, alkyl, alkenyl, or haloalkyl.

A seventeenth disclosure includes the compound according to any one of fourteenth to the sixteenth disclosures, wherein the compound is

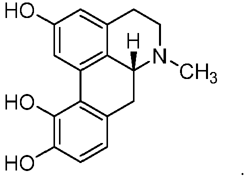

An eighteenth disclosure includes a compound of Formula V, 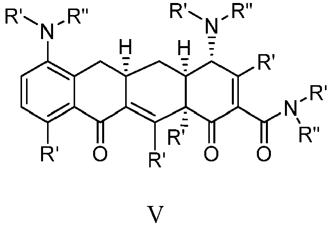 wherein each R' and each R" are independently hydrogen; halogen; haloalkyl optionally substituted with hydrogen, halogen, hydroxy, or alkoxy; alkyl optionally substituted with hydrogen, halogen, hydroxy, alkoxy, or haloalkyl; alkoxy optionally substituted with hydrogen, halogen, hydroxy, alkoxy, or haloalkyl; alkenyl optionally substituted with hydrogen, halogen, hydroxy, alkoxy, or haloalkyl; or alkynyl optionally substituted with hydrogen, halogen, hydroxy, alkoxy, or haloalkyl.

A nineteenth disclosure includes the compound according to the eighteenth disclosure, wherein each R' and each R" are independently hydrogen, halogen, hydroxy, alkyl, alkenyl, alkoxy, or haloalkyl.

A twentieth disclosure includes the compound according to any one of the eighteenth to the nineteenth disclosures, wherein the compound is

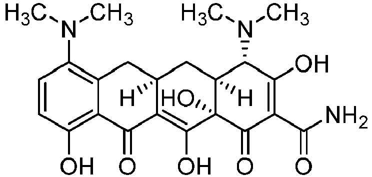

A twenty first disclosure includes a compound of Formula VI, 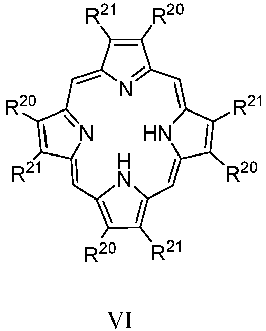 wherein each R²⁰ and each R²¹ are independently hydrogen; halogen; haloalkyl optionally substituted with hydrogen, halogen, hydroxy, or alkoxy; alkyl optionally substituted with hydrogen, halogen, hydroxy, alkoxy, haloalkyl, or a carbonyl, optionally substituted with hydrogen, halogen, alkyl, hydroxy, alkoxy, or haloalkyl; alkoxy optionally substituted with hydrogen, halogen, hydroxy, alkoxy, or haloalkyl; alkenyl optionally substituted with hydrogen, halogen, hydroxy, alkoxy, or haloalkyl; or alkynyl optionally substituted with hydrogen, halogen, hydroxy, alkoxy, or haloalkyl.

A twenty second disclosure includes the compound according to the twenty first disclosure, wherein each R²⁰ and each R²¹ are independently hydrogen, halogen, hydroxy, alkyl, alkenyl, alkoxy, carbonyl, carboxyl, or haloalkyl.

A twenty third disclosure includes the compound according to any one of the twenty first to the twenty second disclosures, wherein the compound is

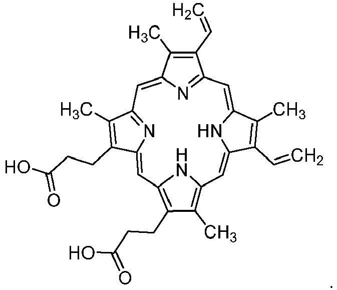

A twenty fourth disclosure includes at least one of the compounds according to any one of the first to the twenty third disclosures, wherein the compound inhibits or reduces the CXXC5-DVL interface, the interaction between CXXC5 and DVL, and/or the activity of CXXC5 and/or the CXXC5-DVL interface.

A twenty fifth disclosure includes at least one of the compounds according to any one of the preceding disclosures, wherein the compound inhibits or reduces the interaction between CXXC5 and DVL by directly competing with CXXC5 for a binding site in DVL, by directly binding to DVL, and/or by directly binding to the PDZ domain of DVL.

A twenty sixth disclosure includes a pharmaceutical composition comprising at least one compound according to any one of the first to the twenty fifth disclosures and/or a pharmaceutically acceptable hydrate, salt, metabolite, or carrier thereof.

The uses disclosed herein include uses in methods of treating at least one clinical condition, which is a metabolic disease, comprising administering to a subject at least one therapeutically effective dose of any of the compositions disclosed herein. The subject can be diagnosed with a metabolic disease. In certain embodiments, the uses disclosed herein further comprise administering to the subject a plurality of therapeutically effective doses of any of the compositions disclosed herein.

A twenty seventh disclosure includes a method of treating a metabolic disease or a similar condition, comprising: administering to a subject at least one therapeutically effective dose of at least one agent that inhibits or reduces the CXXC5-DVL interface the interaction between CXXC5 and DVL, and/or the activity of the CXXC5 and/or the CXXC5-DVL interface; and/or administering to a subject at least one therapeutically effective dose of at least one agent comprising at least one compound according to any one of the first to the twenty fifth disclosures and/or at least one composition according to the twenty sixth disclosure.

A twenty eighth disclosure includes the method according to the twenty seventh disclosure, further comprising: detecting an upregulated expression of CXXC5 in the subject. Consistent with these disclosures, the upregulated expression of CXXC5 can be detected in the liver, adipose tissue, and/or pancreas of the subject. The present invention extends at least to embodiments wherein the subject has upregulated expression of CXXC5.

A twenty ninth disclosure includes the method according to any one of the twenty seventh to the twenty eighth disclosures, further comprising: identifying the subject at risk for a metabolic disease or a similar condition. The present invention extends at least to embodiments wherein the method comprises identifying the subject at risk for a metabolic disease or a similar condition.

A thirtieth disclosure includes at least one of the methods according to any one of the twenty seventh to the twenty ninth disclosures, wherein the metabolic disease or a similar condition includes at least one condition selected from, or comprising, metabolic disorder, metabolic syndrome, systemic inflammation, adipose tissue inflammation, adipocyte hypertrophy, β-cell dysfunction, obesity, high blood pressure, high blood sugar, high serum triglycerides, hyperuricemia, fatty liver, polycystic ovarian syndrome, erectile dysfunction, acanthosis nigricans, type 2 diabetes mellitus, insulin resistance, hypoadiponectinemia, cirrhosis, portal hypertension, cardiovascular diseases, coronary artery disease, lipodystrophy, dyslipidemia, steatosis, hepatic steatosis, non-alcoholic fatty liver disease (NAFLD), and/or non-alcoholic steatohepatitis (NASH). The invention relates to particular compounds for use in the treatment of metabolic diseases, for example obesity, diabetes, non-alcoholic fatty liver disease (NAFLD) and/or non-alcoholic steatohepatitis (NASH).

A thirty first disclosure includes at least one of the methods according to any one of the twenty seventh to the thirtieth disclosures, wherein the subject exhibits abnormal lipid profile, insulin resistance, and/or blood glucose levels. The present invention extends at least to embodiments wherein the subject exhibits an abnormal lipid profile and/or blood glucose levels.

A thirty second disclosure includes at least one of the methods according to any one of the twenty seventh to the thirty first disclosures, wherein the subject is diagnosed with obesity, diabetes, non-alcoholic fatty liver disease (NAFLD), and/or non-alcoholic steatohepatitis (NASH). The present invention extends at least to embodiments wherein the subject is diagnosed with obesity, diabetes, non-alcoholic fatty liver disease (NAFLD), and/or non-alcoholic steatohepatitis (NASH).

A thirty third disclosure includes at least one of the methods according to any one of the twenty seventh to the thirty second disclosures, wherein the at least one agent that inhibits the CXXC5-DVL interface, that inhibits the interaction between CXXC5 and DVL, and/or that inhibits the activity of CXXC5 and/or the CXXC5-DVL interface comprises at least one compound according to any one of the first to the twenty fifth disclosures and/or at least one composition according to the twenty sixth disclosure.

A thirty fourth disclosure includes at least one of the methods according to the thirty third disclosures, wherein the method further includes: administering at least one therapeutically effective dose of at least one additional agent comprising a GSK3β inhibitor, an inhibitor of Wnt/β-catenin pathway, a weight-loss medication, and/or a diabetes medication. Consistent with these disclosures, the at least one additional agent comprises orlistat, lorcaserin, phentermine-topiramate, naltrexone-bupropion, liraglutide, benzphetamine, diethylpropion, sulfonylureas, meglitinides, thiazolidinediones, DPP-4 inhibitors, insulin analog, alpha glucosidase inhibitor, SGLT2 inhibitors, sitagliptin, metformin, rosiglitazone, ocaliva, selonsertib, elafibranol, cenicriviroc, MGL-3196, GR-MD-02, and/or aramchol.

A thirty fifth disclosure includes at least one of the methods according to any one of the twenty seventh to the thirty fourth disclosures, wherein the subject is a human adult, a human child, and/or an animal. The present invention extends at least to embodiments wherein the subject is a human or an animal.

A thirty sixth disclosure includes at least one of the methods according to any one of the twenty seventh to the thirty fifth disclosures, wherein the at least one agent and/or the at least one additional agent is administered orally or intravenously. The present invention extends at least to embodiments wherein the composition is an oral preparation or intravenous preparation.

A thirty seventh disclosure includes at least one of the methods according to any one of the twenty seventh to the thirty sixth disclosures, wherein the therapeutically effective dose of at least one compound according to any one of the first to the twenty fifth disclosures and/or at least one composition according to the twenty sixth disclosure, is on the order of between about 5 mg to about 2000 mg and the dose of the compound is administered to the subject at least once per day. In some disclosures, the therapeutically effective dose of at least one compound according to any one of the first to the twenty fifth disclosures and/or at least one composition according to the twenty sixth disclosure, includes, but is not limited to, on the order of between: about 10 mg to about 1900 mg; about 15 mg to about 1800 mg; about 15 mg to about 1700 mg; about 20 mg to about 1600 mg; about 25 mg to about 1500 mg; about 30 mg to about 1000 mg; about 50 mg to about 1000 mg; about 50 mg to about 800 mg; about 100 mg to about 800 mg; about 300 mg to about 800 mg; about 500 mg to about 800 mg; about 5 mg to about 50 mg; about 1000 mg to about 1700 mg; about 1200 mg to about 1700 mg; about 1500 mg to about 1700 mg; about 10 mg to about 1000 mg; about 10 mg to about 30 mg; about 1500 mg to about 2000 mg; about 100 mg to about 200 mg; about 100 mg to about 150 mg; and/or any combination thereof. Consistent with these disclosures, the therapeutically effective dose of at least one compound according to any one of the first to the twenty fifth disclosures and/or at least one composition according to the twenty sixth disclosure, includes, but not limited to, on the order of between: about 1 mg/m2 to about 1500 mg/m2; about 10 mg/m2 to about 1000 mg/m2; about 20 mg/m2 to about 800 mg/m2; about 10 mg/m2 to about 50 mg/m2; about 800 mg/m2 to about 1200 mg/m2; about 50 mg/m2 to about 500 mg/m2; about 500 mg/m2 to about 1000 mg/m2; about 80 mg/m2 to about 150 mg/m2; about 80 mg/m2 to about 120 mg/m2; and/or any combination thereof.

A thirty eighth disclosure includes at least one of the methods according to any one of the twenty seventh to the thirty sixth disclosures, wherein the therapeutically effective dose of at least one compound according to any one of the first to the twenty fifth disclosures and/or at least one composition according to the twenty sixth disclosure, is on the order of between about 0.01 mg to about 200 mg and the dose of the compound is administered to the subject at least once per day. In some disclosures, the therapeutically effective dose of at least one compound according to any one of the first to the twenty fifth disclosures and/or at least one composition according to the twenty sixth disclosure, includes, but is not limited to, on the order of between: about 0.01 mg to about 150 mg; about 0.01 mg to about 100 mg; about 0.01 mg to about 80 mg; about 0.01 mg to about 60 mg; about 0.05 mg to about 100 mg; about 0.05 mg to about 80 mg; about 0.05 mg to about 50 mg; about 0.1 mg to about 100 mg; about 0.1 mg to about 50 mg; about 0.2 mg to about 100 mg; about 0.2 mg to about 50 mg; about 0.5 mg to about 100 mg; about 0.5 mg to about 50 mg; about 100 mg to about 200 mg; ; about 100 mg to about 150 mg; and/or any combination thereof. In some of these disclosures, the therapeutically effective dose of at least one compound according to any one of the first to the twenty fifth disclosures and/or at least one composition according to the twenty sixth disclosure, includes, but not limited to, on the order of between: about 0.01 mg/m² to about 100 mg/m²; about 0.01 mg/m² to about 80 mg/m²; about 0.01 mg/m² to about 50 mg/m²; about 0.01 mg/m² to about 25 mg/m²; about 0.05 mg/m² to about 100 mg/m²; about 0.05 mg/m² to about 80 mg/m²; about 0.05 mg/m² to about 50 mg/m²; about 80 mg/m² to about 150 mg/m²; about 80 mg/m² to about 120 mg/m²; and/or any combination thereof.

Methods disclosed herein by the present application reduce and/or suppress a side effect of a therapeutic regime, the methods comprising administering to a subject at least one therapeutically effective dose of at least one agent that inhibits or reduces the CXXC5-DVL interface in a subject, and/or administering to a subject at least one therapeutically effective dose of at least one agent comprising at least one compound according to any one of the first to the twenty fifth disclosures and/or at least one composition according to the twenty sixth disclosure; wherein the subject has received at least one therapeutic regime selected from drug therapy, surgical treatment, and/or combinations thereof, and wherein the subject experiences at least one side effect as a consequence of the therapeutic regime. Consistent with these disclosures, side effects can include, but are not limited to, drug-resistance, relapse, inflammation, or any combination thereof.

A thirty ninth disclosure includes a method of detecting one or more metabolic disease markers, comprising: providing a sample of blood, cells, or tissue from a subject suspected of having or known to have a metabolic disease or condition; and detecting upregulation in one or more markers in the sample, wherein the one or more markers comprise CXXC5 and/or β-catenin.

A fortieth disclosure includes the method according to the thirty ninth disclosure, wherein the metabolic disease includes at least one condition selected from, or comprising, metabolic disorder, metabolic syndrome, obesity, insulin resistance, high blood pressure, high blood sugar, high serum triglycerides, hyperuricemia, fatty liver, polycystic ovarian syndrome, erectile dysfunction, acanthosis nigricans, type 2 diabetes mellitus, hypoadiponectinemia, cirrhosis, portal hypertension, cardiovascular diseases, coronary artery disease, lipodystrophy, dyslipidemia, hepatic steatosis, non-alcoholic fatty liver disease (NAFLD), and/or non-alcoholic steatohepatitis (NASH). Consistent with these disclosures, CXXC5 is overexpressed in the liver, adipose tissue, and/or pancreas of the subject at least about 10%, about 20%, about 25%, about 30%, about 40%, about 50%, about 60%, about 70%, about 80%, about 90%, about 100%, about 200%, about 300%, about 400%, about 500%, and/or about 1000%, or any combination thereof, as compared to that of a normal subject known not to have a metabolic disease; and/or CXXC5 is overexpressed in the liver, adipose tissue, and/or pancreas of the subject at least about 1.5 fold, about 2 fold, about 2.5 fold, about 3 fold, about 3.5 fold, about 4 fold, about 4.5 fold, about 5 fold, about 6 fold, about 7 fold, about 8 fold, about 9 fold, about 10 fold, about 15 fold, about 20 fold, about 25 fold, about 50 fold, and/or about 100 fold, or any combination thereof, as compared to that of a normal subject known not to have a metabolic disease.

A forty first disclosure includes at least one of the methods according to the thirty ninth to the fortieth disclosures, further comprising: treating the subject using at least one method according to any one of the twenty seventh to the thirty eighth disclosures.

A forty second disclosure includes a method of suppressing the activity of CXXC5, comprising the steps of: providing a subject at least one therapeutically effective dose of at least one compound according to any the first to the twenty fifth disclosures, or a pharmaceutically acceptable salt thereof, or a metabolite thereof, wherein the effective dose of the at least one compound suppresses the activity of CXXC5.

A forty third disclosure includes the method according to the forty second disclosure, wherein the subject comprises a human, an animal, a cell, and/or a tissue.

A forty fourth disclosure includes a method of reducing resistance to a therapeutic regime, comprising: administering to a subject at least one therapeutically effective dose of at least one agent that inhibits or reduces the CXXC5-DVL interface the interaction between CXXC5 and DVL, and/or the activity of the CXXC5 and/or the CXXC5-DVL interface.

A forty fifth disclosure includes the method according to the forty fourth disclosure, wherein wherein the at least one agent that inhibits the CXXC5-DVL interface, that inhibits the interaction between CXXC5 and DVL, and/or that inhibits the activity of CXXC5 and/or the CXXC5-DVL interface comprises at least one compound according to any one of the first to the twenty fifth disclosures and/or at least one composition according to the twenty sixth disclosure.

A forty sixth disclosure includes the method according to any one of the forty fourth to the forty fifth disclosures, wherein the subject was previously or is being concomitantly treated with at least one therapeutic regime including, but is not limited to, surgery, weight loss, healthy eating, physical activity, insulin therapy, and/or a medication/drug therapy.

A forty seventh disclosure includes the method according to any one of the forty fourth to the forty sixth disclosures, wherein the subject was previously or is being concomitantly treated with at least one medication including, but is not limited to, orlistat, lorcaserin, phentermine-topiramate, naltrexone-bupropion, liraglutide, benzphetamine, diethylpropion, sulfonylureas, meglitinides, thiazolidinediones, DPP-4 inhibitors, insulin analog, alpha glucosidase inhibitor, SGLT2 inhibitors, sitagliptin, metformin, rosiglitazone, ocaliva, selonsertib, elafibranol, cenicriviroc, MGL-3196, GR-MD-02, and/or aramchol.

A forty eighth disclosure includes a kit for for carrying out any one of the preceding methods disclosed herein. Components of the kit include, but are not limited to, one or more of agents/compositions disclosed herein, reagents, containers, equipment and/or instructions for using the kit.

A forty ninth disclosure includes the kit according to the forty eighth disclosure, wherein the one or more of agents/compositions includes at least one compound according to any one of the first to the twenty fifth disclosures and/or at least one composition according to the twenty sixth disclosure.

A fiftieth disclosure includes at least one of the methods according to any one of the twenty seventh to the forty seventh disclosures, wherein the therapeutically effective dose of at least one compound according to any one of the first to the twenty fifth disclosures and/or at least one composition according to the twenty sixth disclosure, is on the order of between about 5 mg to about 2000 mg and the dose of the compound is administered to the subject at least once per day. In some disclosures, the therapeutically effective dose of at least one compound according to any one of the first to the twenty fifth disclosures and/or at least one composition according to the twenty sixth disclosure, includes, but is not limited to, on the order of between: about 10 mg to about 1900 mg; about 15 mg to about 1800 mg; about 15 mg to about 1700 mg; about 20 mg to about 1600 mg; about 25 mg to about 1500 mg; about 30 mg to about 1000 mg; about 50 mg to about 1000 mg; about 50 mg to about 800 mg; about 100 mg to about 800 mg; about 300 mg to about 800 mg; about 500 mg to about 800 mg; about 5 mg to about 50 mg; about 1000 mg to about 1700 mg; about 1200 mg to about 1700 mg; about 1500 mg to about 1700 mg; about 10 mg to about 1000 mg; about 10 mg to about 30 mg; about 1500 mg to about 2000 mg; about 100 mg to about 200 mg; about 100 mg to about 150 mg; and/or any combination thereof. Consistent with these disclosures, the therapeutically effective dose of at least one compound according to any one of the first to the twenty fifth disclosures and/or at least one composition according to the twenty sixth disclosure, includes, but not limited to, on the order of between: about 1 mg/m2 to about 1500 mg/m2; about 10 mg/m2 to about 1000 mg/m2; about 20 mg/m2 to about 800 mg/m2; about 10 mg/m2 to about 50 mg/m2; about 800 mg/m2 to about 1200 mg/m2; about 50 mg/m2 to about 500 mg/m2; about 500 mg/m2 to about 1000 mg/m2; about 80 mg/m2 to about 150 mg/m2; about 80 mg/m2 to about 120 mg/m2; and/or any combination thereof.

A fifty first disclosure includes at least one of the methods according to any one of the twenty seventh to the forty seventh disclosures, wherein the therapeutically effective dose of at least one compound according to any one of the first to the twenty fifth disclosures and/or at least one composition according to the twenty sixth disclosure, is on the order of between about 0.01 mg to about 200 mg and the dose of the compound is administered to the subject at least once per day. In some disclosures, the therapeutically effective dose of at least one compound according to any one of the first to the twenty fifth disclosures and/or at least one composition according to the twenty sixth disclosure, includes, but is not limited to, on the order of between: about 0.01 mg to about 150 mg; about 0.01 mg to about 100 mg; about 0.01 mg to about 80 mg; about 0.01 mg to about 60 mg; about 0.05 mg to about 100 mg; about 0.05 mg to about 80 mg; about 0.05 mg to about 50 mg; about 0.1 mg to about 100 mg; about 0.1 mg to about 50 mg; about 0.2 mg to about 100 mg; about 0.2 mg to about 50 mg; about 0.5 mg to about 100 mg; about 0.5 mg to about 50 mg; about 100 mg to about 200 mg; ; about 100 mg to about 150 mg; and/or any combination thereof. In some of these disclosures, the therapeutically effective dose of at least one compound according to any one of the first to the twenty fifth disclosures and/or at least one composition according to the twenty sixth disclosure, includes, but not limited to, on the order of between: about 0.01 mg/m² to about 100 mg/m²; about 0.01 mg/m² to about 80 mg/m²; about 0.01 mg/m² to about 50 mg/m²; about 0.01 mg/m² to about 25 mg/m²; about 0.05 mg/m² to about 100 mg/m²; about 0.05 mg/m² to about 80 mg/m²; about 0.05 mg/m² to about 50 mg/m²; about 80 mg/m² to about 150 mg/m²; about 80 mg/m² to about 120 mg/m²; and/or any combination thereof.

A fifty second disclosure includes a method of determining the presence of a metabolic disease in a subject, the method comprising assaying for a level of expression of CXXC5 gene and/or a level of expression of CXXC5 protein that is elevated as compared to a reference value.

A fifty third disclosure includes the method according to the fifty second disclosure, wherein the metabolic disease includes at least one condition selected from, or comprising, metabolic disorder, metabolic syndrome, obesity, high blood pressure, high blood sugar, high serum triglycerides, hyperuricemia, fatty liver, polycystic ovarian syndrome, erectile dysfunction, acanthosis nigricans, type 2 diabetes mellitus, hypoadiponectinemia, cirrhosis, portal hypertension, cardiovascular diseases, coronary artery disease, lipodystrophy, dyslipidemia, hepatic steatosis, non-alcoholic fatty liver disease (NAFLD), and/or non-alcoholic steatohepatitis (NASH); wherein the metabolic disease includes at least one condition selected from, or comprising, obesity, type 2 diabetes mellitus, hepatic steatosis, non-alcoholic fatty liver disease (NAFLD), and/or non-alcoholic steatohepatitis (NASH); and/or wherein the metabolic disease includes non-alcoholic steatohepatitis (NASH).

A fifty fourth disclosure includes the method according to any one of the fifty second to the fifty third disclosures, wherein the reference value is the level of expression of CXXC5 gene or the level of expression of CXXC5 protein in a normal subject known not to have a metabolic disease.

A fifty fifth disclosure includes the method according to any one of the fifty second to the fifty fourth disclosures, wherein the level of expression of CXXC5 gene and/or the level of expression of CXXC5 protein that is elevated in the adipose tissue, pancreas, and/or liver of the subject.

A fifty sixth disclosure includes the method according to any one of the fifty second to the fifty fifth disclosures, wherein the level of expression of CXXC5 gene and/or the level of expression of CXXC5 protein is elevated at least about 10%, about 20%, about 25%, about 30%, about 40%, about 50%, about 60%, about 70%, about 80%, about 90%, about 100%, about 200%, about 300%, about 400%, about 500%, and/or about 1000%, or any combination thereof, as compared to the reference value; and/or wherein the level of expression of CXXC5 gene and/or the level of expression of CXXC5 protein is elevated at least about 1.5 fold, about 2 fold, about 2.5 fold, about 3 fold, about 3.5 fold, about 4 fold, about 4.5 fold, about 5 fold, about 6 fold, about 7 fold, about 8 fold, about 9 fold, about 10 fold, about 15 fold, about 20 fold, about 25 fold, about 50 fold, and/or about 100 fold, or any combination thereof, as compared to the reference value.

A fifty seventh disclosure includes at least one of the methods according to the any one of the fifty second to the fifty sixth disclosures, further comprising: contacting CXXC5 with at least one agent comprising at least one compound according to any one of the first to the twenty fifth disclosures and/or at least one composition according to the twenty sixth disclosure.

A fifty eighth disclosure includes at least one of the methods according to the any one of the fifty second to the fifty seventh disclosures, further comprising: detecting the presence of CXXC5 in the subject.

A fifty ninth disclosure includes at least one of the methods according to the any one of the fifty second to the fifty eighth disclosures, further comprising: treating the subject using at least one method according to any one of the twenty seventh to the thirty eighth disclosures and the fiftieth to the fifty first disclosures.

A sixtieth disclosure includes at least one of the methods according to the any one of the fifty second to the fifty ninth disclosures, wherein the subject comprises a cell, an animal, or a human. Consistent with these embodiments, the cell can include at least one type of cells including adipocytes and/or hepatocytes.

### BRIEF DESCRIPTION OF THE DRAWINGS

The following drawings form part of the present specification and are included to further demonstrate certain embodiments. Some embodiments may be better understood by reference to one or more of these drawings alone or in combination with the detailed description of specific embodiments presented.

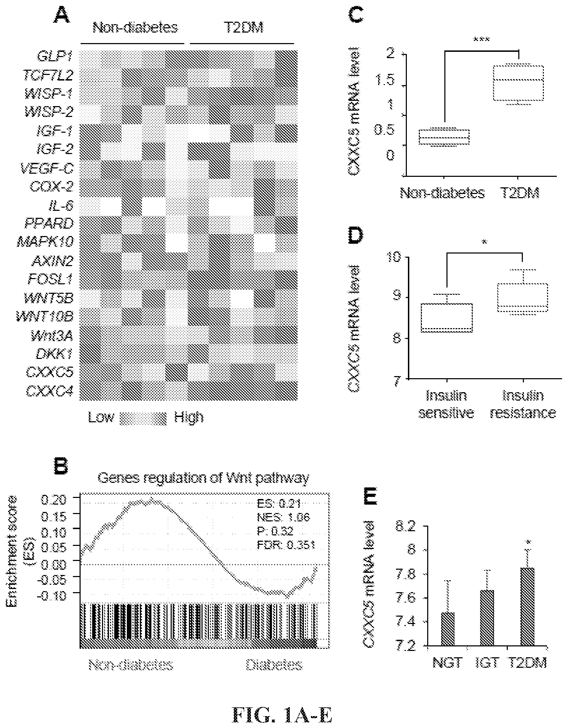

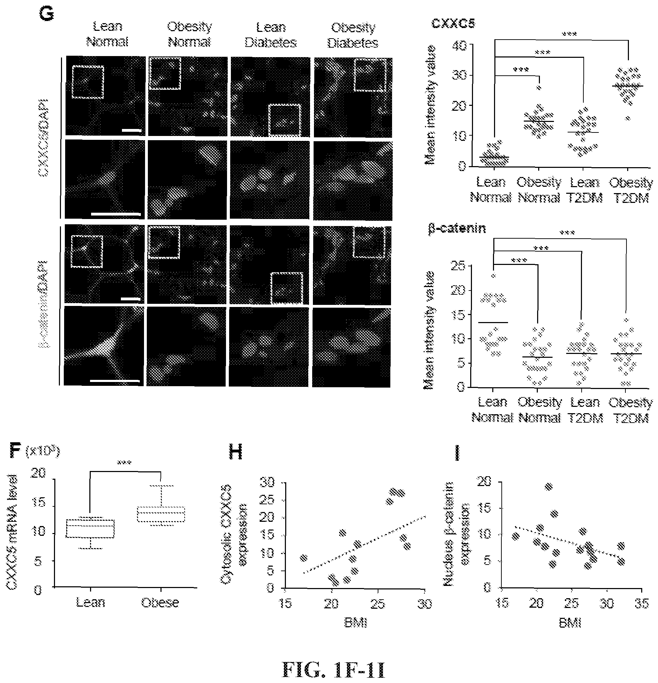

**FIG. 1A****.** Graph illustrating the expression of CXXC5 as an inhibitor of Wnt/β-catenin pathway in human adipose tissues of obesity-induced diabetes patients. Hierarchical clustering and heat-map of RNA-seq data in the visceral adipose tissues from obese diabetic woman (*n* = 5 for all groups). The color scale shows Z-score fragments per kilobase of transcript per million mapped reads representing the mRNA level of each gene in the green (low expression) to red (high expression) colored scheme. All data are presented as the mean ± SD. **P* < 0.01, ****P* < 0.005 determined by Student's *t*-test.
**FIG. 1B****.** Graph illustrating a gene set enrichment analysis of microarray transcriptome data from BMI-matched diabetes patients for Wnt/β-catenin signaling-activated gene signature. Black columns indicate 83 enriched genes in visceral adipose tissues of normal glucose tolerance or diabetes subjects involving the Wnt/β-catenin signaling pathway (*n* = 17 for all groups). NES, normalized enrichment score; ES, enrichment score; FDR, false discovery rate.
**FIG. 1C****.** Graph illustrating the expression level of *CXXC5* in visceral adipose tissues from the lean and obese subjects (*n* = 5 per group)
**FIG. 1D****.** Graph illustrating the expression level of *CXXC5* in visceral adipose tissues from BMI-matched obese patients suffering from insulin sensitive and resistance (*n* = 5 per group).
**FIG. 1E****.** Graph illustrating the expression level of *CXXC5* in subcutaneous adipose tissues from the normal glucose tolerance (NGT), impaired glucose tolerance (IGT), and T2DM subjects (*n* = 4 per group),
**FIG. 1F****.** Graph illustrating the expression level of *CXXC5* in subcutaneous adipose tissues from the lean and obese subjects (*n* = 10 per group).
**FIG. 1G****.** Representative images of CXXC5 and β-catenin in visceral adipose tissue in the adipose tissue and quantitative analyses of IHC staining for CXXC5 and β-catenin. Visceral adipose tissues from lean, obesity, diabetes, and obese-diabetes human subjects (*n* = 4 per group). Quantitative mean intensity value of IHC staining of CXXC5 and β-catenin was performed in three random, representative fields (right)
**FIG. 1H****.** Graph illustrating the correlation between CXXC5 expression and BMI.
**FIG. 1I****.** Graph illustrating the correlation between β-catenin expression and BMI.
**FIG. 2****.** Graph illustrating the expression of oxidative phosphorylation and adipokine genes in human non-diabetes and T2DM subjects. Hierarchical clustering and heat-map of RNA-seq data in the visceral adipose tissues from obese diabetic woman (*n* = 5 for all groups). The color scale shows Z-score fragments per kilobase of transcript per million mapped reads representing the mRNA level of each gene in the green (low expression) to red (high expression) colored scheme.
**FIG. 3A****.** Graph illustrating the expression level of *CXXC5* during the HFD treatment for 24 weeks in epididymal adipose tissues (*n* = 3 per group). All data are presented as the mean ± SD. **P* < 0.01 determined by Student's *t*-test.
**FIG. 3B****.** Graph illustrating the expression level of *CXXC5* during the HFD treatment for 24 weeks in mesenteric adipose tissues (*n* = 1 per group) from HFD-fed mice.
**FIG. 4A****.** Representative photographs of *Cxxc5*^{+/+} and *Cxxc5*^{-/-} mice that were fed on high fat diet (HFD) or normal chow diet (NCD) for 8 weeks (*n* = 9-13 per group). HFD comprises about 20% protein, about 60% fat, and about 20% carbohydrate.
**FIG. 4B****.** Graph illustrating the body weight of *Cxxc5*^{+/+} and *Cxxc5*^{-/-} mice that were fed on HFD or NCD for 8 weeks (*n* = 9-13 per group).
**FIG. 4C****.** Representative photographs of fat pads (epiWAT and BAT), mesenteric, perirenal, and liver of *Cxxc5*^{+/+} and *Cxxc5*^{-/-} mice that were fed on HFD or NCD for 8 weeks (*n* = 9-13 per group).
**FIG. 4D-4I****.** Graphs illustrating fasting glucose level (FIG. 4D), plasma insulin concentration in the overnight fasted state (**FIG. 4E**), glucose tolerance test (**FIG. 4F**), insulin tolerance test (**FIG. 4G**), homeostatic model assessment-insulin resistance (**FIG. 4H**), relative levels of leptin and resistin (**FIG. 4I**) of *Cxxc5*^{+/+} and *Cxxc5*^{-/-} mice that were fed on HFD or NCD for 8 weeks (*n* = 9-13 per group).
**FIG. 4J****.** Graph illustrating relative *Cxxc5* mRNA expression level in epiWAT, BAT, liver, muscle, colon, spleen, kidney, and heart of *Cxxc5*^{+/+} mice that were fed on HFD or NCD for 8 weeks (*n* = 6 per group). All data are presented as the mean ± SD. **P* < 0.01, ***P* < 0.05, ****P* < 0.005 determined by Student's *t*-test.
**FIG. 4K****.** Western blot illustrating the level of β-catenin and Cxxc5 in epiWAT, BAT, and liver tissue (*n* = 3 per group).
**FIG. 5A****.** Graph illustrating wet weight of epiWAT, mesenteric, perirenal, liver, and BAT of HFD-*Cxxc5*^{+/+} and HFD-*Cxxc5*^{-/-} mice.
**FIG. 5B****.** Graph illustrating daily food intake of HFD-*Cxxc5*^{+/+} and HFD-*Cxxc5*^{*-*/-} mice during all study weeks.
**FIG. 6A****.** Graphs illustrating plasma concentration or relative levels of leptin, resistin, adiponectin, TG, total cholesterol, and HDL-cholesterol of HFD-*Cxxc5*^{+/+} and HFD-*Cxxc5*^{-/-} mice in overnight fasted state.
**FIG. 6B****.** Graphs illustrating plasma concentration of Ca⁺⁺ and Mg⁺⁺ of HFD-*Cxxc5*^{+/+} and HFD-*Cxxc5*^{-/-} mice in non-fasted state.
**FIG. 7A-7C****.** Graphs illustrating glucose tolerance (**FIG. 7A**), insulin tolerance (**FIG. 7B**), and plasma concentration of glucose and TG in overnight fasted state (**FIG. 7C**) of *Cxxc5*^{+/+} and *Cxxc5*^{-/-} mice that were fed on NCD for 8 weeks *(n* = 9-12 per group). All data are presented as the mean ± SD. Statistical analysis was determined by Student's *t*-test.
**FIG. 7D****.** Representative photographs and a graph (right panel) illustrating wet weight of epiWAT, BAT, liver, mesenteric, perirenal, and heart of NCD-*Cxxc5*^{+/+} and NCD-*Cxxc5*^{-/-} mice.
**FIG. 8A-8B****.** Graphs illustrating relative expression levels of Wnt/β-catenin signaling target genes (*Tcf7l2*, *Axin2, Dvl1, Wisp1,* and *Fosl1*) in epiWAT, BAT, and liver (**FIG. 8A**) and relative expression level of *Cxxc4* in epiWAT, BAT, liver, muscle, colon, spleen, kidney, and heart (**FIG. 8B**) of *Cxxc5*^{+/+} mice that were fed on HFD or NCD for 8 weeks (*n* = 6 per group). All data are presented as the mean ± SD. **P* < 0.01, ***P* < 0.05, ****P* < 0.005 determined by Student's *t*-test.
**FIG. 9A****.** Representative images of H&E staining (left) and graphs illustrating quantitative analyses of adipocyte cell size (middle) and the percentage of crown-like structures (CLSs) per adipocytes on histological sections (right) of epiWAT from *Cxxc5*^{+/+} and *Cxxc5*^{-/-} mice fed on HFD for 8 weeks *(n* = 9-13 per group).
**FIG. 9B****.** Representative images of IHC staining for β-catenin, Cxxc5, F4/80, and CD11b of epiWAT from *Cxxc5*^{+/+} and *Cxxc5*^{-/-} mice fed on HFD for 8 weeks *(n* = 9-13 per group).
**FIG. 9C-9E****.** Graphs illustrating relative expression levels of marker genes for M1 macrophage (FIG. 9C), M2 macrophage (FIG. 9D), and Wnt/β-catenin signaling pathway target genes (FIG. 9E) in epiWAT of HFD-*Cxxc5*^{+/+} and HFD-*Cxxc5*^{-/-} mice. Scale bars = 100 µm. All data are presented as the mean ± SD. **P* < 0.01, ***P* < 0.05, ****P* < 0.005 determined by Student's *t*-test.
**FIG. 9F****.** Representative images of H&E staining and Oil Red O staining of liver tissues from *Cxxc5*^{+/+} and *Cxxc5*^{-/-} mice fed an HFD for 8 weeks *(n* = 9-13 per group).
**FIG. 9G-9K****.** Graphs illustrating the concentration of triglyceride (TG) in hepatocytes (**FIG. 9G**); plasma concentration of FFA (**FIG. 9H**), ALT and AST (**FIG. 9I**); relative mRNA expression of gluconeogenic (**FIG. 9J**), and lipogenic genes (**FIG. 9K**) in liver tissues of HFD-*Cxxc5*^{+/+} and HFD-*Cxxc5*^{-/-} mice. Scale bars = 100 µm. All data are presented as the mean ± SD. **P* < 0.01, ***P* < 0.05, ****P* < 0.005 determined by Student's *t-*test.
**FIG. 10A****.** Chemical structure of A3334.
**FIG. 10B-10C****.** Graphs illustrating the effect of A3334 on CXXC5-Dvl interaction using in vitro binding assay (FIG. 10B) and TOPFlash reporter activity of HEK293-TOP cells for determining effective concentration of A3334 (FIG. 10C).
**FIG. 10D****.** Oil Red O staining and graphs illustrating the effect of dose-dependent treatment of A3334 on 3T3-L1 preadipocytes. Scale bars = 100 µm. All data are presented as the mean ± SD. **P* < 0.01, ***P* < 0.05, ****P* < 0.005 determined by Student's *t-*test.
**FIG. 10E****.** Oil Red O staining and graphs illustrating the effect of treatment of A3334 on 3T3-L1 preadipocytes transfected with control or β-catenin siRNA. Scale bars = 100 µm. All data are presented as the mean ± SD. **P* < 0.01, ***P* < 0.05, ****P* < 0.005 determined by Student's *t*-test.
**FIG. 11A****.** Schematic diagram illustrating experimental protocols for evaluating the remission effect of A3334 on the progression of diabetes in HFD-fed mice.

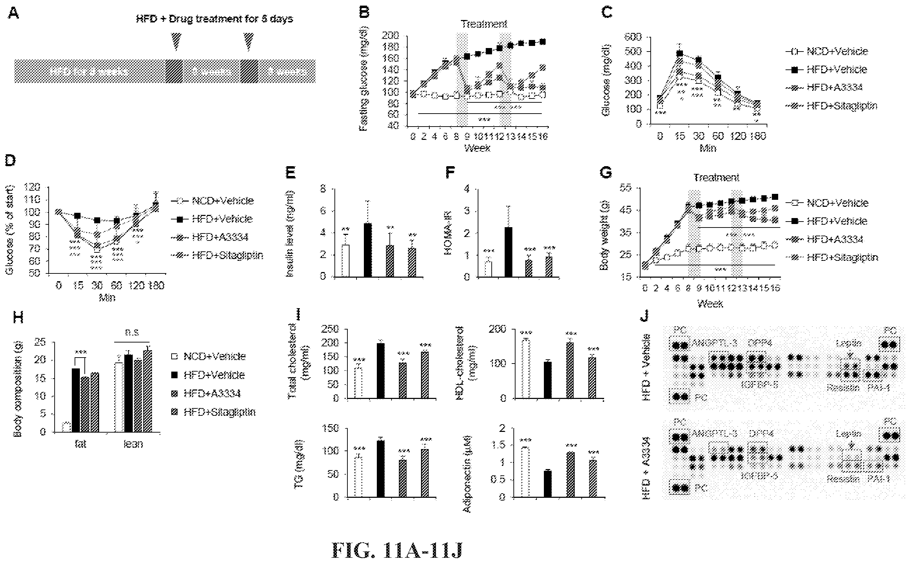

**FIG. 11B-11I****.** Graphs illustrating the effect of A3334 and sitagliptin on diet-induced obesity and insulin resistance by measuring fasting glucose (**FIG. 11B**), glucose tolerance (**FIG. 11C**), insulin tolerance (**FIG. 11D**), plasma insulin concentration in overnight fasted state (**FIG. 11E**), homeostatic model assessment-insulin resistance (HOMA-IR, **FIG. 11F**), body weight (**FIG. 11G**), body composition (**FIG. 11H**), and plasma concentration of total cholesterol, HDL-cholesterol, TG, and adiponectin in the overnight fated state (**FIG. 11I**). A3334 treatment improves diet-induced obesity and insulin resistance. C57BL/6 mice fed the NCD or HFD for 16 weeks were p.o. administered A3334 (25 mg kg⁻¹ d⁻¹) or sitagliptin (50 mg kg⁻¹ d⁻¹) on weeks 8 and 12 (*n* = 10 per group). All data are presented as the mean ± SD. **P* < 0.01, ***P* < 0.05, ****P* < 0.005 determined by Student's *t-*test.
**FIG. 11J****.** Representative mouse adipokine and adipokine-related protein array illustrating the effect of A3334 on HFD-mice.

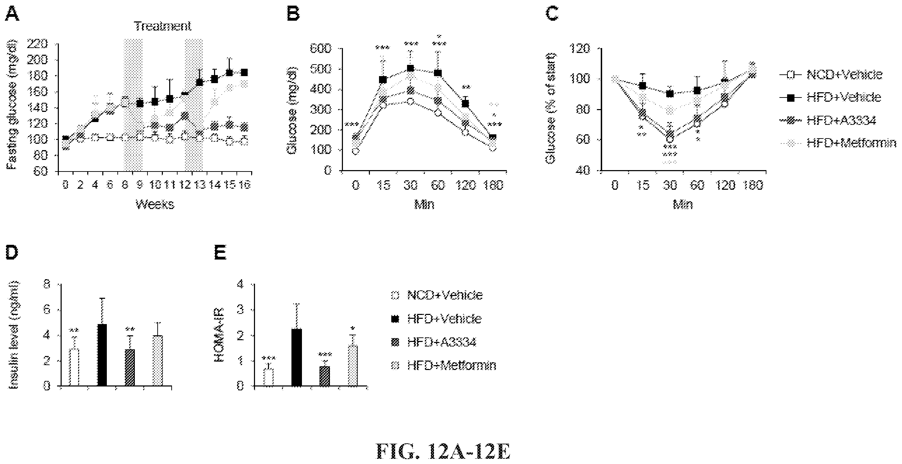

**FIG. 12A-12E****.** Graphs illustrating the effect of A3334 and metformin on diet-induced insulin resistance by measuring fasting glucose (**FIG. 12A**), glucose tolerance (**FIG. 12B**), insulin tolerance (**FIG. 12C**), plasma concentration of insulin in overnight fasted state (**FIG. 12D**), homeostatic model assessment-insulin resistance (HOMA-IR, **FIG. 12E**). C57BL/6 mice fed the NCD or HFD for 16 weeks were p.o. administered A3334 (25 mg kg⁻¹ d⁻¹) or metformin (100 mg kg⁻¹ d⁻¹) on weeks 8 and 12 (*n* = 10 per group). All data are presented as the mean ± SD. **P* < 0.01, ***P* < 0.05, ****P* < 0.005 determined by Student's *t-*test.

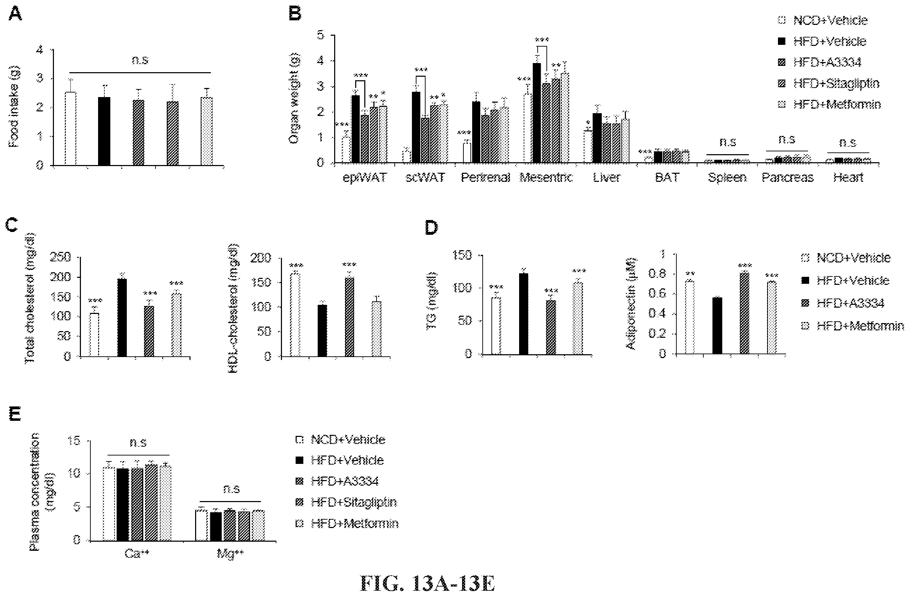

**FIG. 13A-13E****.** Graphs illustrating the effect of A3334, sitagliptin, and metformin on diet-induced obesity by measuring daily food intake during all study weeks (**FIG. 13A**), wet weight of epiWAT, scWAT, perirenal, mesenteric, liver, BAT, spleen, pancreas, and heart (**FIG. 13B**), plasma concentration of total-cholesterol and HDL-cholesterol in overnight fated state (**FIG. 13C**), plasma concentration of TG and adiponectin in overnight fated state (**FIG. 13D**), plasma concentration in overnight fated state of Ca⁺⁺ and Mg⁺⁺ (**FIG. 13E**). C57BL/6 mice fed the NCD or HFD for 16 weeks were p.o. administered A3334 (25 mg kg⁻¹ d⁻¹), sitagliptin (50 mg kg⁻¹ d⁻¹), or metformin (100 mg kg⁻¹ d⁻¹) on weeks 8 and 12 (*n* = 10 per group). All data are presented as the mean ± SD. **P* < 0.01, ***P* < 0.05, ****P* < 0.005 determined by Student's *t*-test.
**FIG. 14A-14H****.** A3334 treatment increases epiWAT remodeling and hepatic glucose homeostasis. C57BL/6 mice fed an NCD or HFD for 16 weeks were p.o. administered A3334 (25 mg kg⁻¹ d⁻¹) or sitagliptin (50 mg kg⁻¹ d⁻¹) on week 8 and 12 (*n* = 10 per group). Scale bars = 100 µm. All data are presented as the mean ± SD. **P* < 0.01, ***P* < 0.05, ****P* < 0.005 determined by Student's t-test.

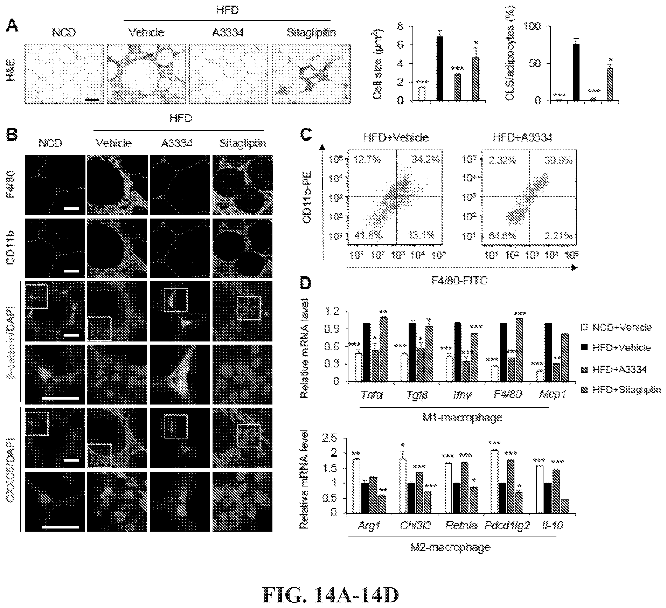

**FIG. 14A****.** Representative images of H&E staining of epi WAT (left) and quantitative analyses of adipocyte cell size of epiWAT (middle) and the percentage of crown-like structures (CLSs) per adipocytes on histological sections (right).
**FIG. 14B****.** Representative IHC images (*n* = 5 independent experiments) for F4/80 and CD11b, β-catenin, and Cxxc5 in the epiWAT.
**FIG. 14C****.** Flow cytometry analysis illustrating the expression of F4/80 and CD11b, and percentage of F4/80⁺CD11b⁺ cells.

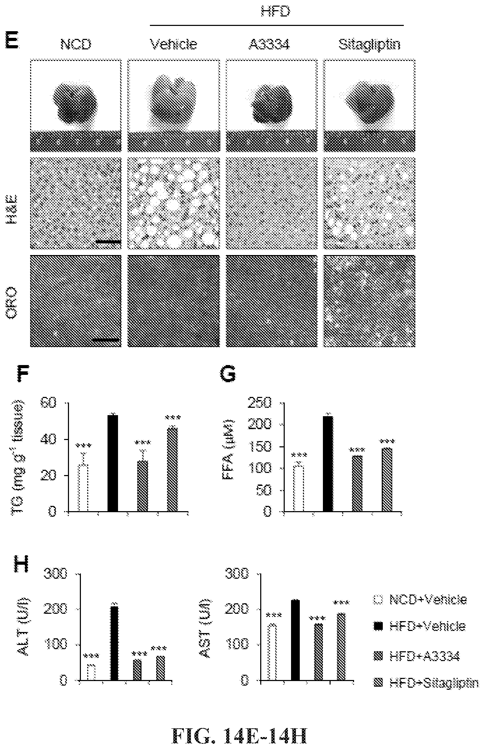

**FIG. 14D-14E****.** Graphs illustrating the effect of A3334 and sitagliptin on relative mRNA expression of M1 macrophage (**FIG. 14D**) and M2 macrophage (**FIG. 14E**).
**FIG. 14E****.** Representative images (three total images per group) of liver, H&E staining, and Oil Red O staining.
**FIG. 14F-14H****.** Graphs illustrating the effect of A3334 and sitagliptin on the level of TG (**FIG. 14F**), FFA (**FIG. 14G**), and ALT and AST (**FIG. 14H**).

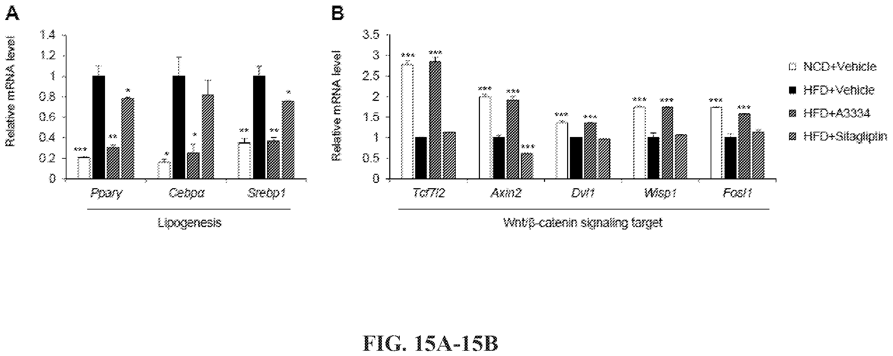

**FIG. 15A-15B****.** Graphs illustrating the effect of A3334 and sitagliptin on relative mRNA expression levels of lipogenesis markers such as *Pparγ*, *Cebpα,* and *Srebp1* (**FIG. 15A**) Wnt/b-catenin signaling target markers such as *Tcf7l2*, *Axin2, Dvl1, Wisp1*, and *Fosl1* (**FIG. 15B**) from the epiWAT described in Figure 14. All data are presented as the mean ± SD. ****P* < 0.005 determined by Student's *t*-test.

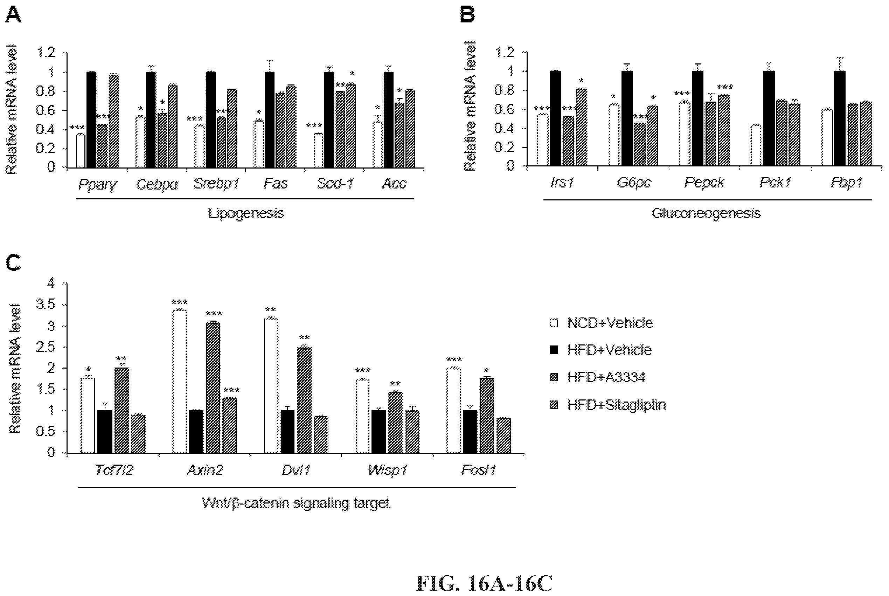

**FIG. 16A-16C****.** Graphs illustrating the effect of A3334 and sitagliptin on relative mRNA expression levels of lipogenesis markers such as *Pparγ*, *Cebpα, Srebp1*, *Fas*, *Scd-1, and Acc* (**FIG. 16A**), gluconeogenesis markers such as *Irs1*, *G6pc, Pepck, Pck1*, and *Fbp1* (**FIG. 16B**), and Wnt/b-catenin signaling target markers such as *Tcf7l2*, *Axin2, Dvl1, Wisp1*, and *Fosl1* (**FIG. 16C**) from liver tissues described in Figure 14. All data are presented as the mean ± SD. **P* < 0.01, ***P* < 0.05, ****P* < 0.005 determined by Student's *t*-test.

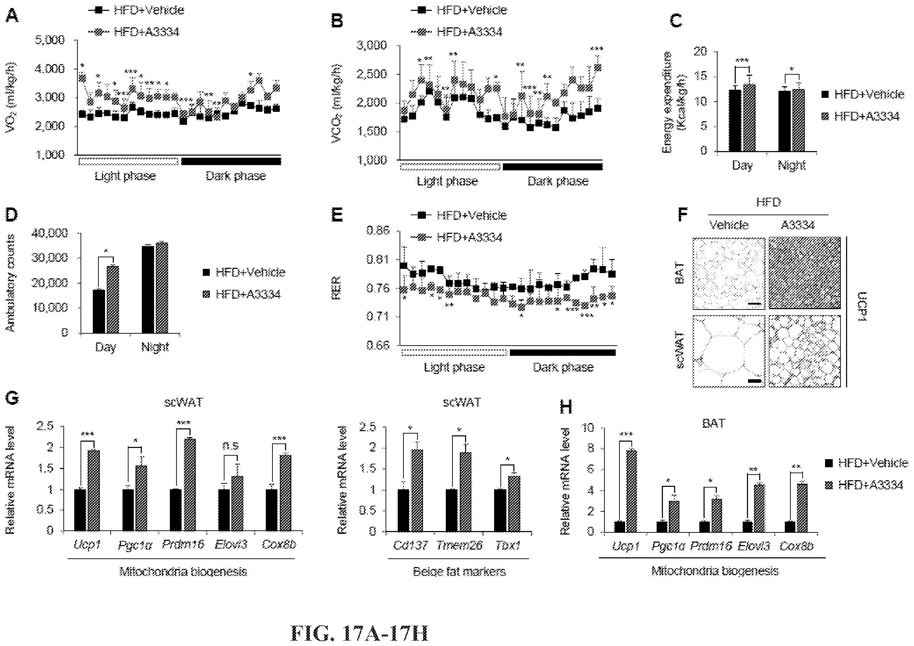

**FIG. 17A-17H****.** A3334 treatment increases energy expenditure via enhancing brown- and beige-fat activation. C57BL/6 mice fed an NCD or HFD for 16 weeks were p.o. administered A3334 (25 mg kg⁻¹ d⁻¹) on week 8 and 12 (*n* = 6 per group). Scale bars = 100 µm. All data are presented as the mean ± SD. **P* < 0.01, ***P* < 0.05, ****P* < 0.005 determined by Student's *t*-test.
**FIG. 17A-17B****.** Graphs illustrating the effect of A3334 on oxygen consumption (**FIG. 17A**) and carbon dioxide production (**FIG. 17B**) in light and dark phases over a 24 h period.
**FIG. 17C-17E****.** Graphs illustrating the effect of A3334 on energy expenditure normalized for body weight (**FIG. 17C**) and cumulative ambulatory counts (**FIG. 17D**), and respiratory exchange ratios (**FIG. 17E**).
**FIG. 17F****.** Representative images (three total images per group) of UCP1 immunohistochemistry of BAT and scWAT.
**FIG. 17G-17H****.** Graphs illustrating the effect of A3334 on relative expression levels of the indicated thermogenic and beige fat markers in scWAT (**FIG. 17G**), and thermogenic markers in BAT (**FIG. 17H**).

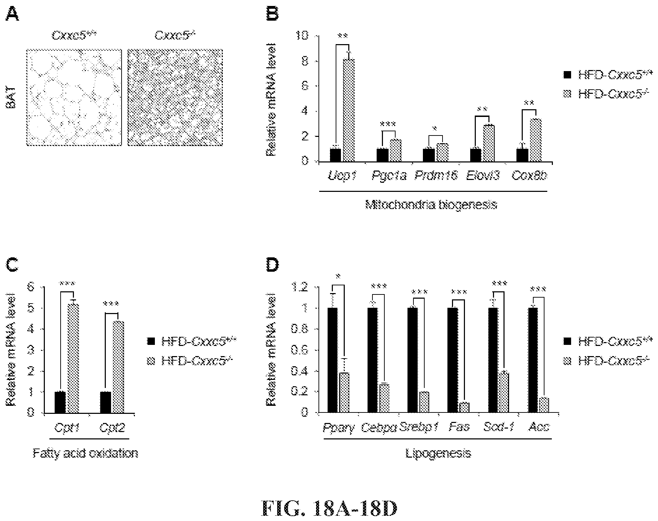

**FIG. 18A****.** Representative images (three total images per group) of UCP1 immunohistochemistry of BAT. Scale bars = 100 µm.
**FIG. 18B-18D****.** Graphs illustrating the effect of Cxxc5 knock out (Cxxc5^{-/-}) on relative expression mRNA levels of mitochondria biogenesis markers such as *Ucp1*, *Pgc1α*, *Prdm16*, *Elovl3,* and *Cox8b* (FIG. 18B), fatty acid oxidation markers such as *Cpt1* and *Cpt2* (FIG. 18C), and lipogenesis markers such as *Pparγ*, *Cebpα,* and *Srebp1* (FIG. 18D) in BAT. All data are presented as the mean ± SD. **P* < 0.01, ***P* < 0.05, ****P* < 0.005 determined by Student's *t*-test.

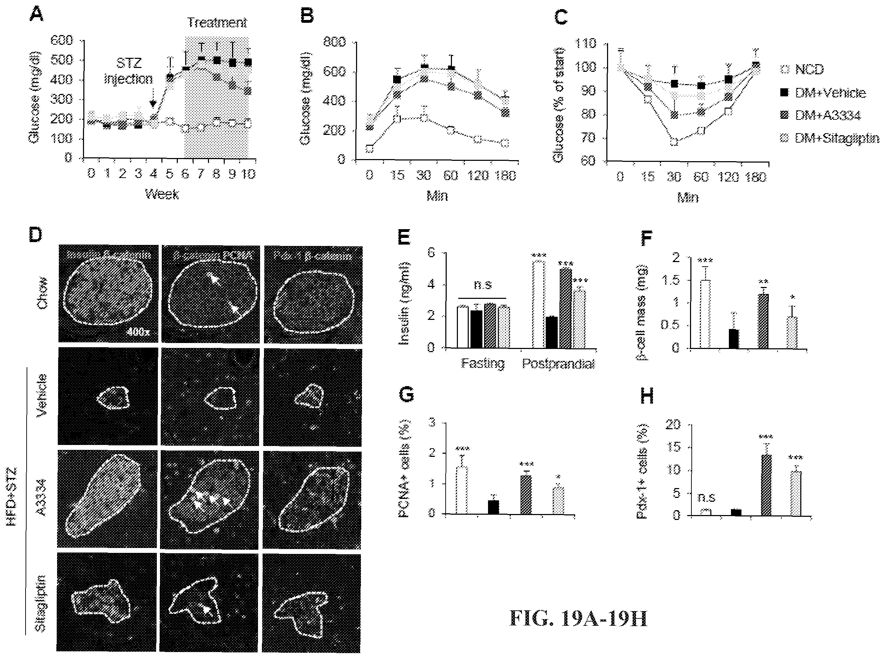

**FIG. 19A-19H****.** A3334 restores β-cell mass and functions in HFD-fed and STZ-induced diabetes mice. C57BL/6 mice fed the NCD or HFD for 4 weeks followed by receiving daily injection of STZ for 1 week were p.o. administered A3334 and sitagliptin (25 mg kg⁻¹ d⁻¹) for 4 weeks (*n* = 6 per group). Scale bars = 100 µm. All data are presented as the mean ± SD. **P* < 0.01, ***P* < 0.05, ****P* < 0.005 determined by Student's *t* test.
**FIG. 19A-19C****.** Graphs illustrating the effect of A3334 and sitagliptin on blood glucose levels (**FIG. 19A**), glucose tolerance (**FIG. 19B**), and insulin tolerance (**FIG. 19C**).
**FIG. 19D****.** Representative images of IHC staining for Insulin, β-catenin, PCNA, and PDX-1.
**FIG. 19E-19H****.** Graphs illustrating the effect of A3334 and sitagliptin on insulin level (**FIG. 19E**), β-cell mass (**FIG. 19F**), PCNA positive cells (**FIG. 19G**), and PDX-1 positive cells in the β-cells (**FIG. 19H**).

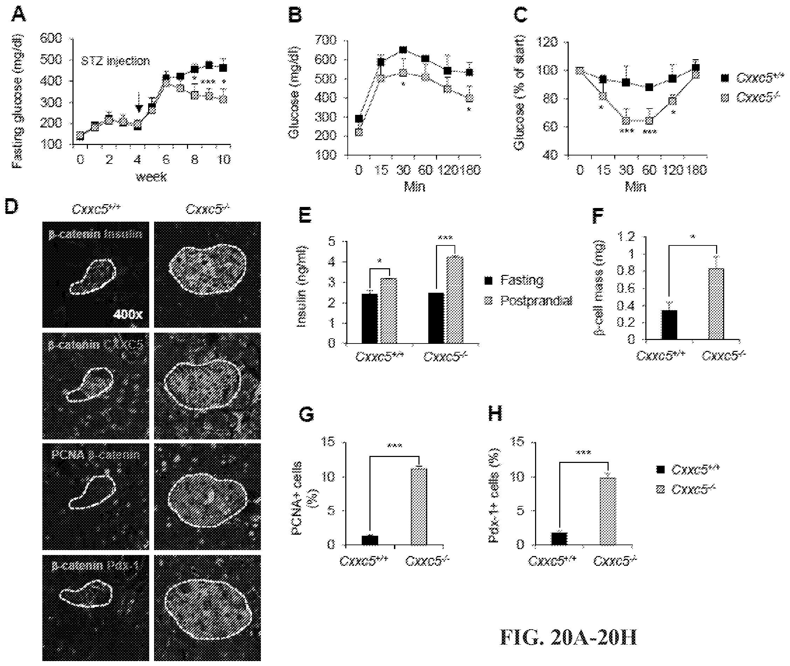

**FIG. 20A-20H****.** Ablation of *Cxxc5* preserves β-cell mass and functions in HFD-fed and STZ-induced diabetes mice. *Cxxc5*^{+/+} and *Cxxc5*^{-/-} mice fed the HFD for 4 weeks followed by receiving daily injection of STZ for 1 week (*n* = 6 per group). Scale bars = 100 µm. All data are presented as the mean ± SD. **P* < 0.01, ***P* < 0.05, ****P* < 0.005 determined by Student's t- test.
**FIG. 20A-20C****.** Graphs illustrating the effect of Cxxc5 knock out (Cxxc5^{-/-}) on blood glucose levels (**FIG. 20A**), glucose tolerance (**FIG. 20B**), and insulin tolerance (**FIG. 20C**).
**FIG. 20D****.** Representative images of IHC staining for Insulin, β-catenin, PCNA, and PDX-1.
**FIG. 20E-20H****.** Graphs illustrating the effect of Cxxc5 knock out (Cxxc5^{-/-}) on insulin level (**FIG. 20E**), β-cell mass (**FIG. 20F**), PCNA positive cells (**FIG. 20G**), and PDX-1 positive cells in the β-cells (**FIG. 20H**).

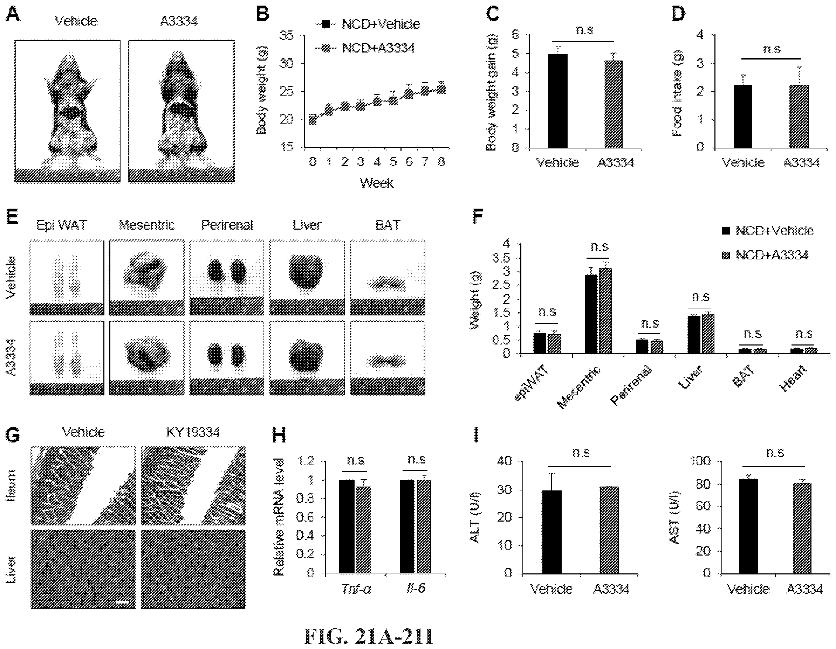

**FIG. 21A-21I****.** A3334 treatment has no effect in NCD mice. NCD fed C57BL/6 mice were p.o. administered A3334 (25 mg kg⁻¹ d⁻¹) for 8 weeks (*n* = 10 per group). Scale bars =100 µm. All data are presented as the mean ± SD.
**FIG. 21A****.** Representative photographs of vehicle- or A3334-treated NCD-mice.
**FIG. 21B-21D****.** Graphs illustrating the effect of A3334 on body weight (**FIG. 21B**), body weight gain (**FIG. 21C**), and food intake (**FIG. 21D**) of NCD-mice.
**FIG. 21E****.** Representative images (three total images per group) illustrating H&E staining of ileum and liver tissue of NCD-mice with or without A3334 treatment.
**FIG. 21F****.** Graph illustrating the effect of A3334 on wet weight of epiWAT, mesenteric, perirenal, liver, BAT, and heart of NCD-mice.
**FIG. 21G****.** Representative images (three total images per group) illustrating H&E staining of ileum and liver tissue of NCD-mice with or without A3334 treatment.
**FIG. 21H-21I****.** Graphs illustrating the effect of A3334 on relative mRNA levels (**FIG. 21H**) and plasma concentration of ALT and AST (**FIG. 21I**) in NCD-mice.

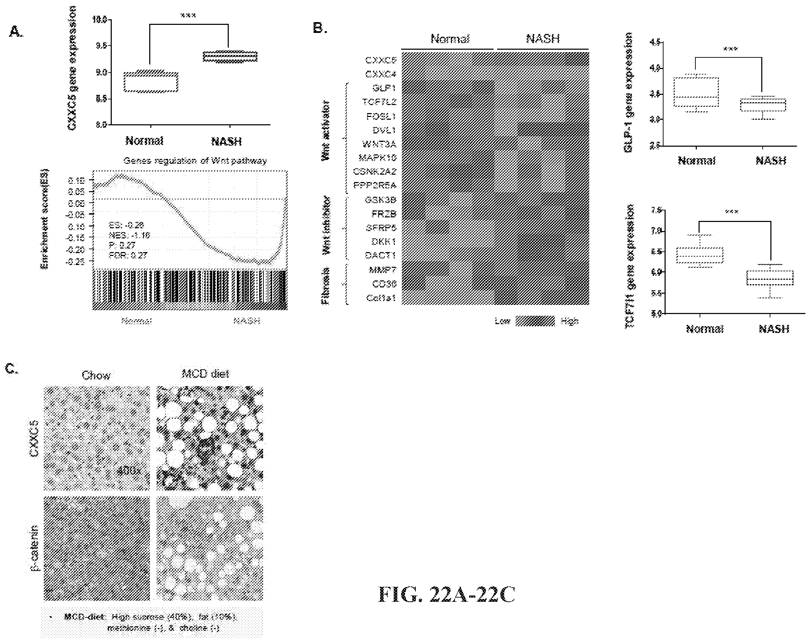

**FIG. 22A****.** Graphs illustrating CXXC5 gene expression analyzed by gene set enrichment analysis (GSEA) of microarray transcriptome data from human liver tissues of normal (*n* = 12) and NASH patents (*n* = 12) (GEO: GSE48452).
**FIG. 22B****.** Graphs illustrating heat map analysis of gene expression for the Wnt/β-catenin pathway target genes in normal (*n* = 4) and NASH (*n* = 4) subjects (dataset; GSE48452) (left panel) and the expressions of the key factors, e.g., TCF7L1, in NASH patient tissues (right panel). The specific induction of CXXC5, but not an analog of CXXC5, in NASH patients is illustrated.
**FIG. 22C****.** Immunohistochemical analyses illustrating the expression of CXXC5 and β-catenin in liver tissues of normal (Chow) and MCD-induced NASH (MCD-diet) mice. MCD-diet comprises high sucrose (40%), fat (10%), methionine (-), and choline (-).

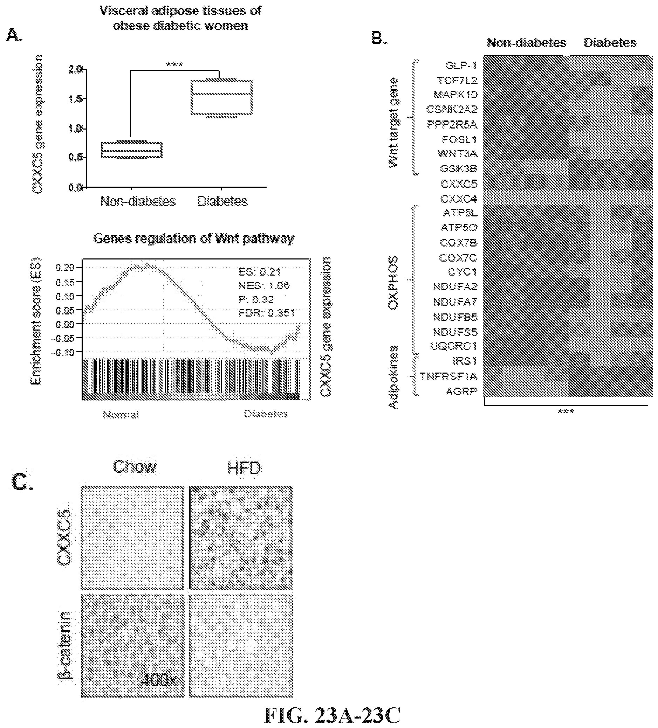

**FIG. 23A****.** Graphs illustrating the expression of the Wnt/β-catenin pathway target genes by gene set enrichment analysis (GSEA) of microarray transcriptome data from human liver tissues of different phases of normal (*n* = 4) and Type II diabetes (*n* = 4) patient (GEO: GSE16415).
**FIG. 23B****.** Graph illustrating heat map analysis of gene expression for the Wnt/β-catenin pathway target genes in non-diabetes (*n* = 4) and diabetes (*n* = 4) subjects.
**FIG. 23C****.** Immunohistochemical analyses illustrating the expression of CXXC5 and β-catenin in liver tissues of normal "Chow-fed" mice (Chow) and high fat diet-induced diabetes mice (HFD).

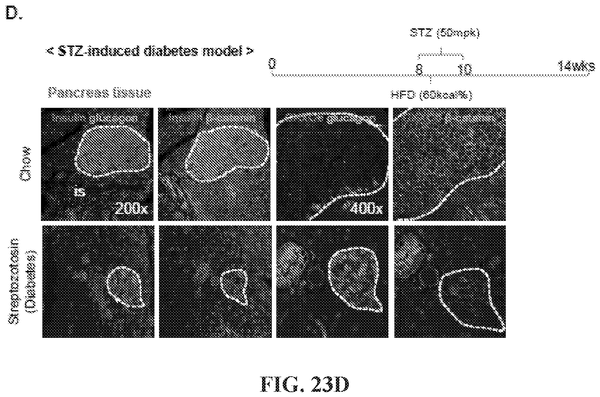

**FIG. 23D****.** Immunohistochemical analyses illustrating the expression of CXXC5 and β-catenin in pancreatic tissues of normal (Chow) and streptozotocin-induced diabetes (STZ) mice.

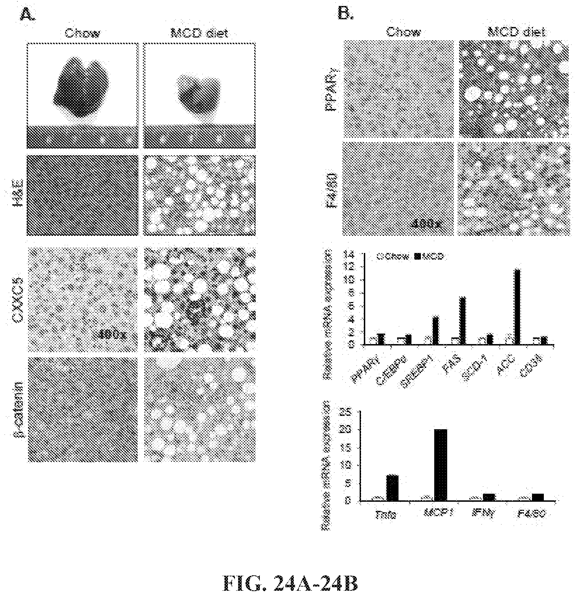

**FIG. 24A****.** H&E staining and immunohistochemical analyses illustrating steato / fatty liver and the expression of CXXC5 and β-catenin in liver tissues of normal (Chow) and methionine-choline deficient diet-induced NASH (MCD-diet) mice.
**FIG. 24B****.** Immunohistochemical and quantitative analyses illustrating the expression of inflammation markers in liver tissues of normal (Chow; white box) and MCD-induced NASH (MCD; black box) mice.

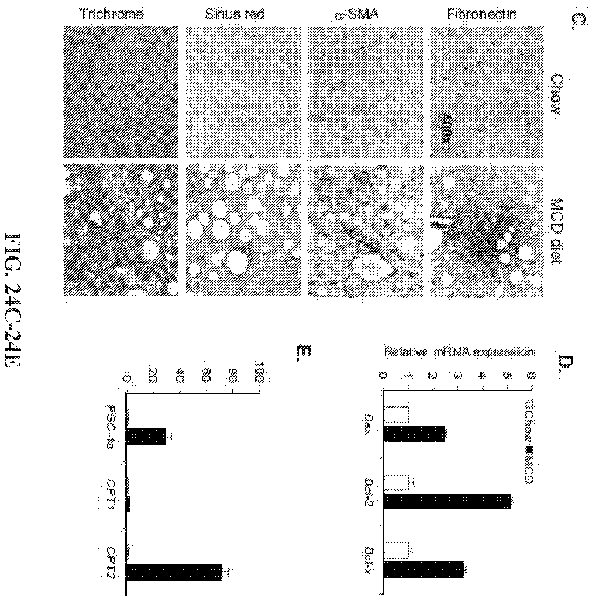

**FIG. 24C****.** Immunohistochemical analyses illustrating the expression of fibrosis markers in liver tissues of normal (Chow) and MCD-induced NASH (MCD-diet) mice.
**FIG. 24D****.** Graph illustrating the expression of apoptosis markers in liver tissues of normal (Chow; white box) and MCD-induced NASH (MCD; black box) mice.
**FIG. 24E****.** Graph illustrating the expression of fatty acid oxidation markers in liver tissues of normal (Chow; white box) and MCD-induced NASH (MCD; black box) mice.

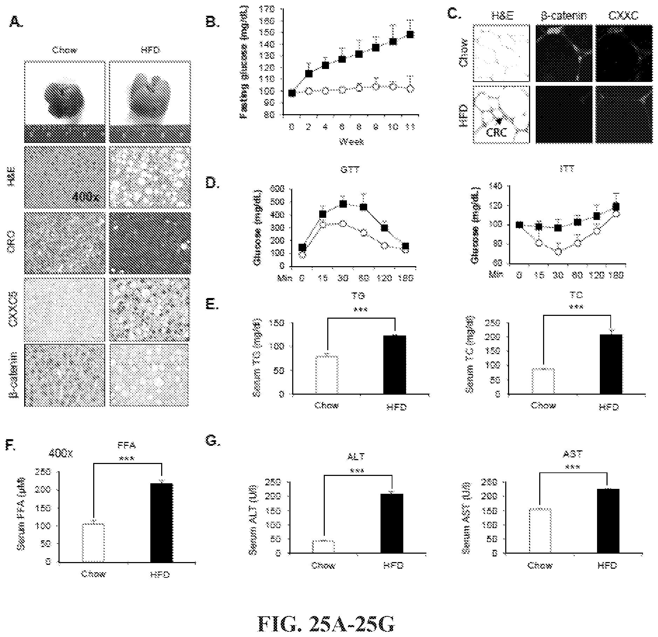

**FIG. 25A****.** H&E staining and immunohistochemical analyses illustrating steato / fatty liver and the expression of CXXC5 and β-catenin in liver tissues of normal (Chow) and HFD-induced diabetes (HFD) mice.
**FIG. 25B****.** Graph illustrating fasting glucose levels in normal (Chow; white circle) and HFD-induced diabetes (HFD; black box) mice.
**FIG. 25C****.** H&E staining and immunohistochemical analyses illustrating inflammation in normal (Chow) and HFD-induced diabetes (HFD) mice.
**FIG. 25D****.** Graphs illustrating glucose tolerance (GTT) and insulin tolerance (ITT) in normal (Chow; white circle) and HFD-induced diabetes (HFD; black box) mice.
**FIG. 25E****.** Graphs illustrating level of triglyceride (TG) and total cholesterol (TC) in normal (Chow) and HFD-induced diabetes (HFD) mice.
**FIG. 25F****.** Graph illustrating level of free fatty acid (FFA) in normal (Chow) and HFD-induced diabetes (HFD) mice (mean ± s.e.m., n=3, and ****P* < 0.005).
**FIG. 25G****.** Graphs illustrating level of alanine transaminase (ALT) and aspartate transaminase (AST) in normal (Chow) and HFD-induced diabetes (HFD) mice (mean ± s.e.m., n=3, and ****P* < 0.005).

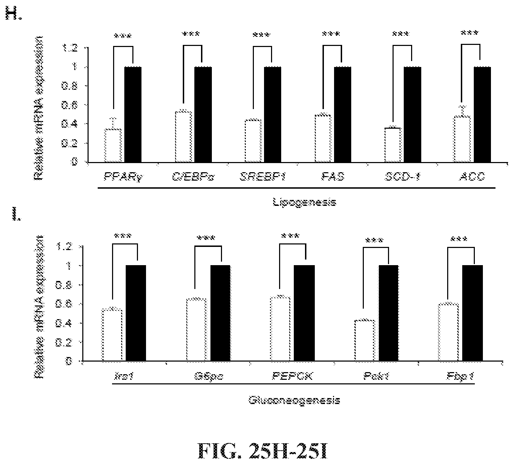

**FIG. 25H****.** Graph illustrating level of lipogenesis markers in normal (Chow; white bar) and HFD-induced diabetes (HFD; black bar) mice (mean ± s.e.m., n=3, and ****P* < 0.005).
**FIG. 25I**. Graph illustrating level of gluconeogenesis markers in normal (Chow; white bar) and HFD-induced diabetes (HFD; black bar) mice (mean ± s.e.m., n=3, and ****P* < 0.005).

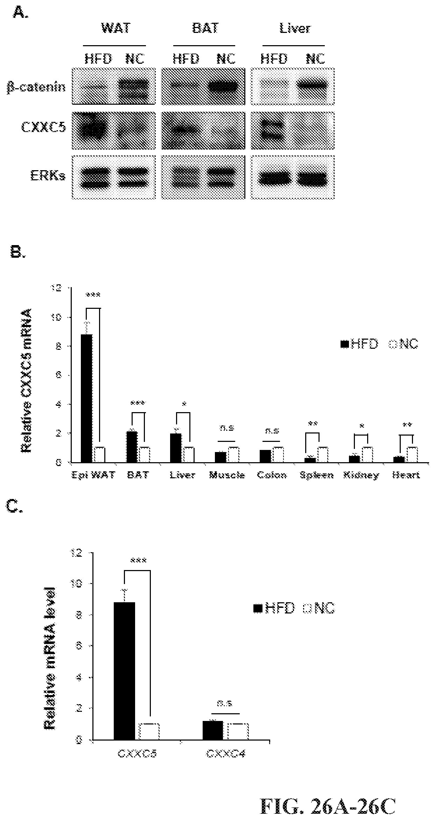

**FIG. 26A****.** Immunoblot illustrating the expressions of β-catenin and CXXC5 in various tissues (white adipose tissues (WAT), brown adipose tissues (BAT), liver tissues) of normal (Chow-fed; NC) and HFD-induced diabetes (HFD) mice.
**FIG. 26B****.** Graph illustrating the expression of relative CXXC5 mRNA levels in various tissues of normal (Chow-fed; NC) and HFD-induced diabetes (HFD) mice (mean ± s.e.m., n=3, **P* < 0.01, ***P* < 0.05, and ****P* < 0.005; n.s. not significant).
**FIG. 26C****.** Graph illustrating the expression of relative mRNA levels of CXXC5 and CXXC4 in normal (Chow-fed; NC) and HFD-induced diabetes (HFD) mice (mean ± s.e.m., n=3, and ****P* < 0.005; n.s. not significant).

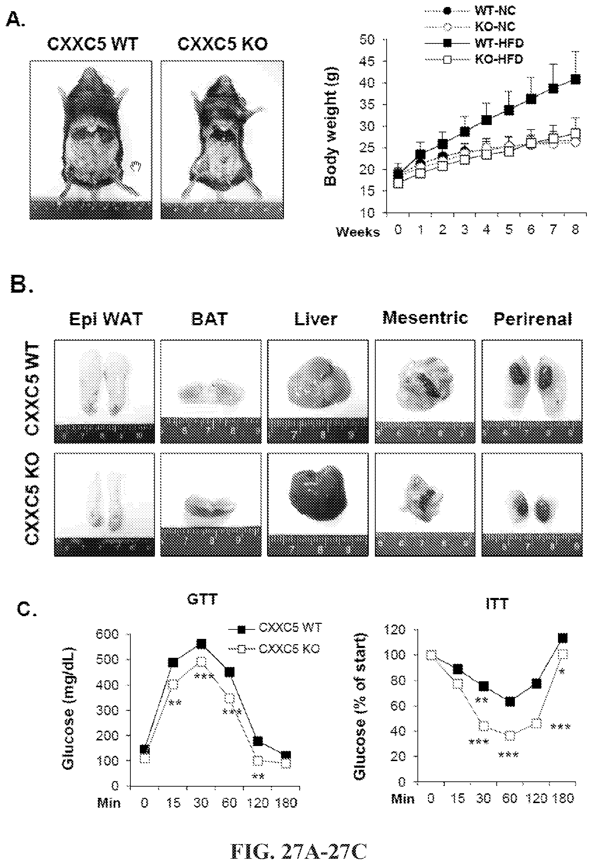

**FIG. 27A****.** Representative photographs (left) and quantitative analyses (mean ± s.e.m., n=3, ***P* < 0.005) (right) illustrating the effect of a normal diet (Chow-fed; NC) and a HFD on body weight of wild-type (WT) and CXXC5 knock out (CXXC5 KO) mice.
**FIG. 27B****.** Representative photographs illustrating the effect of HFD on organ fats of wild-type (WT) and CXXC5 knock out (CXXC5 KO) mice.
**FIG. 27C****.** Graphs illustrating the effect of HFD on glucose tolerance (GTT) and insulin tolerance (ITT) in WT (white box) and CXXC5 KO (black box) mice.

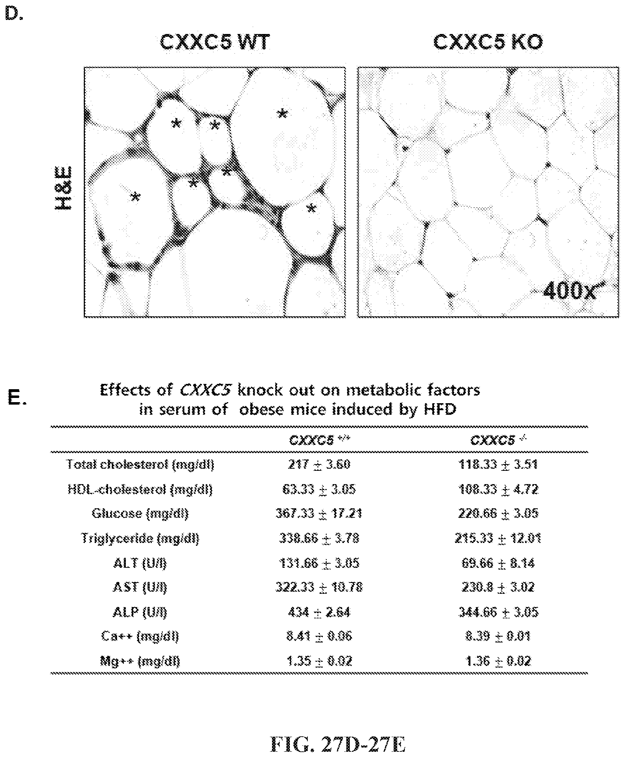

**FIG. 27D****.** H&E staining illustrating the effect of HFD on inflammation (e.g., formation of the crown like structures (CRC)) in WT and CXXC5 KO mice.
**FIG. 27E****.** Summary of data illustrating the effect of HFD on metabolic factors in WT (CXXC5^{+/+}) and CXXC5 KO (CXXC5^{-/-}) mice.

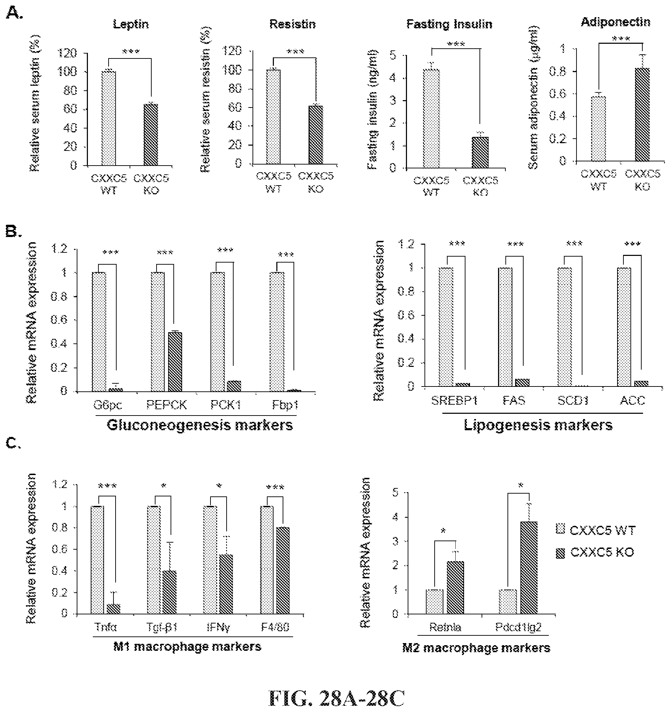

**FIG. 28A****.** Graphs illustrating the effect of HFD-induced accumulation of adipokines in serum of WT and CXXC5 KO mice (mean ± s.e.m., n=3, and ****P* < 0.005).
**FIG. 28B****.** Graphs illustrating the effect of HFD on gluconeogenesis markers and lipogenesis markers in WT (grey box) and CXXC5 KO (red box) mice (mean ± s.e.m., n=3, and ****P* < 0.005).
**FIG. 28C****.** Graphs illustrating the effect of HFD on M1 macrophage markers and M2 macrophage markers in WT (grey box) and CXXC5 KO (red box) mice (mean ± s.e.m., n=3, **P* < 0.01 and ****P* < 0.005).

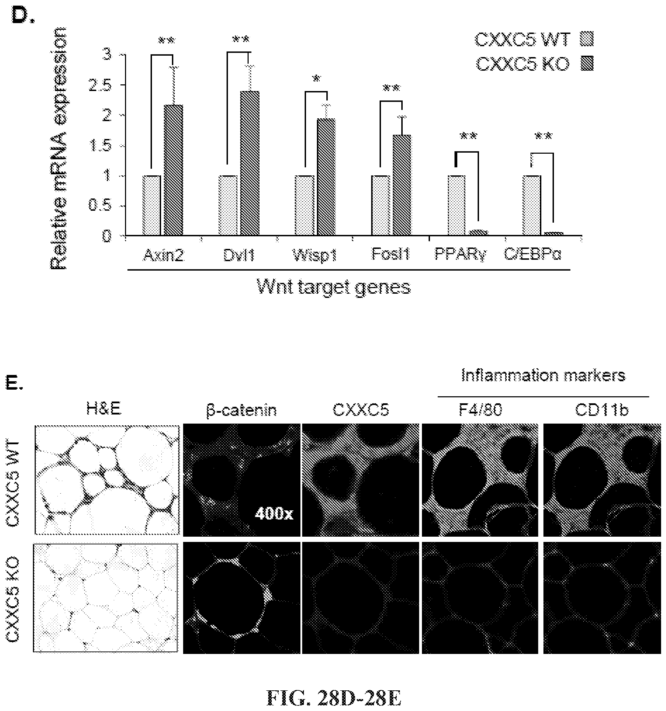

**FIG. 28D****.** Graph illustrating the effect of HFD on Wnt target gene expreesion in WT (grey box) and CXXC5 KO (red box) mice (mean ± s.e.m., n=3, **P* < 0.01 and ***P* < 0.05).
**FIG. 28E****.** H&E staining and immunohistochemical analyses illustrating the effect of HFD on inflammation in WT and CXXC5 KO mice.

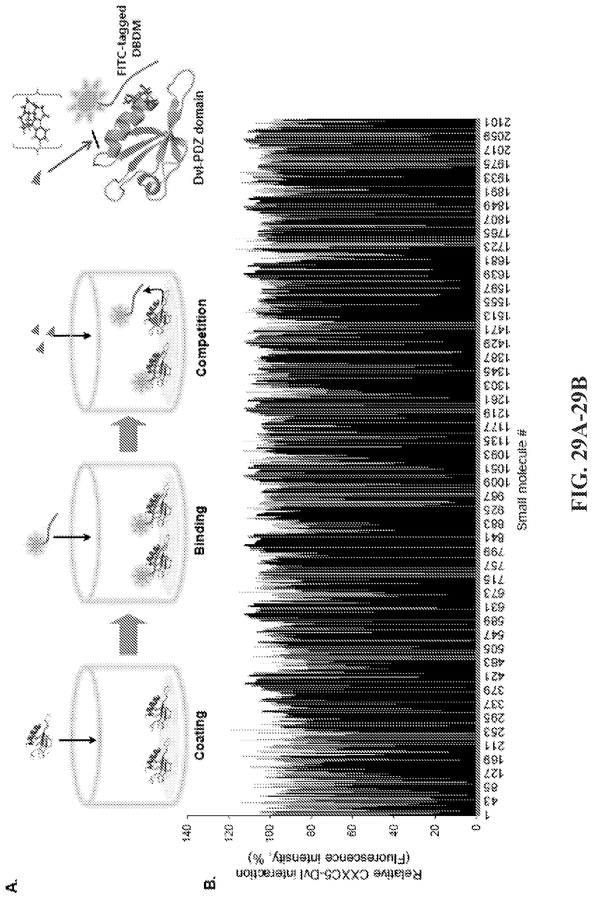

**FIG. 29A****.** Schematic diagram illustrating a *in vitro* screening system useful for the development of CXXC5-DVL PPI inhibitors.
**FIG. 29B****.** Graph showing the screening results of small molecules. An *in vitro* binding assay was performed for 2,280 compounds (30 µM) to identify inhibitors of the CXXC5-DVL interaction. The binding values were calculated by percent ratio of fluorescent intensity normalized to the DMSO-treated control.
**FIG. 30****.** Chemical structures of examples of indirubin analogs disclosed herein.

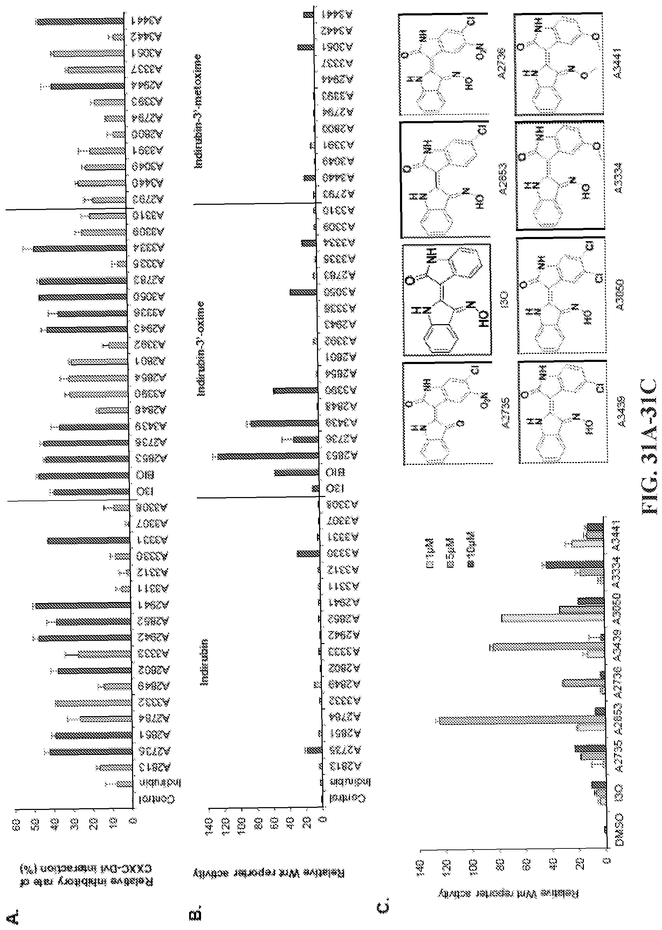

**FIG. 31A****.** Graph illustrating small molecules that exhibited higher CXXC-DVL PPI inhibition than that of indirubin-3'-oxime (I3O; a positive control).
**FIG. 31B****.** Graph illustrating small molecules that exhibited higher Wnt/β-catenin reporter activity than that of I3O (a positive control).
**FIG. 31C****.** Graph illustrating dose dependent Wnt/β-catenin reporter activities of the indicated small molecules.

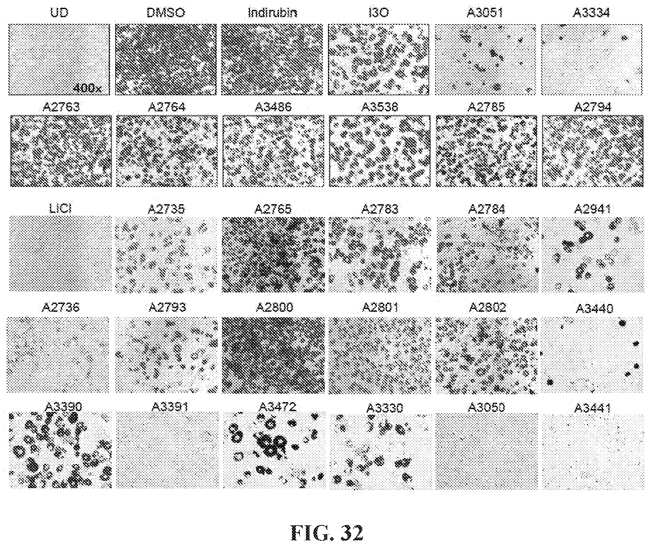

**FIG. 32****.** Immunostaining illustrating the effect of the selected small molecules on adipocyte differentiation by using 3T3L1 pre-adipocyte cells. 3T3L1 cells were treated with various small molecules at a concentration of 5 µM and the results were obtained by oil red O (ORO) staining of cells.

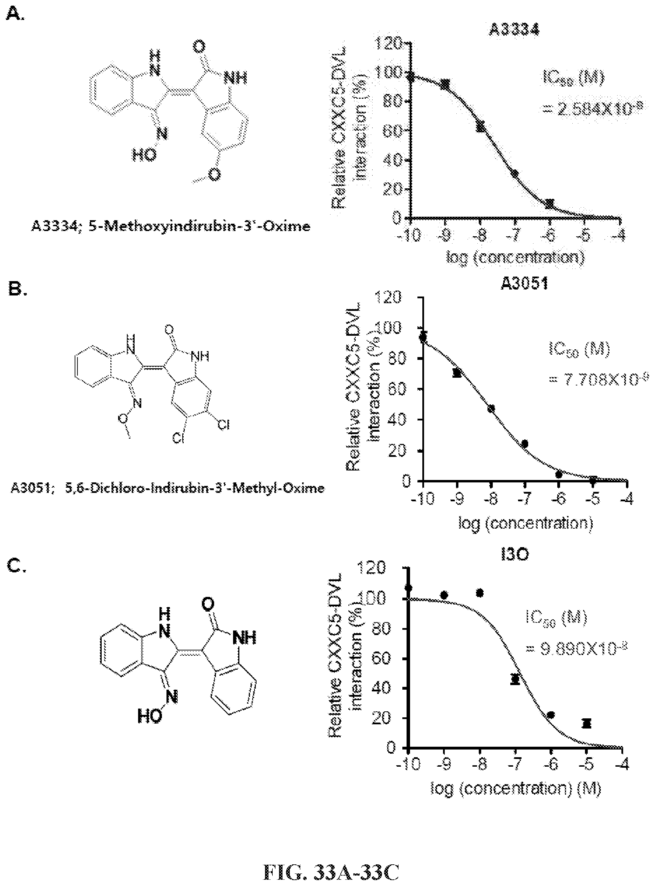

**FIG. 33A****.** Graph illustrating the concentration dependent inhibitory effect of the selected small molecule (A3334) using the *in vitro* (PTD-DBMP)-(PDZ-DVL) binding analyses.
**FIG. 33B****.** Graph illustrating the concentration dependent inhibitory effect of the selected small molecule (A3051) using the *in vitro* (PTD-DBMP)-(PDZ-DVL) binding analyses.
**FIG. 33C****.** Graph illustrating the concentration dependent inhibitory effect of the selected small molecule (I3O) using the *in vitro* (PTD-DBMP)-(PDZ-DVL) binding analyses.

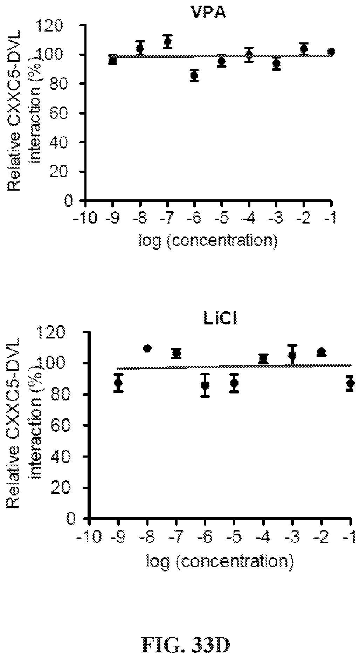

**FIG. 33D****.** Graphs illustrating the concentration dependent effect of VPA and LiCl (negative controls) using the *in vitro* (PTD-DBMP)-(PDZ-DVL) binding analyses.

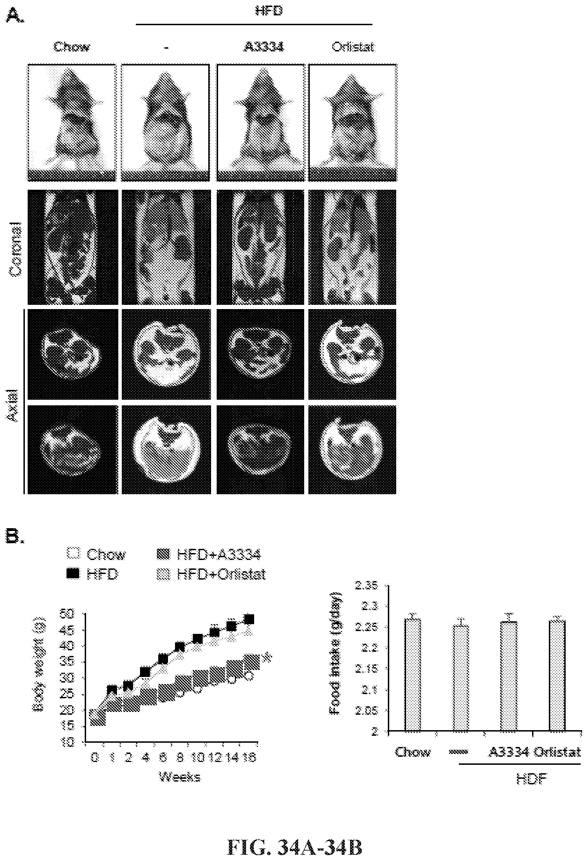

**FIG. 34A****.** Representative photographs and MRI analyses illustrating the effect of control (no treatment), A3334, and orlistat on HFD-induced obese mice compared to the untreated normal (Chow) mice.
**FIG. 34B****.** Graphs illustrating the relative effects of control (no treatment), A3334, orlistat on body weight (left) and food intake (right) of HFD-induced obese mice compared to the untreated normal (Chow) mice.

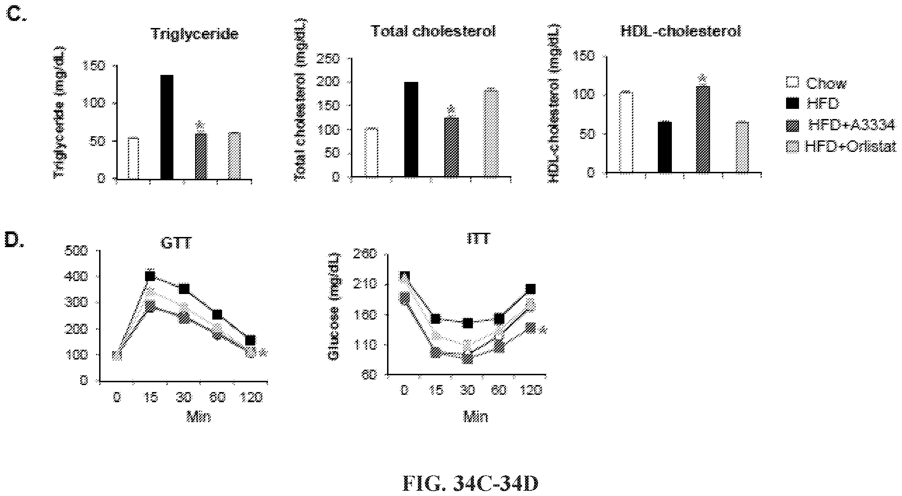

**FIG. 34C****.** Graphs illustrating the relative effects of control (no treatment), A3334, and orlistat on triglyceride, total cholesterol, and HDL-cholesterol levels in HFD-induced obese mice compared to the untreated normal (Chow) mice.
**FIG. 34D****.** Graphs illustrating the relative effects of control (no treatment), A3334, and orlistat on glucose tolerance (GTT; left) and insulin tolerance (ITT; right) in HFD-induced obese mice compared to the untreated normal (Chow) mice.

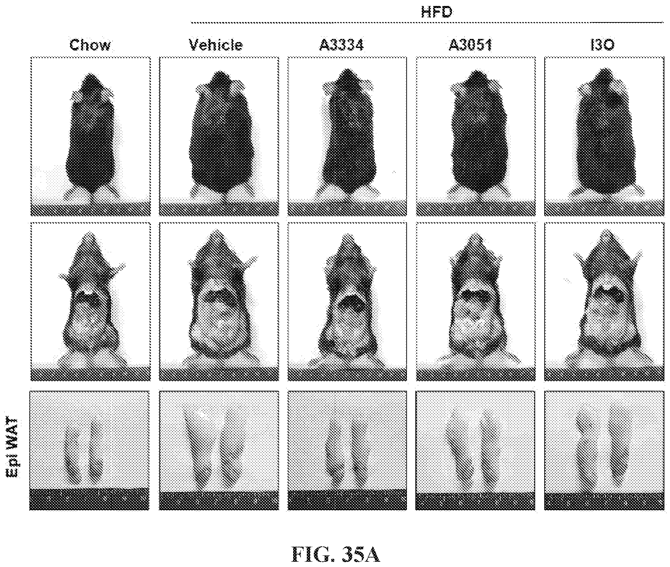

**FIG. 35A****.** Representative photographs illustrating the effect of control (vehicle), A3334, A3051, and I3O on HFD-induced obese mice compared to the untreated normal (Chow) mice.

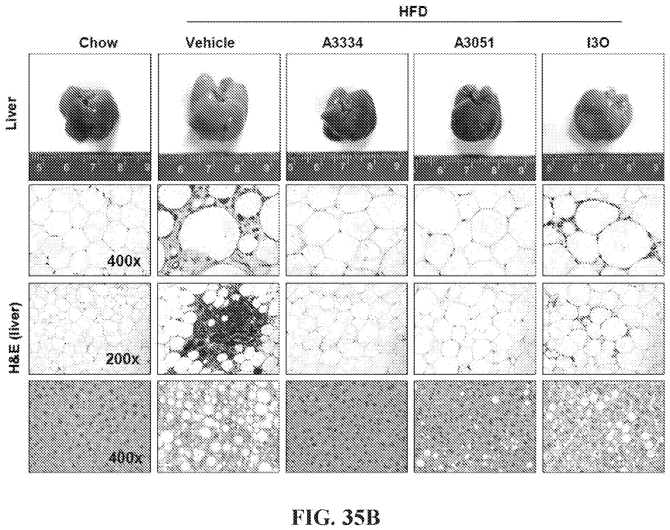

**FIG. 35B****.** Representative photographs and H&E staining of liver tissues illustrating the effect of control (vehicle), A3334, A3051, and I3O on HFD-induced obese mice compared to the untreated normal (Chow) mice.

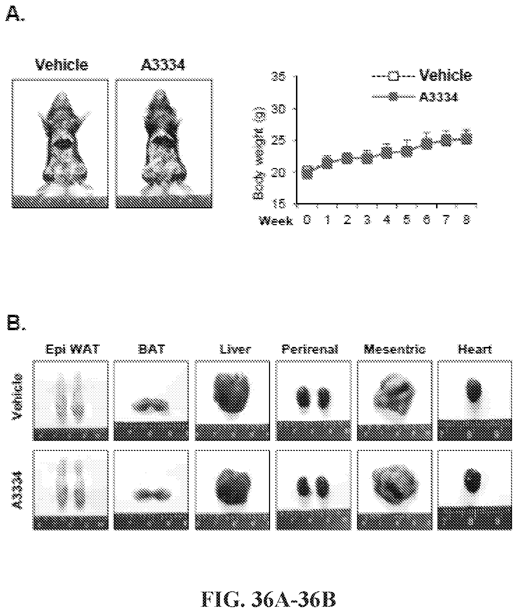

**FIG. 36A****.** Representative photographs and quantitative analyses illustrating the effect of A3334 on normal (Chow) mice. Mice were fed normal diet with or without the A3334 treatment for 8 weeks once per day at 25 mg per kg ("mpk" or "mg kg⁻¹"). The treatment with A3334 did not have any effect on normal (Chow) mice.
**FIG. 36B****.** Representative photographs of organs of normal Chow-fed (Chow) mice with or without the A3334 treatment.

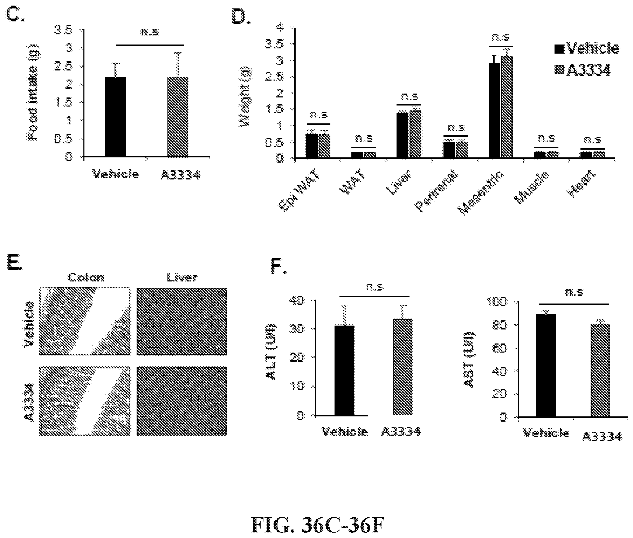

**FIG. 36C****.** Graph illustrating the effect of A3334 on food intake of normal Chow-fed (Chow) mice.
**FIG. 36D****.** Graph illustrating the effect of A3334 on the weight of indicated organs of normal (Chow) mice.
**FIG. 36E****.** Immunohistochemistry illustrating the effect of A3334 on colon and liver tissues in normal (Chow) mice.
**FIG. 36F****.** Graphs illustrating the effect of A3334 on the level of alanine transaminase (ALT) and aspartate transaminase (AST) in normal Chow-fed (Chow) mice.

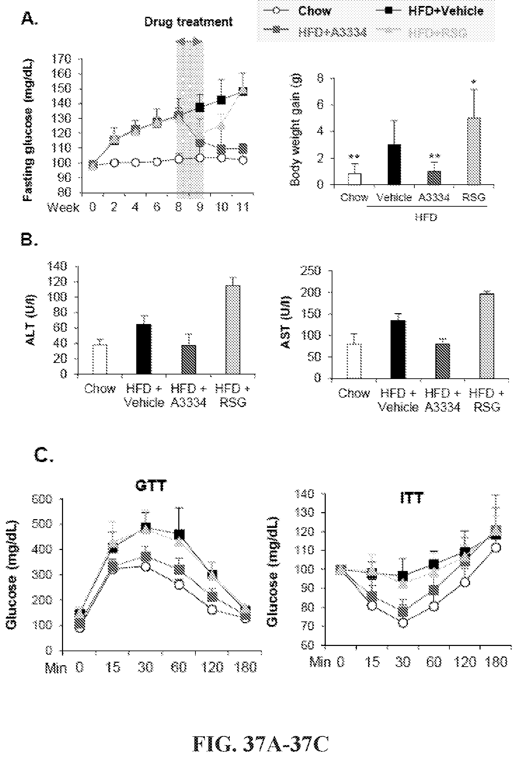

**FIG. 37A****.** Graphs illustrating the effect of control (vehicle), A3334, and rosiglitazone (RSG) on the level of fasting glucose (left) and body weight of HFD-induced diabetes mice compared to untreated normal Chow-fed (Chow) mice.
**FIG. 37B****.** Graphs illustrating the effect of control (vehicle), A3334, and rosiglitazone (RSG) on the level of alanine transaminase (ALT) and aspartate transaminase (AST) in HFD-induced diabetes mice compared to untreated normal Chow-fed (Chow) mice.
**FIG. 37C****.** Graphs illustrating the effect of control (vehicle), A3334, and rosiglitazone (RSG) on glucose tolerance (GTT; left) and insulin tolerance (ITT; right) in HFD-induced diabetes mice compared to the untreated normal Chow-fed (Chow) mice.

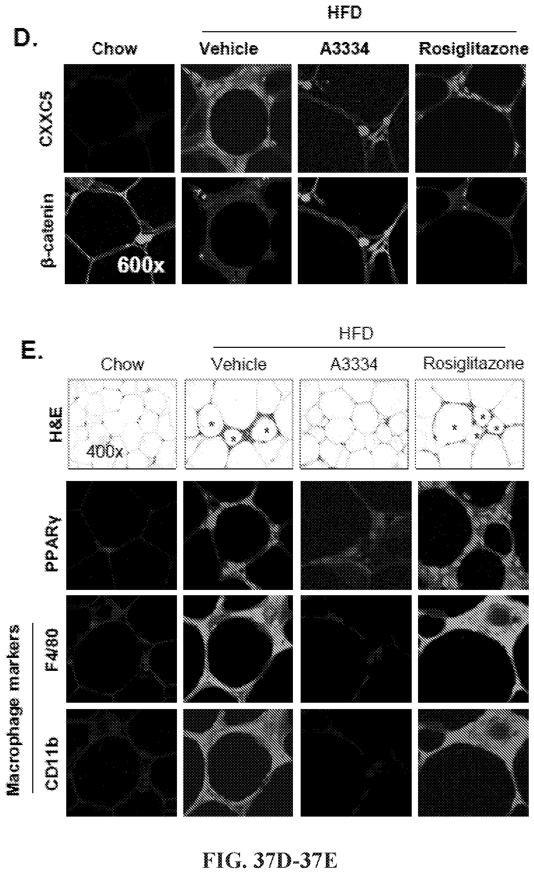

**FIG. 37D****.** Immunostaining illustrating the effect of control (vehicle), A3334, and rosiglitazone (RSG) on CXXC5 and β-catenin expression in HFD-induced diabetes mice compared to the untreated normal Chow-fed (Chow) mice.
**FIG. 37E****.** H&E and Immunostaining illustrating the effect of control (vehicle), A3334, and rosiglitazone (RSG) on the expression of PPARγ and macrophage markers in HFD-induced diabetes mice compared to the untreated normal Chow-fed (Chow) mice.

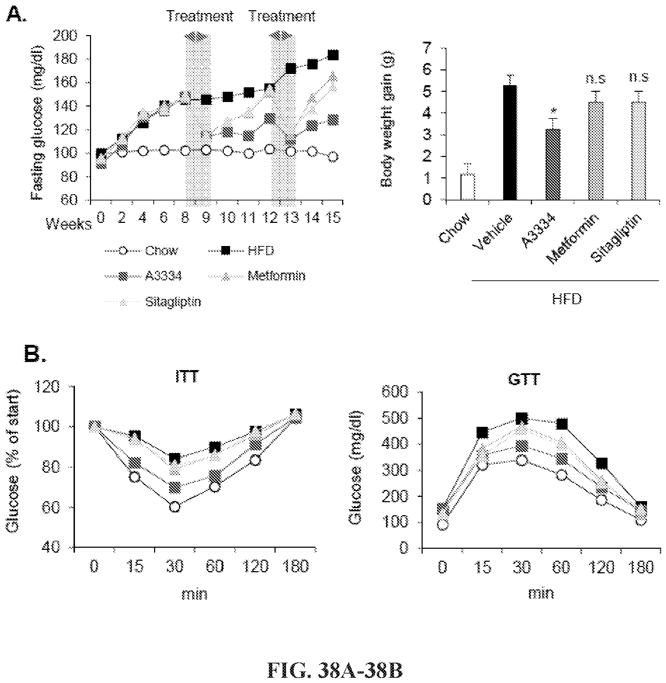

**FIG. 38A****.** Graphs illustrating the effect of control (vehicle; black box), A3334 (red box), metformin (grey triangle), and sitagliptin (yellow triangle) on the level of fasting glucose (left) and body weight of HFD-induced diabetes mice compared to untreated normal (Chow; white circle) mice.
**FIG. 38B****.** Graphs illustrating the effect of control (vehicle; black box), A3334 (red box), metformin (grey triangle), and sitagliptin (yellow triangle) on glucose tolerance (GTT; left) and insulin tolerance (ITT; right) in HFD-induced diabetes mice compared to the untreated normal (Chow; white circle) mice.

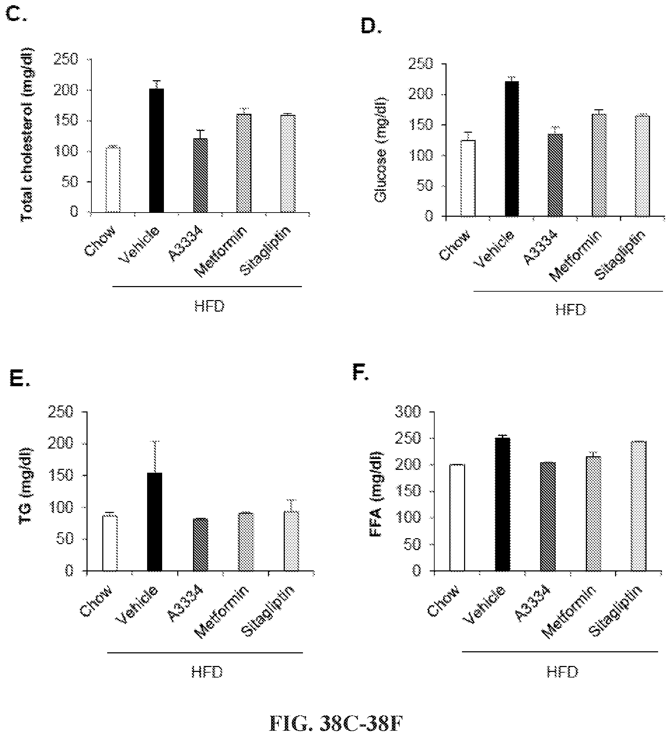

**FIG. 38C****.** Graph illustrating the effect of control (vehicle), A3334, metformin, and sitagliptin on total cholesterol levels in HFD-induced diabetes mice compared to the untreated normal (Chow) mice.
**FIG. 38D****.** Graph illustrating the effect of control (vehicle), A3334, metformin, and sitagliptin on glucose levels in HFD-induced diabetes mice compared to the untreated normal (Chow) mice.
**FIG. 38E****.** Graph illustrating the effect of control (vehicle), A3334, metformin, and sitagliptin on triglyceride (TG) levels in HFD-induced diabetes mice compared to the untreated normal (Chow) mice.
**FIG. 38F****.** Graph illustrating the effect of control (vehicle), A3334, metformin, and sitagliptin on free fatty acid (FFA) levels in HFD-induced diabetes mice compared to the untreated normal (Chow) mice.

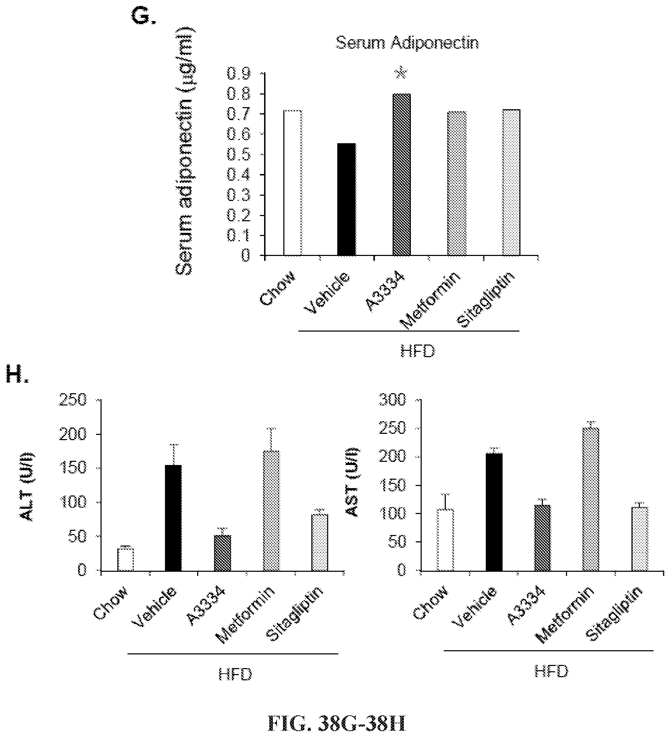

**FIG. 38G****.** Graph illustrating the effect of control (vehicle), A3334, metformin, and sitagliptin on adiponectin levels in HFD-induced diabetes mice compared to the untreated normal (Chow) mice.
**FIG. 38H****.** Graphs illustrating the effect of control (vehicle), A3334, metformin, and sitagliptin on alanine transaminase (ALT) and aspartate transaminase (AST) levels in HFD-induced diabetes mice compared to the untreated normal (Chow) mice.

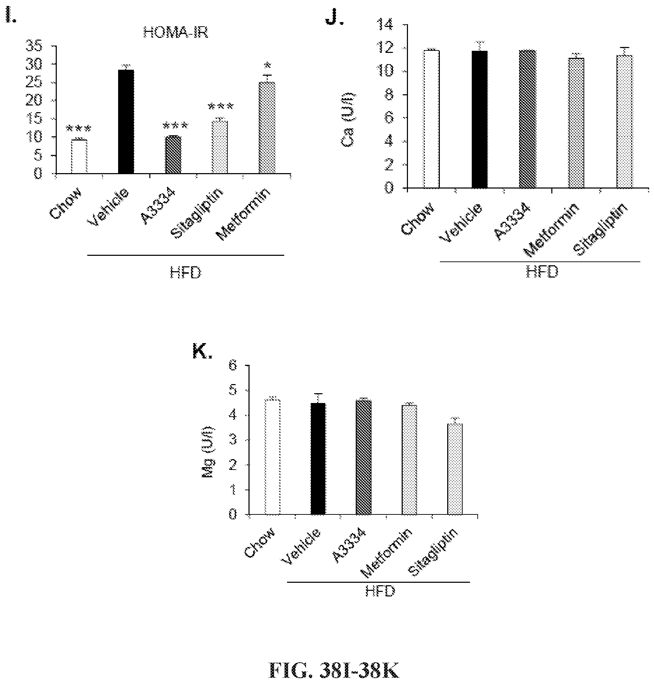

**FIG. 38I**. Graph illustrating the effect of control (vehicle), A3334, metformin, and sitagliptin on Homeostatic Model Assessment for Insulin Resistance (HOMA-IR) in HFD-induced diabetes mice compared to the untreated normal (Chow) mice.
**FIG. 38J****.** Graph illustrating the effect of control (vehicle), A3334, metformin, and sitagliptin on Ca²⁺ serum levels in HFD-induced diabetes mice compared to the untreated normal (Chow) mice.
**FIG. 38K****.** Graph illustrating the effect of control (vehicle), A3334, metformin, and sitagliptin on Mg²⁺ serum levels in HFD-induced diabetes mice compared to the untreated normal (Chow) mice.

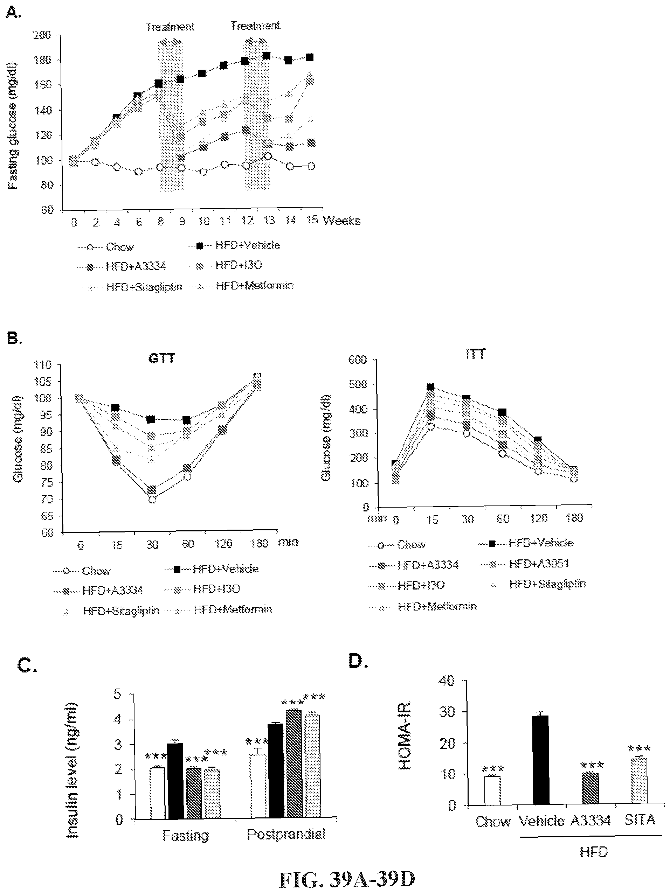

**FIG. 39A****.** Graph illustrating the effect of control (vehicle; black box), A3334 (red box), 130 (green box), metformin (grey triangle), and sitagliptin (yellow triangle) on fasting glucose serum levels in HFD-induced diabetes mice compared to untreated normal (Chow; white circle) mice.
**FIG. 39B****.** Graphs illustrating the effect of control (vehicle; black box), A3334 (red box), 130 (green box), metformin (grey triangle), and sitagliptin (yellow triangle) on glucose tolerance (GTT; left) and insulin tolerance (ITT; right) in HFD-induced diabetes mice compared to the untreated normal (Chow; white circle) mice.
**FIG. 39C****.** Graph illustrating the effect of control (vehicle; black), A3334 (red), and sitagliptin (SITA; yellow) on fasting insulin serum levels in HFD-induced diabetes mice compared to untreated normal (Chow; white) mice.
**FIG. 39D****.** Graph illustrating the effect of control (vehicle; black), A3334 (red), and sitagliptin (SITA; yellow) on Homeostatic Model Assessment for Insulin Resistance (HOMA-IR) in HFD-induced diabetes mice compared to untreated normal (Chow; white) mice.

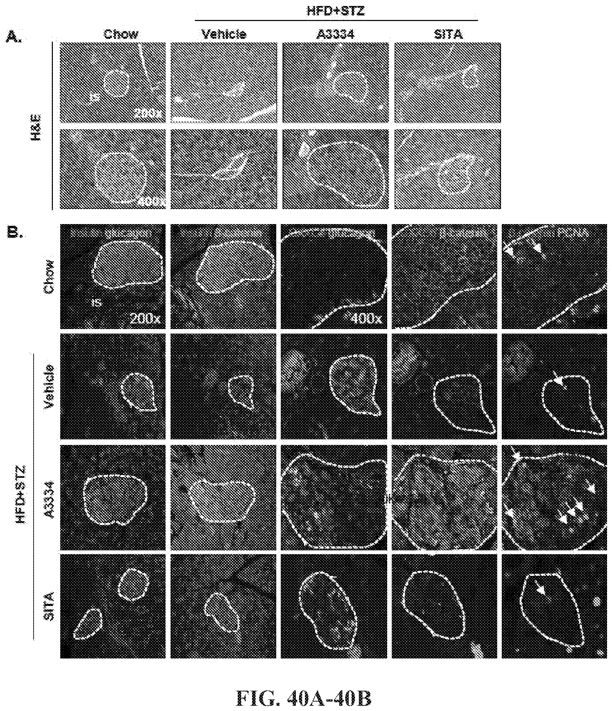

**FIG. 40A****.** H&E staining illustrating the effect of control (vehicle), A3334, and sitagliptin (SITA) on pancreas in HFD- and streptozotosin (STZ)-induced (HFD+STZ-induced) diabetes mice compared to untreated normal (Chow) mice.
**FIG. 40B****.** Immunostaining illustrating the effect of control (vehicle), A3334, and sitagliptin (SITA) on pancreas in HFD+STZ-induced diabetes mice compared to untreated normal (Chow) mice.

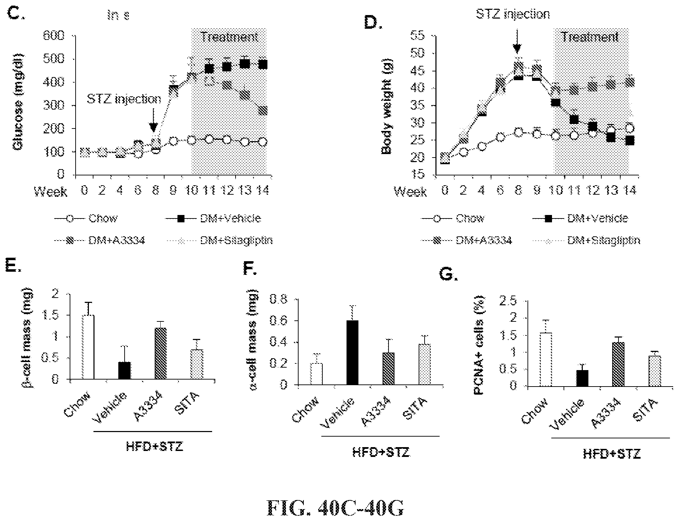

**FIG. 40C****.** Graph illustrating the effect of control (vehicle; black box), A3334 (red box), and sitagliptin (SITA; yellow triangle) on fasting glucose serum levels in HFD+STZ-induced diabetes mice compared to untreated normal (Chow; white circle) mice.
**FIG. 40D****.** Graph illustrating the effect of control (vehicle; black box), A3334 (red box), and sitagliptin (SITA; yellow triangle) on body weight of HFD+STZ-induced diabetes mice compared to untreated normal (Chow; white circle) mice.
**FIG. 40E****.** Graph illustrating the effect of control (vehicle), A3334, and sitagliptin (SITA) on β-cell mass in HFD+STZ-induced diabetes mice compared to untreated normal (Chow) mice.
**FIG. 40F****.** Graph illustrating the effect of control (vehicle), A3334, and sitagliptin (SITA) on α-cell mass in HFD+STZ-induced diabetes mice compared to untreated normal (Chow; white circle) mice.
**FIG. 40G****.** Graph illustrating the effect of control (vehicle), A3334, and sitagliptin (SITA) on PCNA⁺ cells in HFD+STZ-induced diabetes mice compared to untreated normal (Chow; white circle) mice.

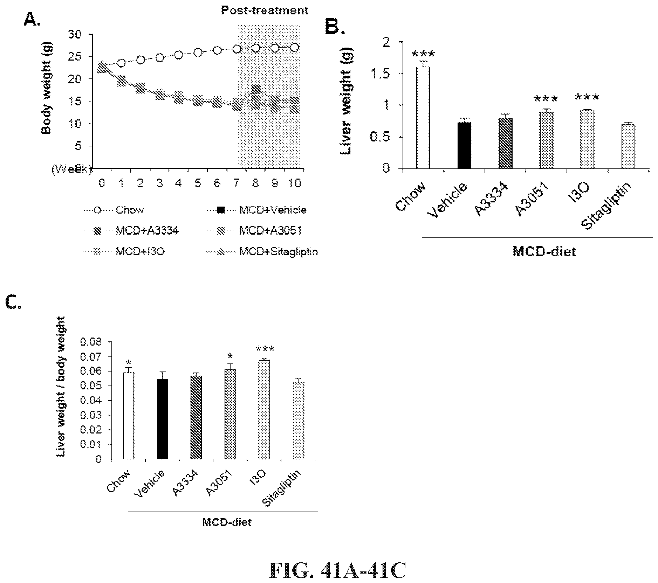

**FIG. 41A****.** Graph illustrating the effect of control (vehicle; black box), A3334 (red box), A3051(pink box), I3O (light grey box), and sitagliptin (SITA; triangle) on body weight of MCD-induced NASH mice compared to untreated normal (Chow; white circle) mice.
**FIG. 41B****.** Graph illustrating the effect of control (vehicle), A3334, A3051, I3O, and sitagliptin (SITA) on liver weight in MCD-induced NASH mice compared to untreated normal (Chow; white circle) mice.
**FIG. 41C****.** Graph illustrating the effect of control (vehicle), A3334, A3051, I3O, and sitagliptin (SITA) on ratio of liver weight/ body weight in MCD-induced NASH mice compared to untreated normal (Chow; white circle) mice.

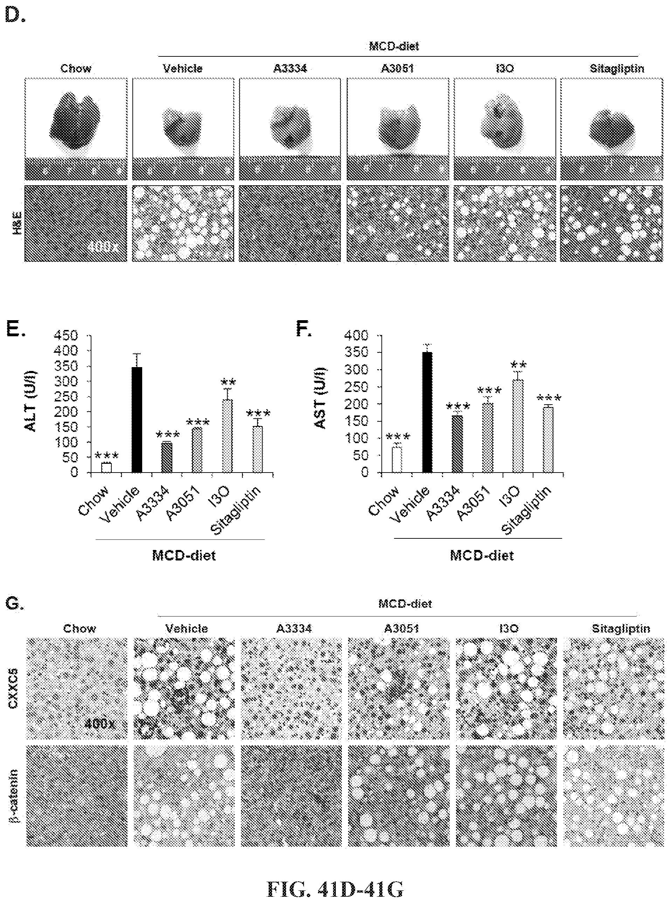

**FIG. 41D****.** Representative photographs and H&E staining illustrating the effect of control (vehicle), A3334, A3051, I3O, and sitagliptin (SITA) on liver tissues in MCD-induced NASH mice compared to untreated normal (Chow; white circle) mice.
**FIG. 41E****.** Graph illustrating the effect of control (vehicle), A3334, A3051, I3O, and sitagliptin (SITA) on alanine transaminase (ALT) levels in MCD-induced NASH mice compared to untreated normal (Chow; white circle) mice.
**FIG. 41F****.** Graph illustrating the effect of control (vehicle), A3334, A3051, I3O, and sitagliptin (SITA) on aspartate transaminase (AST) levels in MCD-induced NASH mice compared to untreated normal (Chow; white circle) mice.
**FIG. 41G****.** Immunostaining illustrating the effect of control (vehicle), A3334, A3051, I3O, and sitagliptin (SITA) on CXXC5 and β-catenin expression in liver tissues of MCD-induced NASH mice compared to untreated normal (Chow) mice.

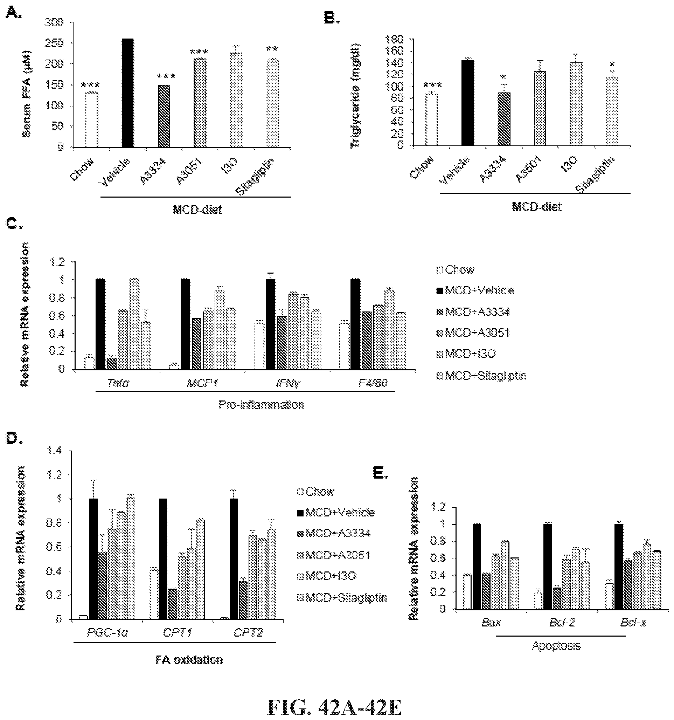

**FIG. 42A****.** Graph illustrating the effect of control (vehicle), A3334, A3051, I3O, and sitagliptin (SITA) on serum FFA levels in MCD-induced NASH mice compared to untreated normal (Chow) mice.
**FIG. 42B****.** Graph illustrating the effect of control (vehicle), A3334, A3051, I3O, and sitagliptin (SITA) on serum triglyceride levels in MCD-induced NASH mice compared to untreated normal (Chow) mice.
**FIG. 42C****.** Graph illustrating the effect of control (vehicle), A3334, A3051, I3O, and sitagliptin (SITA) on pro-inflammation markers in MCD-induced NASH mice compared to untreated normal (Chow) mice.
**FIG. 42D****.** Graph illustrating the effect of control (vehicle), A3334, A3051, I3O, and sitagliptin (SITA) on fatty acid (FA) oxidation in MCD-induced NASH mice compared to untreated normal (Chow) mice.
**FIG. 42E****.** Graph illustrating the effect of control (vehicle), A3334, A3051, I3O, and sitagliptin (SITA) on apoptosis in MCD-induced NASH mice compared to untreated normal (Chow) mice.

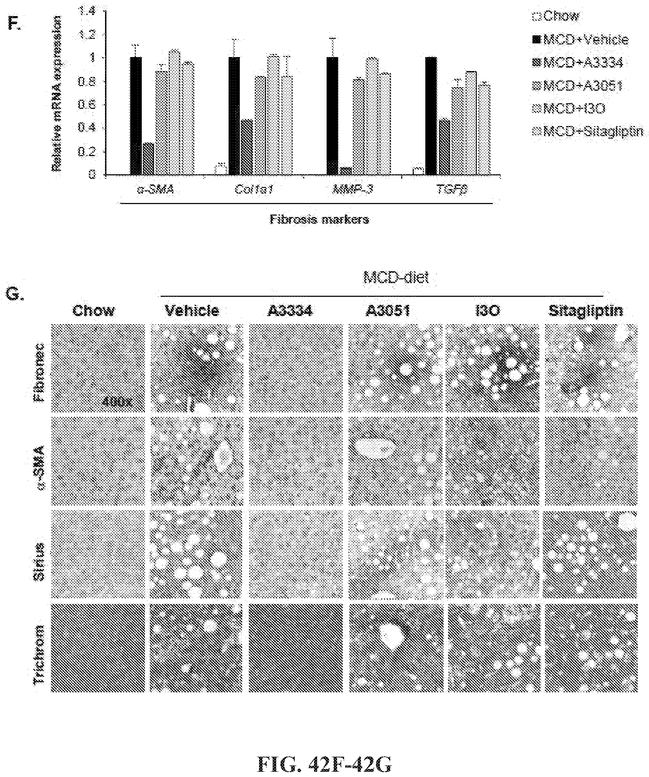

**FIG. 42F****.** Graph illustrating the effect of control (vehicle), A3334, A3051, I3O, and sitagliptin (SITA) on fibrosis in MCD-induced NASH mice compared to untreated normal (Chow) mice.
**FIG. 42G****.** Immunostaining illustrating the effect of control (vehicle), A3334, A3051, I3O, and sitagliptin (SITA) on fibrosis in MCD-induced NASH mice compared to untreated normal (Chow) mice.

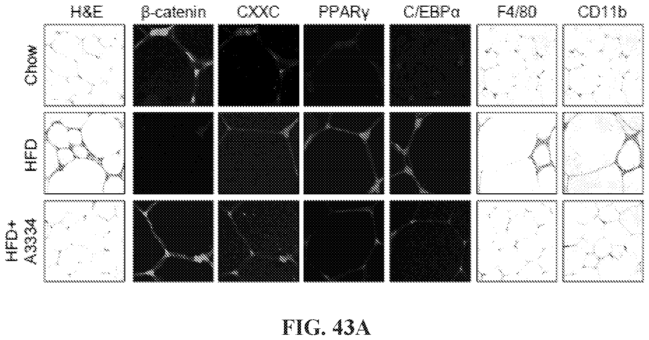

**FIG. 43A****.** H&E staining and immunostaining illustrating the effect of A3334 on the expression of the indicated markers in HFD-induced diabetes mice compared to untreated normal (Chow) mice.

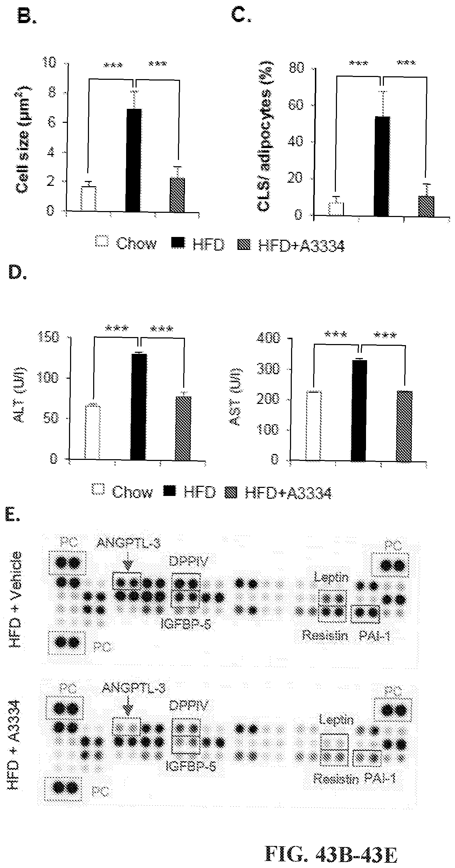

**FIG. 43B****.** Graphs illustrating the effect of A3334 on cell size in HFD-induced diabetes mice compared to untreated normal (Chow) mice.
**FIG. 43C****.** Graphs illustrating the effect of A3334 on percent CLS/adipocytes in HFD-induced diabetes mice compared to untreated normal (Chow) mice.
**FIG. 43D****.** Graphs illustrating the effect of A3334 on alanine transaminase (ALT) and aspartate transaminase (AST) levels in HFD-induced diabetes mice compared to untreated normal (Chow) mice.
**FIG. 43E****.** Protein Chip assay illustrating the effect of A3334 on various metabolic disease factors in HFD-induced diabetes mice compared to untreated normal (Chow) mice.

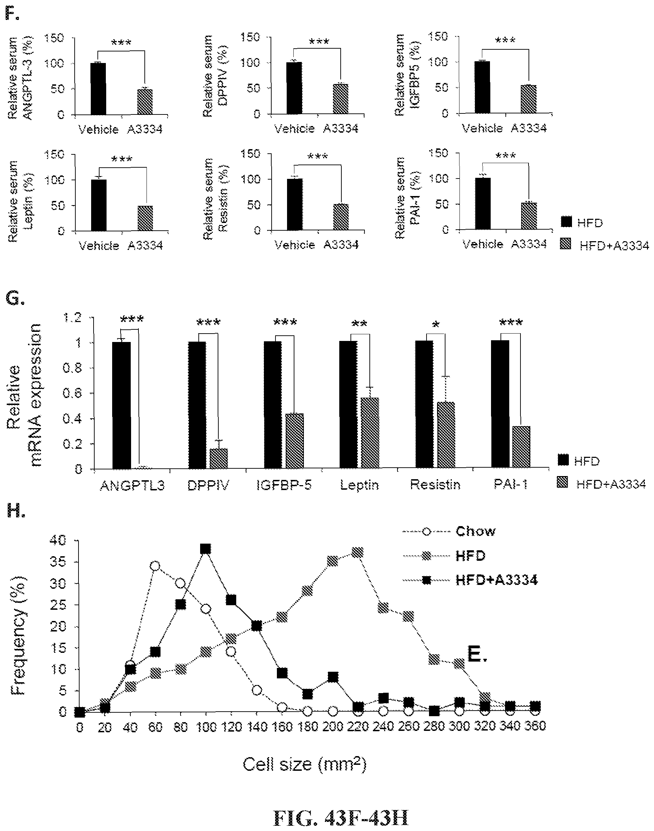

**FIG. 43F****.** Graph illustrating the effect of A3334 on the level of the indicated serum proteins in HFD-induced diabetes mice compared to untreated normal (Chow) mice.
**FIG. 43G****.** Graph illustrating the effect of A3334 on the level of the indicated proteins in adipose tissues of HFD-induced diabetes mice compared to untreated normal (Chow) mice.
**FIG. 43H****.** Graph illustrating the effect of A3334 on cell size in HFD-induced diabetes mice compared to untreated normal (Chow) mice.

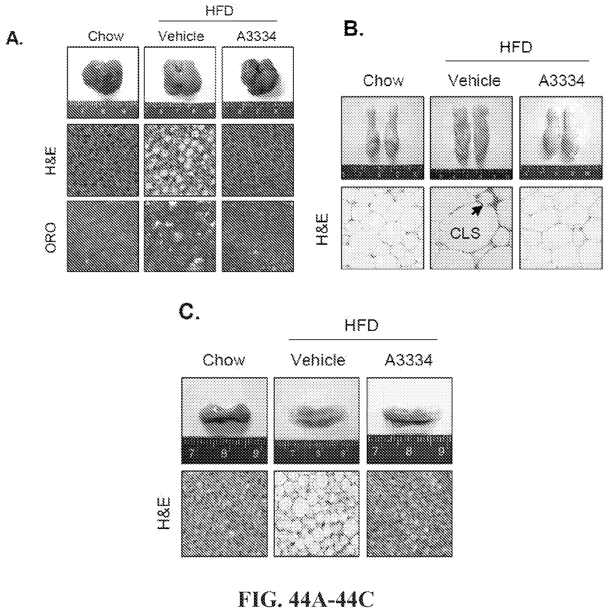

**FIG. 44A****.** H&E staining and Oil Red O (ORO) staining illustrating the effect of A3334 on liver tissues of HFD-induced diabetes mice compared to untreated normal (Chow) mice.
**FIG. 44B****.** Representative photographs and H&E staining illustrating the effect of A3334 on white adipose tissues of HFD-induced diabetes mice compared to untreated normal (Chow) mice.
**FIG. 44C****.** Representative photographs and H&E staining illustrating the effect of A3334 on brown adipose tissues of HFD-induced diabetes mice compared to untreated normal (Chow) mice.

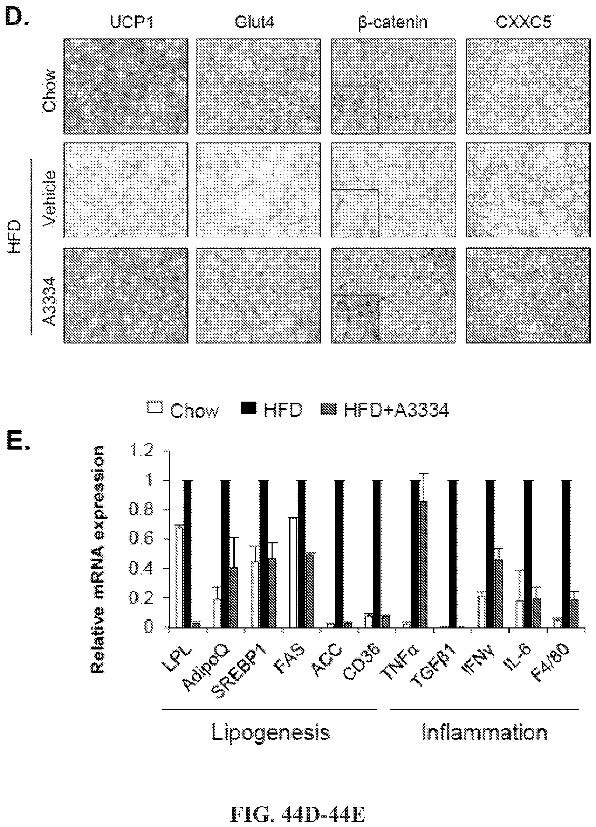

**FIG. 44D****.** Immunostaining illustrating the effect of A3334 on the expression of the indicated proteins in brown adipose tissues of HFD-induced diabetes mice compared to untreated normal (Chow) mice.
**FIG. 44E****.** Graph illustrating the effect of A3334 on lipogenesis and inflammation in white adipose tissues of HFD-induced diabetes mice compared to untreated normal (Chow) mice.

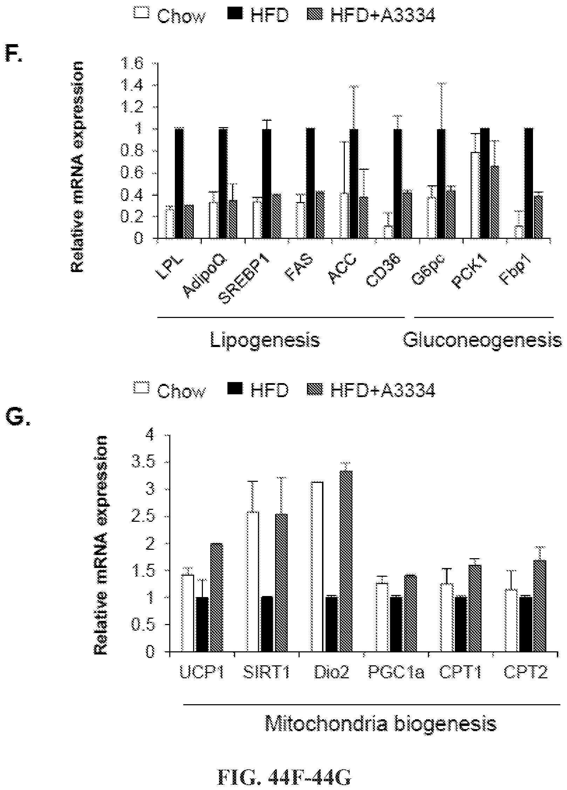

**FIG. 44F****.** Graph illustrating the effect of A3334 on lipogenesis and gluconeogenesis in liver tissues of HFD-induced diabetes mice compared to untreated normal (Chow) mice.
**FIG. 44G****.** Graph illustrating the effect of A3334 on mitochondria biogenesis in brown adipose tissues of HFD-induced diabetes mice compared to untreated normal (Chow) mice.

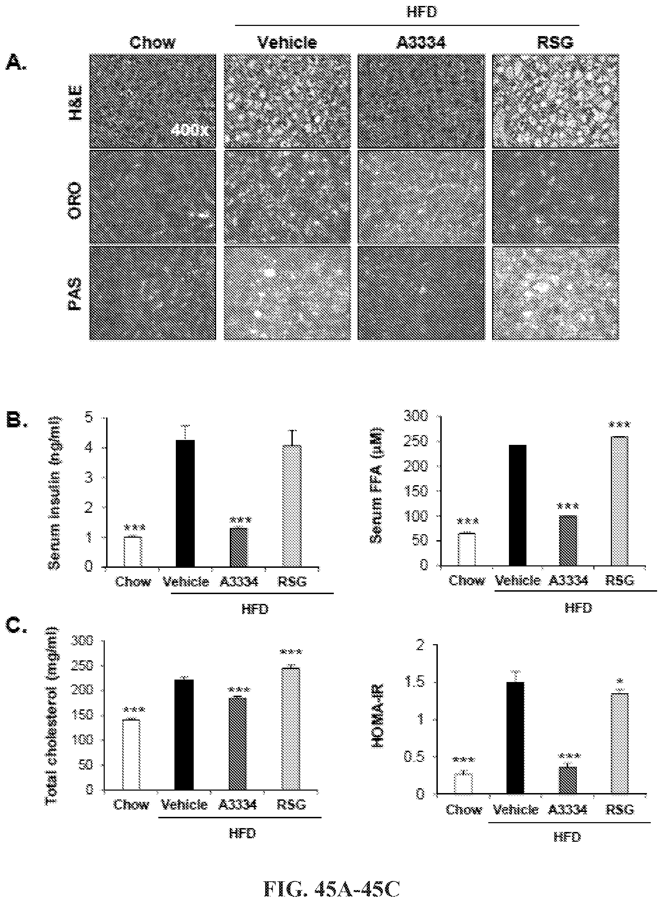

**FIG. 45A****.** H&E staining, Oil red O (ORO) staining, and Periodic Acid-Schiff (PAS) staining illustrating the effect of control (vehicle), A3334, and rosiglitazone (RSG) on liver tissues of HFD-induced diabetes mice compared to untreated normal (Chow) mice.
**FIG. 45B****.** Graphs illustrating the effect of control (vehicle), A3334, and rosiglitazone (RSG) on serum insulin levels and serum free fatty acid (FFA) levels in HFD-induced diabetes mice compared to untreated normal (Chow) mice.
**FIG. 45C****.** Graphs illustrating the effect of control (vehicle), A3334, and rosiglitazone (RSG) on total cholesterol levels and Homeostatic Model Assessment for Insulin Resistance (HOMA-IR) in HFD-induced diabetes mice compared to untreated normal (Chow) mice.

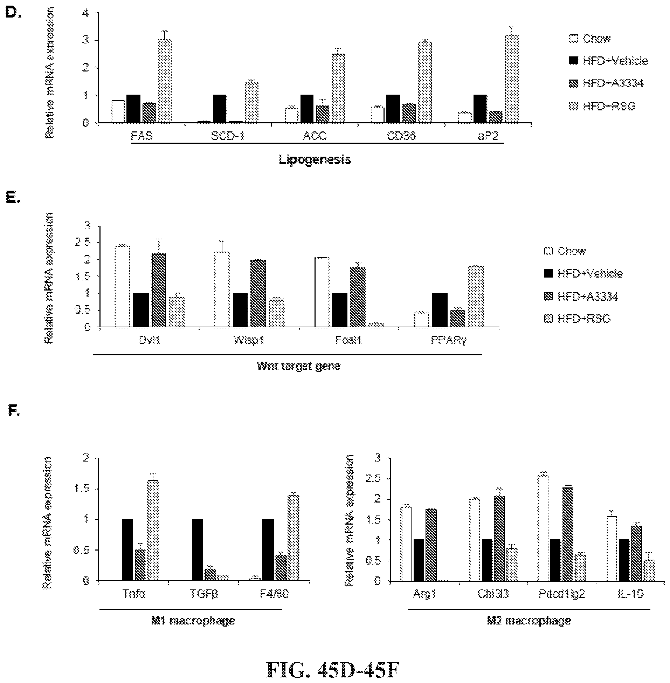

**FIG. 45D****.** Graph illustrating the effect of control (vehicle; black box), A3334 (red box), and rosiglitazone (RSG; yellow box) on lipogenesis in liver tissues of HFD-induced diabetes mice compared to untreated normal (Chow) mice.
**FIG. 45E****.** Graph illustrating the effect of control (vehicle; black box), A3334 (red box), and rosiglitazone (RSG; yellow box) on Wnt/β-catenin signaling target genes in white adipose tissues of HFD-induced diabetes mice compared to untreated normal (Chow) mice.
**FIG. 45F****.** Graphs illustrating the effect of control (vehicle; black box), A3334 (red box), and rosiglitazone (RSG; yellow box) on M1 and M2 macrophage markers in white adipose tissues of HFD-induced diabetes mice compared to untreated normal (Chow) mice.

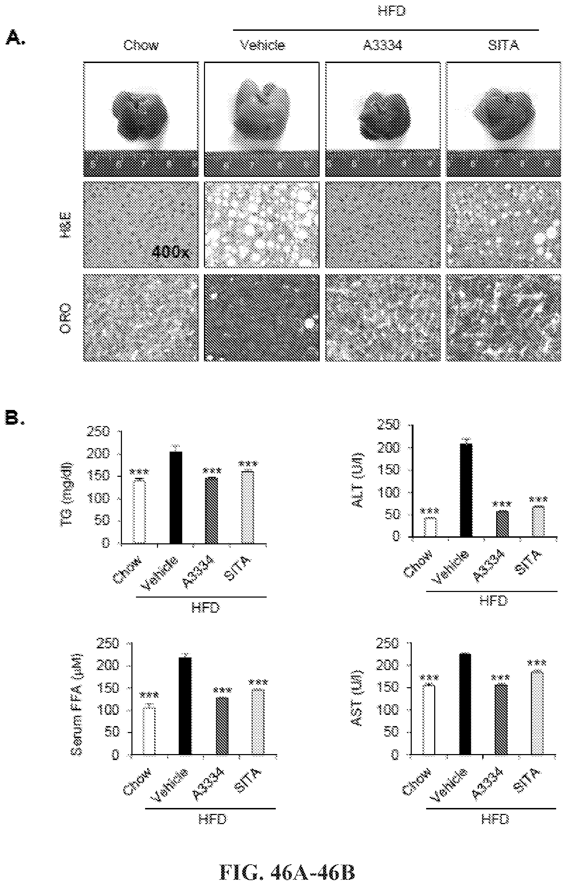

**FIG. 46A****.** H&E staining and Oil red O (ORO) staining illustrating the effect of A3334 and sitagliptin (SITA) on liver tissues of HFD-induced diabetes mice compared to untreated normal (Chow) mice.
**FIG. 46B****.** Graphs illustrating the effect of control (vehicle), A3334, and sitagliptin (SITA) on serum triglyceride levels and serum alanine transaminase (ALT) levels in HFD-induced diabetes mice compared to untreated normal (Chow) mice.
**FIG. \46C.** Graphs illustrating the effect of control (vehicle), A3334, and sitagliptin (SITA) on serum free fatty acid (FFA) levels and serum aspartate transaminase (AST) levels in HFD-induced diabetes mice compared to untreated normal (Chow) mice.

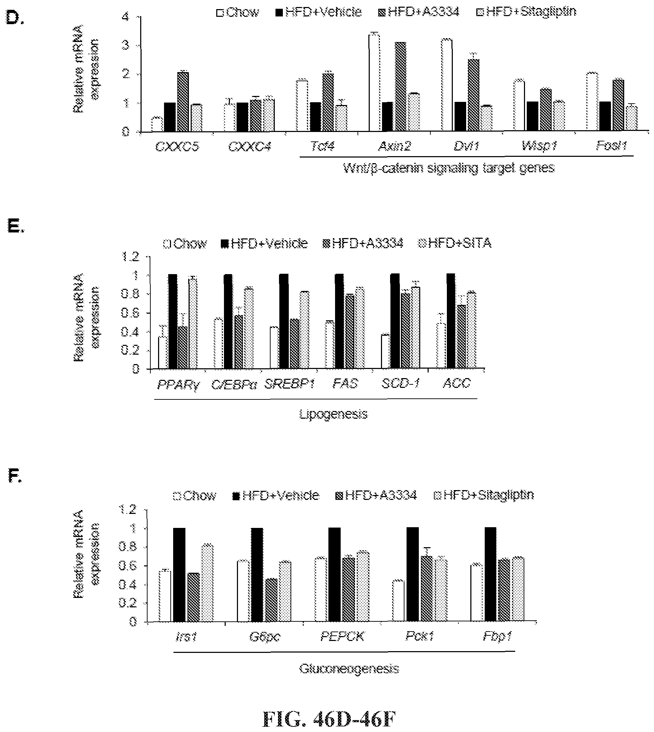

**FIG. 46D****.** Graphs illustrating the effect of control (vehicle; black box), A3334 (red box), and sitagliptin (SITA; yellow box) on Wnt/β-catenin signaling target genes in liver tissues of HFD-induced diabetes mice compared to untreated normal (Chow; white box) mice.
**FIG. 46E****.** Graphs illustrating the effect of control (vehicle; black box), A3334 (red box), and sitagliptin (SITA; yellow box) on lipogenesis in liver tissues of HFD-induced diabetes mice compared to untreated normal (Chow; white box) mice.
**FIG. 46F****.** Graphs illustrating the effect of control (vehicle; black box), A3334 (red box), and sitagliptin (SITA; yellow box) on gluconeogenesis in liver tissues of HFD-induced diabetes mice compared to untreated normal (Chow; white box) mice.

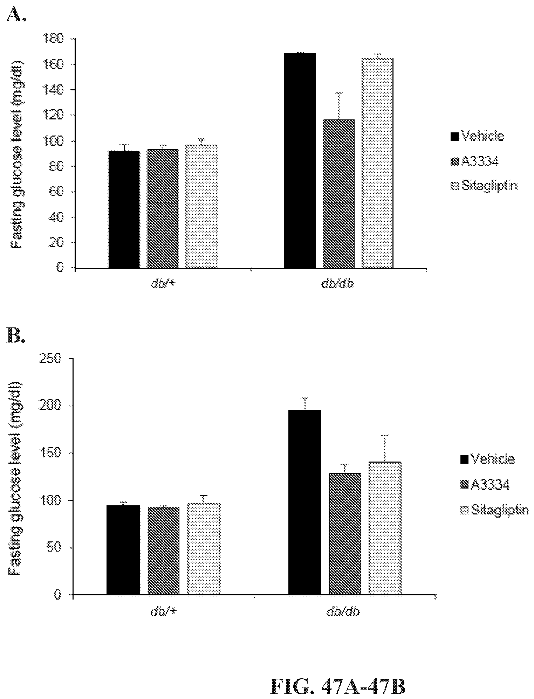

**FIG. 47A****.** Graph illustrating the effect of control (vehicle), A3334, and sitagliptin (SITA) on fasting glucose level in *db*/+ mice and *db*/*db* diabetes mice after 1 week of treatment.
**FIG. 47B****.** Graph illustrating the effect of control (vehicle), A3334, and sitagliptin (SITA) on fasting glucose level in *db*/+ mice and *db*/*db* diabetes mice after 2 weeks of treatment.

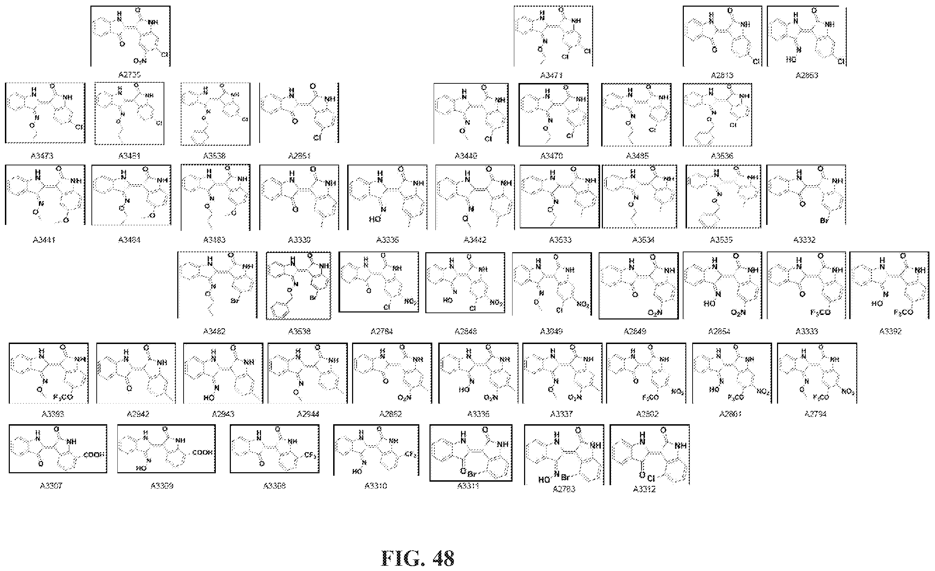

**FIG. 48****.** Chemical structures of examples of indirubin analogs disclosed herein.

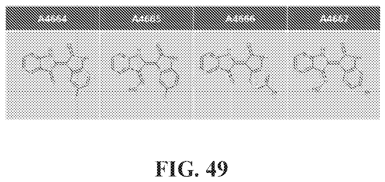

**FIG. 49****.** Chemical structures of examples of indirubin analogs disclosed herein.

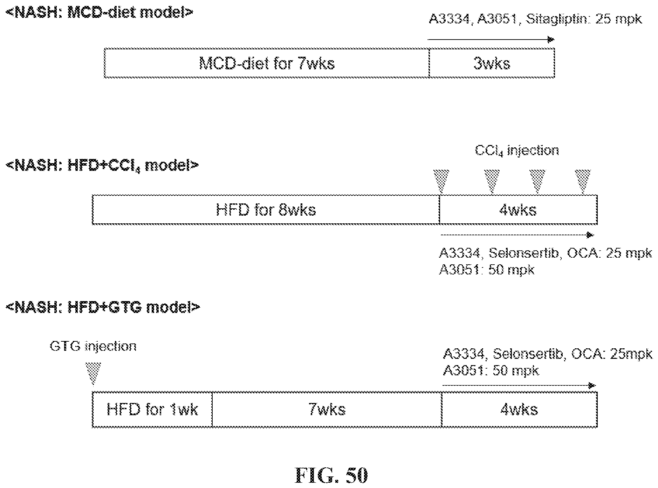

**FIG. 50****.** Schematic diagrams illustrating schemes for developing different NASH models. OCA, Ocaliva; GTG, gold thioglucose.

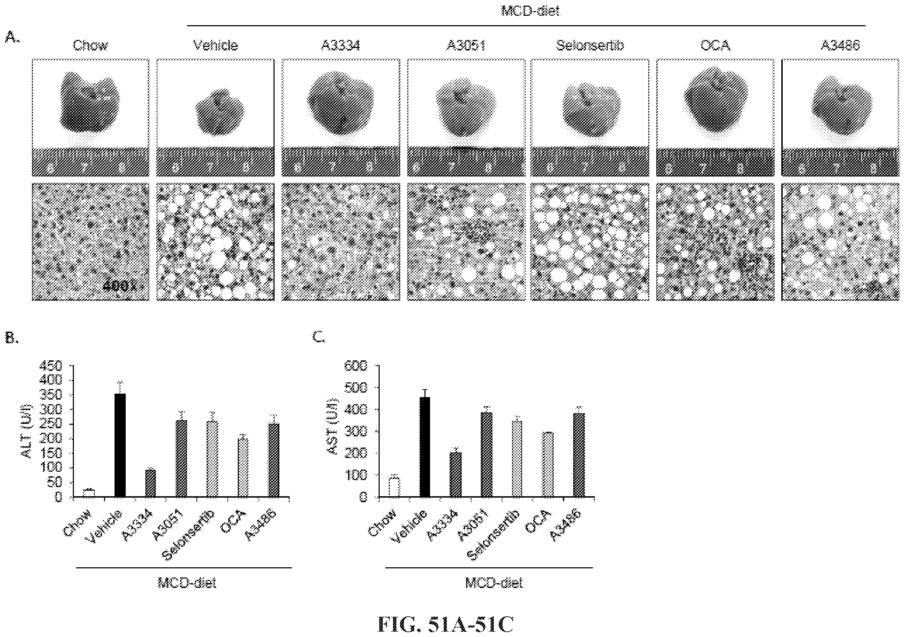

**FIG. 51A****.** H&E staining illustrating the effect of A3334, A3051, A3486, selonsertib, and OCA (ocaliva) on liver tissues of MCD diet-induced NASH mice compared to untreated normal (Chow) mice.
FIG. 51B-51D. Graphs illustrating the effect of A3334, A3051, A3486, selonsertib, and OCA (ocaliva) on ALT (**FIG. 51B**), and AST (**FIG. 51C**) in MCD diet-induced NASH mice.

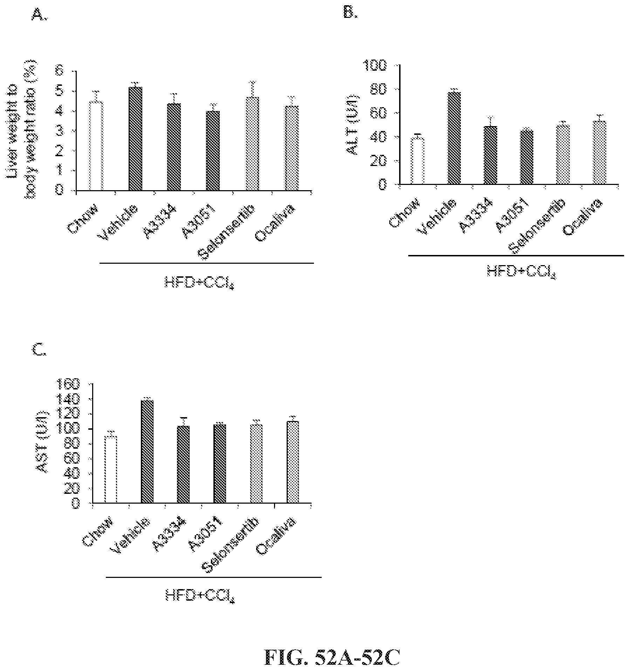

FIG. 52A-52D. Graphs illustrating the effect of A3334, A3051, selonsertib, and ocaliva on liver to body weight ratio (**FIG. 52A**), ALT (**FIG. 52B**), and AST (**FIG. 52C**) in HFD + CCl₄-induced NASH mice.

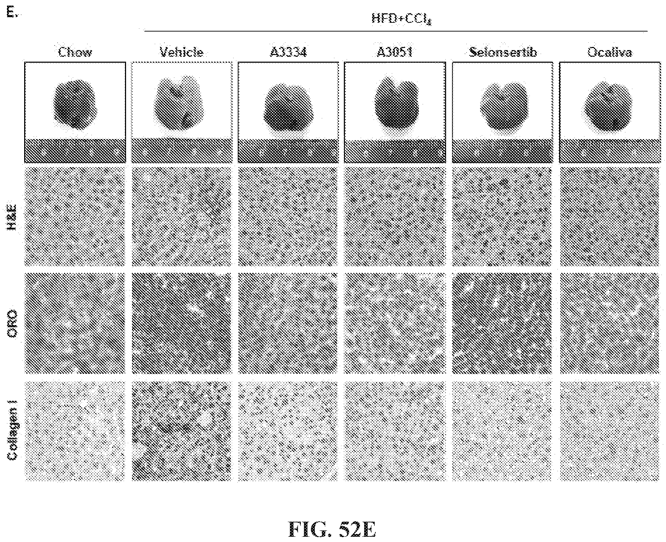

**FIG. 52E****.** H&E staining, ORO staining, and Collagen I staining illustrating the effect of A3334, A3051, selonsertib, and ocaliva on liver tissues of HFD + CCl₄-induced NASH mice compared to untreated normal (Chow) mice.

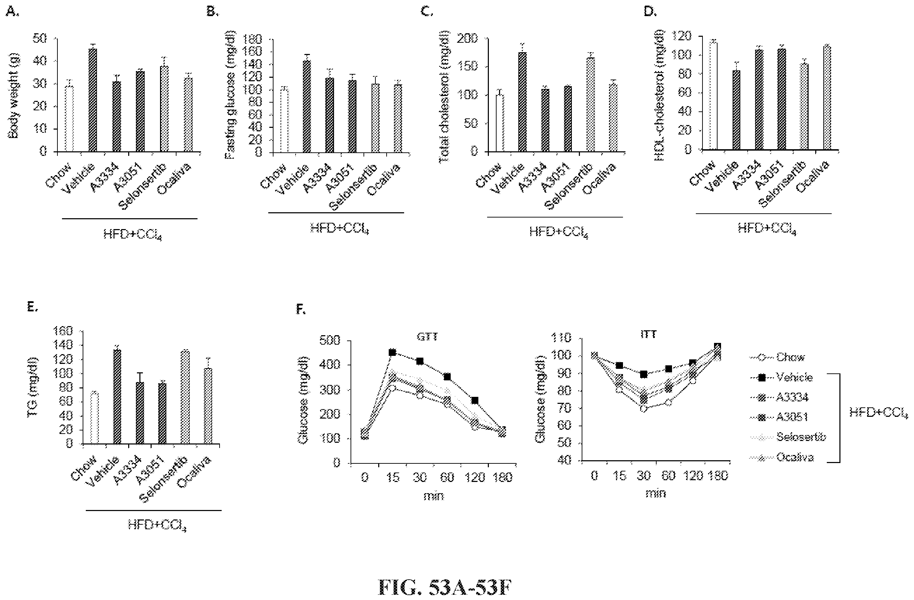

**FIG. 53A-53F****.** Graphs illustrating the effect of A3334, A3051, selonsertib, and ocaliva on body weight (**FIG. 53A**), fasting glucose (**FIG. 53B**), total cholesterol (**FIG. 53C**), HDL-cholesterol (**FIG. 53D**), TG (**FIG. 53E**) and glucose and insulin tolerance (**FIG. 53F**) in HFD + CCl₄-induced NASH mice.

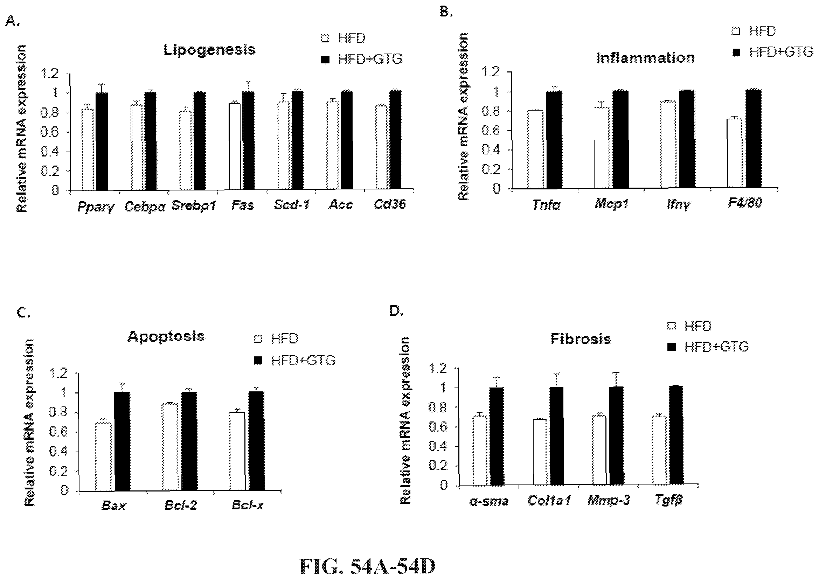

**FIG. 54A-54D****.** Graphs illustrating the characterization of HFD+GTG induced NASH model by measuring relative mRNA expression of lipogenesis markers (**FIG. 54A**), inflammatory markers (**FIG. 54B**), apoptosis markers (**FIG. 54C**), and fibrosis markers (**FIG. 54D**) in HFD+GTG induced NASH mice.

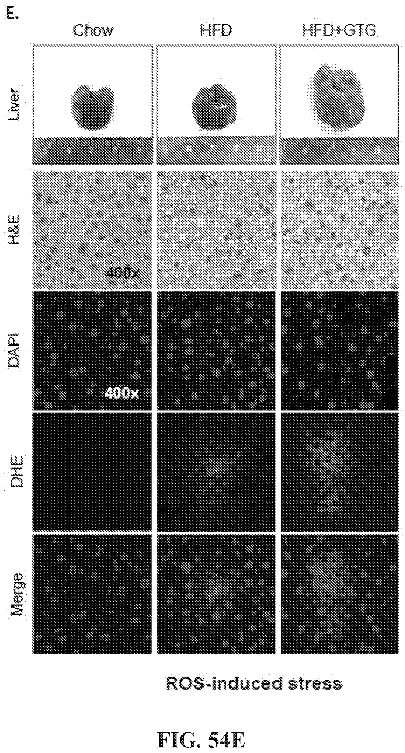

**FIG. 54E****.** H&E staining, DAPI staining, and DHE (dihydroethidium: ROS detection) staining illustrating the effect of HFD induced mice and HFD + GTG-induced NASH mice compared to untreated normal (Chow) mice.

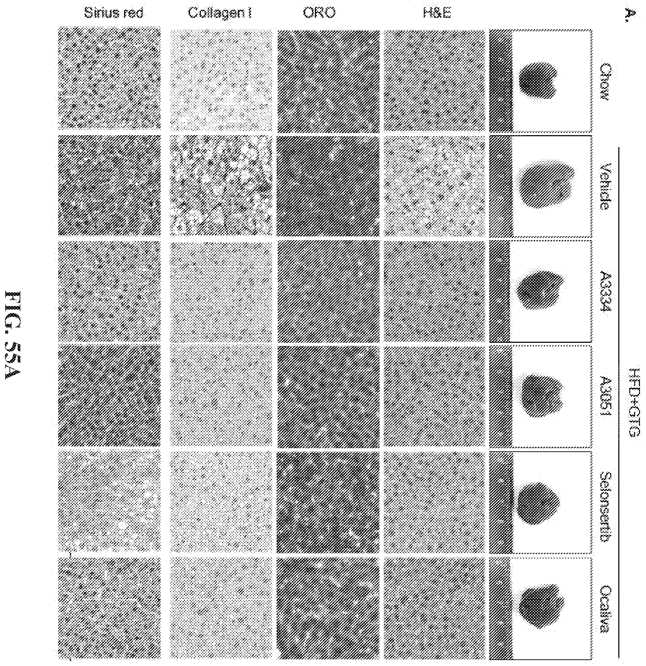

**FIG. 55A****.** H&E staining, ORO staining, Collagen I, and Sirius red staining illustrating the effect of A3334, A3051, selonsertib, and ocaliva on liver tissues of HFD + GTG-induced NASH mice compared to untreated normal (Chow) mice.

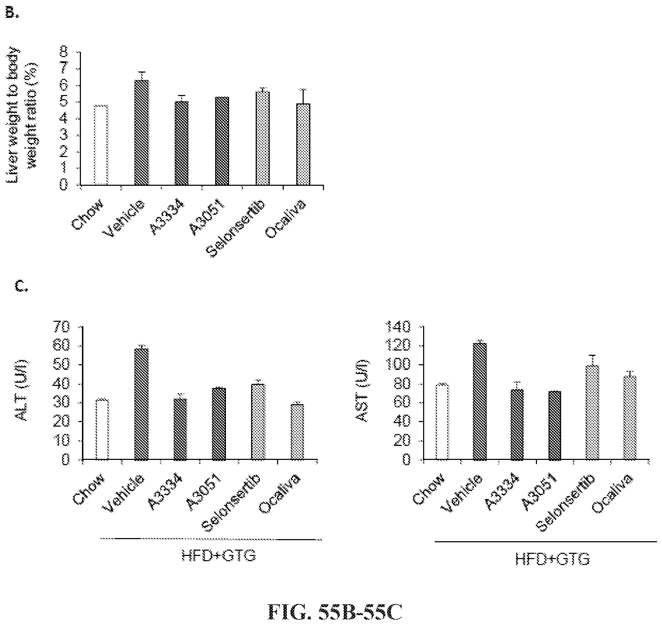

**FIG. 55B-55C****.** Graphs illustrating effect of A3334, A3051, selonsertib, and ocaliva on liver to body weight ratio (**FIG. 55B**) and ALT and AST (**FIG. 55C**) in HFD + GTG-induced NASH mice.

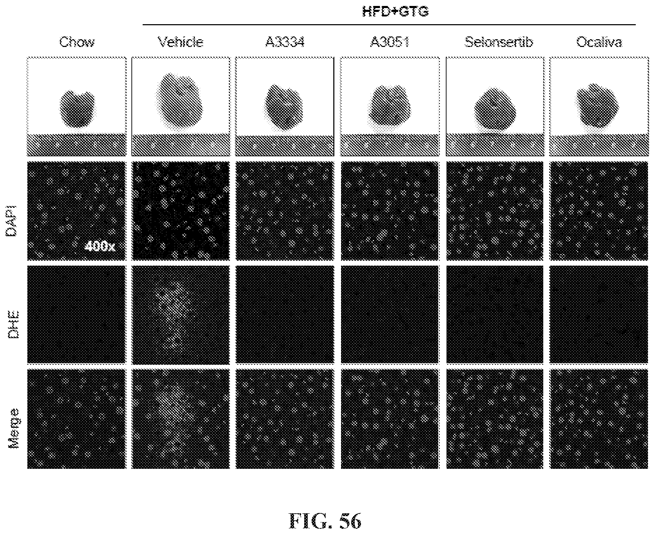

**FIG. 56****.** H&E staining and DHE (dihydroethidium: ROS detection) staining illustrating the effect of HFD induced mice and HFD + GTG-induced NASH mice compared to untreated normal (Chow) mice.

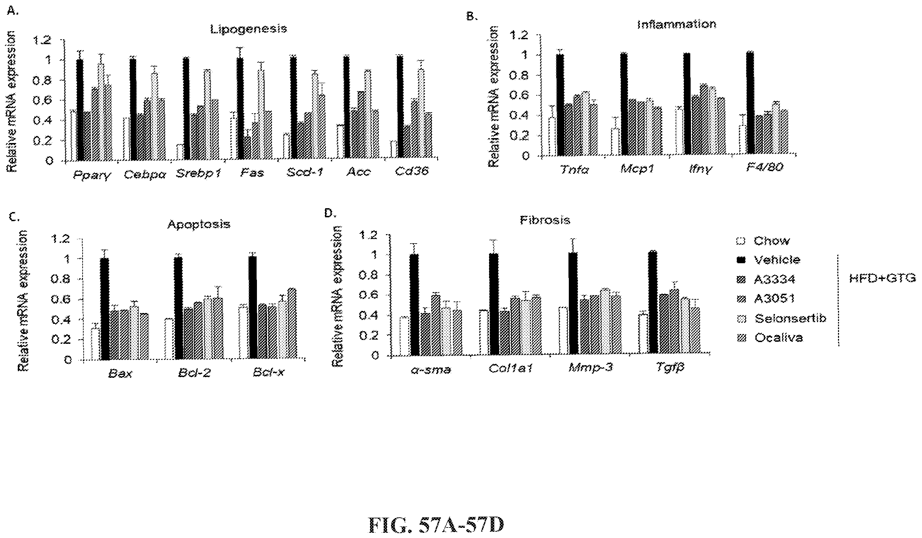

**FIG. 57A-57D****.** Graphs illustrating the effect of A3334, A3051, selonsertib, and ocaliva on relative mRNA expression of lipogenesis markers (**FIG. 57A**), inflammation markers (**FIG. 57B**), apoptosis markers (**FIG. 57C**), and fibrosis markers (**FIG. 57D**) in HFD+GTG induced NASH mice.

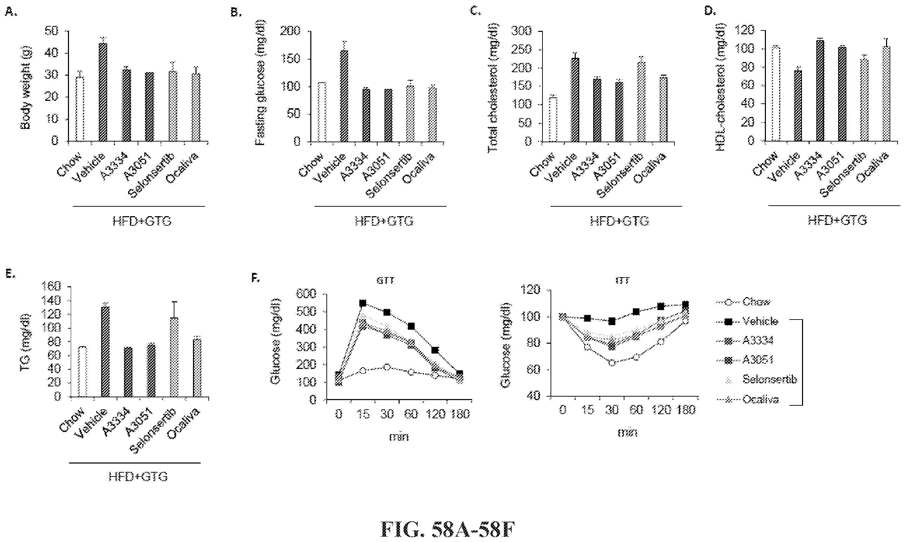

**FIG. 58A-58F****.** Graphs illustrating the effect of A3334, A3051, selonsertib, and ocaliva on body weight (**FIG. 58A**), fasting glucose (**FIG. 58B**), total cholesterol (**FIG. 58C**), HDL-cholesterol (**FIG. 58D**), TG (**FIG. 58E**) and glucose and insulin tolerance (**FIG. 58F**) in HFD + GTG-induced NASH mice.

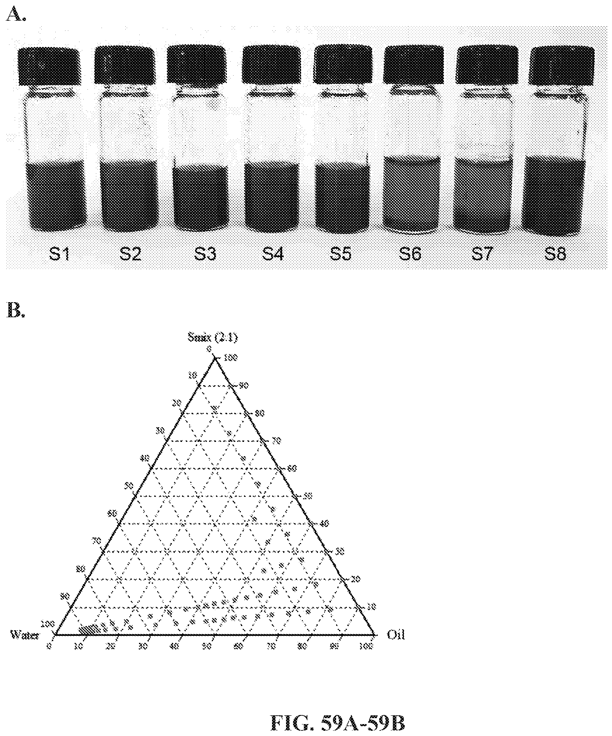

**FIG. 59A****.** Photograph of emulsion solution made by dissolving A3334 of the present disclosure in oil or surfactant.
**FIG. 59B****.** Ternary diagram of the emulsion solution made by dissolving A3334 of the present disclosure using surfactants, Tween 80 and PEG 400, in a ratio of 2:1.

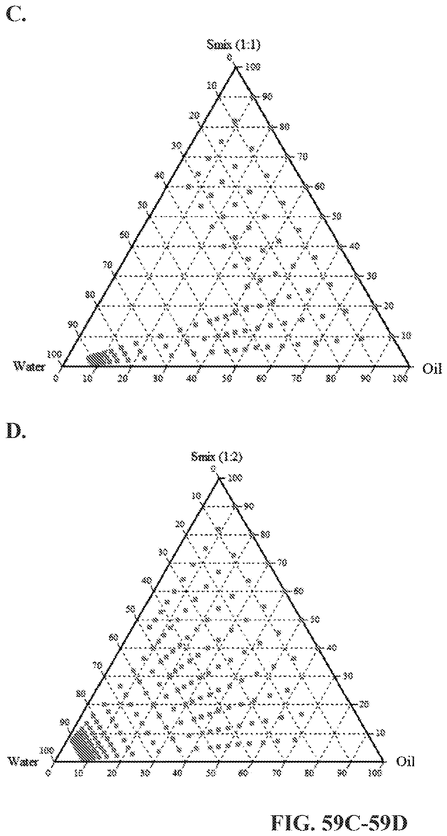

**FIG. 59C****.** Ternary diagram of the emulsion solution made by dissolving A3334 of the present disclosure using surfactants, Tween 80 and PEG 400, in a ratio of 1:1.
**FIG. 59D****.** Ternary diagram of the emulsion solution made by dissolving A3334 of the present disclosure using surfactants, Tween 80 and PEG 400, in a ratio of 1:2.

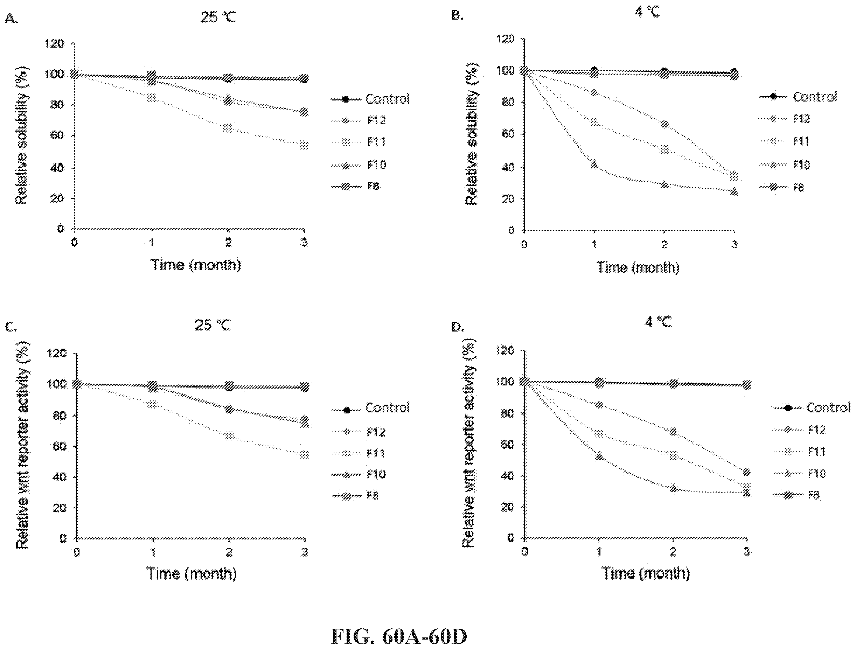

**FIG. 60A-60B****.** Graphs illustrating relative solubility of emulsion solutions (F8, F10, F11, and F12) at room temperature, 25 °C (**FIG. 60A**) or at a low temperature, 4 °C (**FIG. 60B**).
**FIG. 60C-60D****.** Graphs illustrating relative Wnt/β-catenin reporter activity of emulsion solutions (F8, F10, F11, and F12) at room temperature, 25 °C (**FIG. 60C**) or at a low temperature, 4 °C (**FIG. 60D**).

### BRIEF DESCRIPTION OF SEQUENCES

**SEQ ID NO. 1.** CAAGAAGAAGCGGAAACGCTGC
**SEQ ID NO. 2.** TCTCCAGAGCAGCGGAAGGCTT
**SEQ ID NO. 3.** CAACCCAGCCAAGAAGAAGA
**SEQ ID NO. 4.** AGATCTGGTGTCCCGTTTTG
**SEQ ID NO. 5.** TGTGTACCCAATCACGACAGGAG
**SEQ ID NO. 6.** GATTCCGGTCGTGTGCAGAG
**SEQ ID NO. 7.** TGGAGAGTGAGCGGCAGAGC
**SEQ ID NO. 8.** TGGAGACGAGCGGGCAGA
**SEQ ID NO. 9.** CCTTCCATCCAAATGTTGC
**SEQ ID NO. 10.** GTGACTGACCATAGACTCTGTGC
**SEQ ID NO. 11.** ATCGCCCGAGGTACGCAATAGG
**SEQ ID NO. 12.** CAGCCCACCGTGCCATCAATG
**SEQ ID NO. 13.** AACCGGAGGAAGGAACTGAC
**SEQ ID NO. 14.** AACCGGAGGAAGGAACTGAC
**SEQ ID NO. 15.** TGTGGGGATAAAGCATCAGGC
**SEQ ID NO. 16.** CCGGCAGTTAAGATCACACCTAT
**SEQ ID NO. 17.** GGTGGACAAGAACAGCAACGA
**SEQ ID NO. 18.** TGTCCAGTTCACGGCTCAGCT
**SEQ ID NO. 19.** GGAGCCATGGATTGCACATT
**SEQ ID NO. 20.** GGCCCGGGAAGTCACTGT
**SEQ ID NO. 21.** GCGATGAAGAGCATGGTTTAG
**SEQ ID NO. 22.** GGCTCAAGGGTTCCATGTT
**SEQ ID NO. 23.** CTGTACGGGATCATACTGGTTC
**SEQ ID NO. 24.** GCCGTGCCTTGTAAGTTCTG
**SEQ ID NO. 25.** CCTCCGTCAGCTCAGATACA
**SEQ ID NO. 26.** TTTACTAGGTGCAAGCCAGACA
**SEQ ID NO. 27.** CGGAGTCCGGGCAGGT
**SEQ ID NO. 28.** GCTGGGTAGAGAATGGATCA
**SEQ ID NO. 29.** TGACGTCACTGGAGTTGTACGG
**SEQ ID NO. 30.** GGTTCATGTCATGGATGGTGC
**SEQ ID NO. 31.** TCAAGTGGCATAGATGTGGAAGAA
**SEQ ID NO. 32.** TGGCTCTGCAGGATTTTCATG
**SEQ ID NO. 33.** CTTTGGCTATGGGCTTCCAGTC
**SEQ ID NO. 34.** GCAAGGAGGACAGAGTTTATCGTG
**SEQ ID NO. 35.** ACTGAAGCCAGCTCTCTCTTCCTC
**SEQ ID NO. 36.** TTCCTTCTTGGGGTCAGCACAGAC
**SEQ ID NO. 37.** CTCCAAGCCAAAGTCCTTAGAG
**SEQ ID NO. 38.** GGAGCTGTCATTAGGGACATCA
**SEQ ID NO. 39.** CAGGTCTGGCAATTCTTCTGAA
**SEQ ID NO. 40.** GTCTTGCTCATGTGTGTAAGTGA
**SEQ ID NO. 41.** CCAATCCAGCTAACTATCCCTCC
**SEQ ID NO. 42.** ACCCAGTAGCAGTCATCCCA
**SEQ ID NO. 43.** TTGTCGGTGTGATTGGCTTC
**SEQ ID NO. 44.** AAAAGGCAGCACACAGTTGC
**SEQ ID NO. 45.** GCTATGCTGCCTGCTCTTACT
**SEQ ID NO. 46.** CCTGCTGATCCTCATGCCA
**SEQ ID NO. 47.** GGACTTGAGCTATGACACGGG
**SEQ ID NO. 48.** GCCAATCAGGTTCTTTGTCTGAC
**SEQ ID NO. 49.** GTCGTGGCTGGAGTCTTG
**SEQ ID NO. 50.** CGGAGGCTGGCATTGTAG
**SEQ ID NO. 51.** ATCTCCTTTGGAAGCGGATATG
**SEQ ID NO. 52.** CGCAACGCAAAGCATTTCTT
**SEQ ID NO. 53.** GGTATTGAACTGACAGACTC
**SEQ ID NO. 54.** CCAGTTGTTGACCAAAGG
**SEQ ID NO. 55.** GTAACATCTACAGCCTTAATGAG
**SEQ ID NO. 56.** CCAGAGTGCGGTGAATATC
**SEQ ID NO. 57.** AGGCTTCCAGTACCATTAGGT
**SEQ ID NO. 58.** CTGAGTGAGGCAAAGCTGATTT
**SEQ ID NO. 59.** TTGGCACCGATCCTCGAAC
**SEQ ID NO. 60.** CCCAGCTCCAGTCAGAACTAT
**SEQ ID NO. 61.** CAGTGTGGTGCACGTCTCCAATC
**SEQ ID NO. 62.** TGAACCAAAGTTGACCACCAG
**SEQ ID NO. 63.** AGCCGTGACCACTGACAACGAG
**SEQ ID NO. 64.** GCTGCATGGTTCTGAGTGCTAAG
**SEQ ID NO. 65.** GCTGGAGGTGGCTTTGGT
**SEQ ID NO. 66.** GCTTGGCGGATGTGGTTC
**SEQ ID NO. 67.** GGATAAACAGAATAAGCACACCA
**SEQ ID NO. 68.** GAAGGAACAAAGCGGATGAG
**SEQ ID NO. 69.** CCGACCGAATGCAGAAGGA
**SEQ ID NO. 70.** ACAGAGTATTTGCGCTCCGAA
**SEQ ID NO. 71.** AAGGTATTGCTGGACAGCGT
**SEQ ID NO. 72.** TGTTTGCCAGGTTCACCAGA
**SEQ ID NO. 73.** AACATCTGGCACTCCACACC
**SEQ ID NO. 74.** GCAGAAGTTCTTTGGCCTGC

### DEFINITIONS

"About" refers to a range of values plus or minus 10 percent, e.g. about 1.0 encompasses values from 0.9 to 1.1.

"CXXCS-DVL interface" refers to an interaction and/or association between CXXCS(CXXC finger protein 5) and DVL (dishevelled), which can induce biological activities known in the art. The interactions and/or associations can be physical or chemical interactions that would activate a CXXC5-DVL pathway within a subject. CXXC5-DVL interface can be present in a form of a complex.

"Inhibitor of CXXC5-DVL interface" refers to an agent that alters the function and/or activity of the CXXC5-DVL interface or induces conformational changes in the CXXC5-DVL interface. Examples of inhibitors of CXXC5-DVL interface include, but are not limited to, agents that alter association/dissociation between CXXC5 and DVL and/or agents that inhibit CXXC5-DVL complex assembly/function.

"Metabolic disease or a similar condition" can include, but is not limited to, metabolic disorder, metabolic syndrome, obesity, high blood pressure, high blood sugar, high serum triglycerides, hyperuricemia, fatty liver, polycystic ovarian syndrome, erectile dysfunction, acanthosis nigricans, type 2 diabetes mellitus, hypoadiponectinemia, cirrhosis, portal hypertension, cardiovascular diseases, coronary artery disease, lipodystrophy, dyslipidemia, hepatic steatosis, non-alcoholic fatty liver disease (NAFLD), and/or non-alcoholic steatohepatitis (NASH).

"Pharmaceutically acceptable" refers to approved or approvable by a regulatory agency of a government, such as the U.S. FDA or the EMA, or listed in the U.S. Pharmacopoeia or other generally recognized pharmacopoeia for use in mammals and/or animals, and more particularly in humans.

"Pharmaceutically acceptable vehicle" or "pharmaceutically acceptable carrier," unless stated or implied otherwise, is used herein to describe any ingredient other than the active component(s) that can be included in a formulation. The choice of carrier will to a large extent depend on factors such as the mode of administration, the effect of the carrier on solubility and stability, and the nature of the dosage form.

"Pharmaceutical composition" refers to a therapeutically active inhibitor of CXXC5-DVL interface or a therapeutically active inhibitor of GSKβ, and at least one pharmaceutically acceptable vehicle/carrier, with which the inhibitor of CXXC5-DVL interface and/or inhibitor of GSKβ is administered to a subject.

"Subject" refers to a human (adult and/or child), an animal, a livestock, a cell, and/or a tissue.

"Therapeutically effective amount" refers to the amount of an inhibitor of CXXC5-DVL interface or inhibitor of GSKβ that, when administered to a subject for treating a disease, or at least one of the clinical symptoms of a disease, is sufficient to affect such treatment of the disease or symptom thereof. The "therapeutically effective amount" can vary depending, for example, on the inhibitor of CXXC5-DVL interface, inhibitor of GSKβ, the disease and/or symptoms of the disease, severity of the disease and/or symptoms of the disease or disorder, the age, weight, and/or health of the subject to be treated, and the judgment of the prescribing physician.

"Therapeutically effective dose" refers to a dose that provides effective treatment of a disease or disorder in a subject. A therapeutically effective dose can vary from compound to compound, and from subject to subject, and can depend upon factors such as the condition of the subject and the route of delivery.

"Therapeutic regime(s)" and/or "therapeutic regimen(s)" include, but are not limited to, surgery, weight loss, healthy eating, physical activity, insulin therapy, and/or a medication/drug therapy. In some embodiments, the medication/drug therapy includes one or more treatments with at least one agent including, but is not limited to, orlistat, lorcaserin, phentermine-topiramate, naltrexone-bupropion, liraglutide, benzphetamine, diethylpropion, sulfonylureas, meglitinides, thiazolidinediones, DPP-4 inhibitors, insulin analog, alpha glucosidase inhibitor, SGLT2 inhibitors, sitagliptin, metformin, rosiglitazone, ocaliva, selonsertib, elafibranol, cenicriviroc, MGL-3196, GR-MD-02, and/or aramchol.

"Treat," "treating" or "treatment" of any disease or condition refers to reversing, alleviating, arresting, or ameliorating a disease or at least one of the clinical symptoms of a disease, reducing the risk of acquiring a disease or at least one of the clinical symptoms of a disease, inhibiting the progress of a disease or at least one of the clinical symptoms of the disease or reducing the risk of developing a disease or at least one of the clinical symptoms of a disease. In some embodiments, "treat," "treating" or "treatment" also refers to inhibiting the disease, either physically, (e.g., stabilization of a discernible symptom), physiologically, (e.g., stabilization of a physical parameter), or both, and to inhibiting at least one physical parameter that can or cannot be discernible to the subject. In certain embodiments, "treat," "treating" or "treatment" refers to delaying the onset of the disease or condition or at least one or more symptoms thereof in a subject which can be exposed to or predisposed to a disease or condition even though that subject does not yet experience or display symptoms of the disease.

### DETAILED DESCRIPTION

The technical information set out below may in some respects go beyond the scope of the present invention, which is defined by the appended claims. The additional technical information is provided to place the present invention in a broader technical context and to illustrate possible related technical developments. References herein to methods of treatment of the human or animal body are to be understood as references to medicaments for use in a method of treatment.

For the purposes of promoting an understanding of the principles of the novel technology, reference will now be made to the preferred embodiments thereof, and specific language will be used to describe the same. It will nevertheless be understood that no limitation of the scope of the novel technology is thereby intended, such alterations, modifications, and further applications of the principles of the novel technology being contemplated as would normally occur to one skilled in the art to which the novel technology relates are within the scope of this disclosure and the claims.

Metabolic diseases possess multiplex pathological status associated with obesity, atherogenic dyslipidemia, insulin resistance, and increased risk of developing type 2 diabetes mellitus (T2DM). Metabolic diseases have long been considered as incurable, chronic conditions that require glycemic control in peripheral insulin target tissues. Although weight reduction by restricting the consumption of calories has been considered as an underlying direction to reverse metabolic diseases, an effective medication that improves the overall condition of metabolic diseases by targeting the systemic pathological process is not currently available.

Non-alcoholic steatohepatitis (NASH) can be characterized as inflammation and damage in liver caused by accumulation of fat in the liver. Many affected patients exhibit obesity, type 2 diabetes mellitus, glucose intolerance, dyslipidemia, and/or metabolic disease. Although incidences of NASH have been increasing worldwide with increase in obesity, its pathological mechanism(s) is not well understood.

In recent years, accumulating evidence from basic and clinical studies indicate that Wnt/β-catenin signaling target genes are involved in inducing inflammation, lipogenesis, fatty acid oxidation, glucose oxidation, mitochondria biogenesis, insulin resistance, glucose tolerance, and/or free fatty acid production. For example, the following Wnt/β-catenin pathway target genes are found to be involved in obesity, type II diabetes, and NASH: 1) Obesity : *TCF7L2, Wnt10b, PPARγ, C*/*EBPα, Cttnb1, Axin2, Lef1, Myc, Wisp1*, *Wisp2* and *Tle3*) Type II diabetes : *Wnt5b, PPARγ, TCF7L2 (TCF4), PPARγ, c-Myc, cyclin D1*, and *CDK4, AMPK, PGC-1,* and 3) NASH : *PPARγ*, *COL4, COL3, α-SMA,* and *Fibronectin.*

CXXC finger protein 5 (CXXC5) is a negative regulator of Wnt/β-catenin signaling, functioning via interaction with PDZ domain of dishevelled (DVL) in the cytosol. Inhibition of the CXXC5-DVL interaction improved several pathophysiological phenotypes involving Wnt/β-catenin signaling including osteoporosis, longitudinal bone growth, cutaneous wounds, and hair loss through activation of the Wnt/β-catenin signaling.

As disclosed herein, CXXC5 expression were progressively increased in the white adipocytes and the liver tissues of patients diagnosed with NASH and diabetes. Further, Wnt/β-catenin pathway target genes such as *TCF7L2,* and *FOSL1* were found to be suppressed in patients diagnosed with NASH and/or Type II diabetes. *Cxxc5*^{-/-} mice did not develop any phenotypes of metabolic diseases including obesity, diabetes, and/or NASH. The results disclosed herein suggest that CXXC5 contributes to the development of metabolic diseases. Thus, the instant disclosure provides a novel function of CXXC5-DVL interface that may lead to the treatment of metabolic diseases including, but are not limited to, obesity, diabetes, and/or NASH.

The present disclosure provides, *inter alia,* a discovery platform for developing therapeutic inhibitors of a CXXC5-DVL interface that negatively affects the Wnt/β-catenin pathway, for example, in liver of a subject having or suspected of having metabolic diseases. For example, small molecules that activate the Wnt/β-catenin pathway by inhibiting the CXXC5-DVL interface were obtained by use of an *in vitro* screening system monitoring fluorescent intensity that reveals binding of the PTD-DBMP (protein transduction domain fused DVL binding motif peptide), which contains sequence of CXXC5 binding to DVL and is conjugated to FITC, onto PDZ domain of DVL. *See e.g.,* Kim HY, et al (2016), Small molecule inhibitors of the Dishevelled-CXXC5 interaction are new drug candidates for bone anabolic osteoporosis therapy. EMBO Mol Med 8: 375-387. Interestingly, several GSK3β inhibitors, including 6-bromoindirubin-3'-oxime (BIO) and indirubin 3'-oxyme (I3O), were identified as initial hits. Further, the instant disclosure provides that a functionally improved, and newly synthesized, indirubin derivatives, e.g., A3334 and A3051, effectively inhibited interaction between CXXC5 and DVL. Moreover, A3334 and A3051 markedly reduced and/or inhibited the development of high fat diet (HFD)-induced and methionine-choline deficient diet (MCD)-induced phenotypes such as obesity, diabetes, and/or NASH. By identifying this CXXC5-DVL induced mechanism of developing metabolic diseases, the present disclosure provides a platform for screening compound libraries for inhibitors of the specific interaction of CXXC5 and DVL by binding to the CXXC5-DVL interface that involves the DVL binding motif.

Embodiments disclosed herein relate to compositions for use in methods for treating a metabolic disease. In certain embodiments, compositions and methods disclosed herein concern suppression of a side effect of a therapeutic regime. Other embodiments relate to compositions and methods for treating a subject diagnosed with a metabolic disease or having a condition contributed to fatty liver disease, non-alcoholic fatty liver disease (NAFLD), non-alcoholic steatohepatitis (NASH), obesity, diabetes, hyperlipidemia, chronic liver disease, cirrhosis, coronary artery disease, portal hypertension, lipodystrophy, rheumatic disease, psoriasis, and/or psoriatic arthritis.

Uses disclosed herein include a method of treating a clinical condition, comprising administering to a subject at least one therapeutically effective dose of any one of the compounds and/or compositions disclosed herein. The subject can be diagnosed with a clinical condition selected from and/or comprising a metabolic disease or a similar condition thereof. In certain embodiments, the methods disclosed herein further comprise administering to the subject at plurality of therapeutically effective doses of any one of the compounds and/or compositions disclosed herein.

In some disclosures, compositions disclosed herein comprise at least one agent that inhibits CXXC5-DVL interface in a subject. Consistent with these disclosures, the at least one agent that inhibits CXXCS-DVI, interface comprises at least one compound disclosed herein. In some disclosures, the at least one agent that inhibits CXXC5-DVL interface can disrupt conformation of the CXXCS-DVI, interface physically and/or chemically. The compositions use in the invention are those as claimed.

### Pharmaceutical Compositions

Pharmaceutical compositions provided by the present disclosure can comprise a therapeutically effective amount of one or more compositions disclosed herein, together with a suitable amount of one or more pharmaceutically acceptable vehicles to provide a composition for proper administration to a subject. Suitable pharmaceutical vehicles are described in the art.

Pharmaceutical compositions of the present disclosure suitable for oral administration can be presented as discrete units, such as a capsule, cachet, tablet, or lozenge, each containing a predetermined amount of the active ingredient; as a powder or granules; as a solution or a suspension in an aqueous liquid or non-aqueous liquid such as a syrup, elixir or a draught, or as an oil-in-water liquid emulsion or a water-in-oil liquid emulsion. The composition can also be presented as a bolus, electuary, or paste. A tablet can be made by compressing or moulding the active ingredient with the pharmaceutically acceptable carrier. Compressed tablets can be prepared by compressing in a suitable machine the active ingredient in a free-flowing form, such as a powder or granules, in admixture with, for example, a binding agent, an inert diluent, a lubricating agent, a disintegrating and/or a surface-active agent. Moulded tablets can be prepared by moulding in a suitable machine a mixture of the powdered active ingredient moistened with an inert liquid diluent. The tablets can optionally be coated or scored and can be formulated to provide slow or controlled release of the active ingredient.

Pharmaceutical compositions of the present disclosure suitable for parenteral administration include aqueous and non-aqueous sterile injection solutions, and can also include an antioxidant, buffer, a bacteriostat and a solution which renders the composition isotonic with the blood of the recipient, and aqueous and non-aqueous sterile suspensions which can contain, for example, a suspending agent and a thickening agent. The formulations can be presented in a single unit-dose or multi-dose containers and can be stored in a lyophilized condition requiring the addition of a sterile liquid carrier prior to use.

Pharmaceutically acceptable salts include salts of compounds provided by the present disclosure that are safe and effective for use in mammals and that possess a desired therapeutic activity. Pharmaceutically acceptable salts include salts of acidic or basic groups present in compounds provided by the present disclosure. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain disclosed compounds may form pharmaceutically acceptable salts with various amino acids. Suitable base salts include, but are not limited to, aluminum, calcium, lithium, magnesium, potassium, sodium, zinc, and diethanolamine salts. For additional information on some pharmaceutically acceptable salts that can be used to practice the methods described herein please review articles such as Berge, et al., 66 J. PHARM. SCI. 1-19 (1977), Haynes, et al, J. Pharma. Sci., Vol. 94, No. 10, Oct. 2005, pgs. 2111-2120, and the like.

In some embodiments, the composition can contain pharmaceutically acceptable lubricant(s). The pharmaceutically acceptable lubricant(s) prevent the components of the pharmaceutical composition from clumping together and from sticking to the pellet press that generates the disclosed compositions. The lubricant(s) also ensure that the formation of the pellet, as well as its ejection from the pellet press, occurs with low friction between the composition and the wall of the die press. In some embodiments, the lubricant(s) are added to a pharmaceutical composition to improve processing characteristics, for example to help increase the flexibility of the compositions, thereby reducing breakage.

The type of lubricant that can be used in the disclosed pharmaceutical compositions can vary. In some embodiments, the pharmaceutically acceptable lubricant is selected from talc, silica, vegetable stearin, magnesium stearate, stearic acid, calcium stearate, glyceryl behenate, glyceryl monostearate, glyceryl palmitostearate, mineral oil, polyethylene glycol, sodium stearyl fumarate, sodium lauryl sulfate, vegetable oil, zinc stearate, and combinations thereof. In some embodiments, the pharmaceutically acceptable lubricant is stearic acid.

The type of vehicles that can be used in the disclosed pharmaceutical compositions can vary. In some embodiments, the pharmaceutically acceptable vehicles are selected from binders, fillers and combinations thereof. In some embodiments, the pharmaceutically acceptable vehicle is selected from ascorbic acid, polyvinylpyrrolidone, polyvinylpyrrolidone K-30 (povidone K-30), glyceryl monostearate (GMS) or glyceryl monostearate salts, glyceryl behenate, glyceryl palmitostearate, hydroxypropyl cellulose, hydroxypropyl methylcellulose, methylcellulose, hydroxyethyl cellulose, sugars, dextran, cornstarch, dibasic calcium phosphate, dibasic calcium phosphate dihydrate, calcium sulfate, dicalcium phosphate, tricalcium phosphate, lactose, cellulose including microcrystalline cellulose, mannitol, sodium chloride, dry starch, pregelatinized starch, compressible sugar, mannitol, lactose monohydrate, starch, dibasic calcium phosphate dihydrate, calcium sulfate, dicalcium phosphate, tricalcium phosphate, powdered cellulose, microcrystalline cellulose, lactose, glucose, fructose, sucrose, mannose, dextrose, galactose, the corresponding sugar alcohols and other sugar alcohols, such as mannitol, sorbitol, xylitol, and combinations of any of the foregoing. In some embodiments, the pharmaceutically acceptable vehicle is polyvinylpyrrolidone K-30, also known as povidone K-30. In some embodiments, the pharmaceutically acceptable vehicle is polyvinylpyrrolidone K-30, also known as povidone K-30, having an average molecular weight of MW of 40,000 (CAS 9003-39-8). In some embodiments, the pharmaceutically acceptable vehicle is selected from glyceryl monostearate (GMS) or glyceryl monostearate salts, glyceryl behenate and glyceryl palmitostearate. In some embodiments, the pharmaceutically acceptable vehicle is glyceryl monostearate (GMS) or glyceryl monostearate salts. In some embodiments, the pharmaceutically acceptable vehicle is glyceryl behenate. In some embodiments, the pharmaceutically acceptable vehicle is glyceryl palmitostearate.

In some embodiments, the antioxidants prevent oxidation of the other components of the disclosed compositions. Oxidation can occur, for example, during sterilization where free radicals are generated. Addition of the antioxidants, or free radical scavengers, significantly reduces oxidation and makes the composition more pharmaceutically acceptable for use in subjects.

The type of antioxidants that can be used in the disclosed pharmaceutical compositions can vary. In some embodiments, the antioxidant is selected from methyl paraben and salts thereof, propyl paraben and salts thereof, vitamin E, vitamin E TPGS, propyl gallate, sulfites, ascorbic acid (aka L-ascorbic acid, also including the L-enantiomer of ascorbic acid, vitamin C), sodium benzoate, citric acid, cyclodextrins, peroxide scavengers, benzoic acid, ethylenediaminetetraacetic acid (EDTA) and salts thereof, chain terminators (e.g., thiols and phenols), butylated hydroxy toluene (BHT), butylated hydroxyanisole (BHA), and combinations thereof.

### Uses or Methods of Treatment

The compositions for use in methods and compositions disclosed herein can be used in the treatment of subjects suffering from diseases, disorders, conditions, and symptoms for which inhibitors of CXXC5-DVL interface and/or GSKβ are known to provide or are later found to provide therapeutic benefit. In the invention, the disease is a metabolic disease.

In some embodiments, the compositions for use in methods disclosed herein include a composition for use in a method of treating a clinical condition, comprising administering to a subject at least one therapeutically effective dose of any of the compositions disclosed herein. The subject can be diagnosed with a clinical condition selected from and/or comprising fatty liver disease, non-alcoholic fatty liver disease (NAFLD), non-alcoholic steatohepatitis (NASH), obesity, diabetes, hyperlipidemia, chronic liver disease, cirrhosis, coronary artery disease, portal hypertension, lipodystrophy, rheumatic disease, psoriasis, and psoriatic arthritis, and/or any other conditions associated with, induced by, or that are already resistant to drug treatments, therapies and/or surgical treatments. In certain embodiments, the compositions for use in methods disclosed herein further comprise administering to the subject at least one additional therapeutically effective dose of any of the compositions disclosed herein. In some embodiments, the at least one therapeutically effective dose of any of the compositions disclosed herein can be administered orally, parenterally, intravenously, by inhalation and/or transdermally.

Yet other embodiments can include compositions for use in methods for reducing a side effect of a therapeutic regime, comprising administering to a subject at least one therapeutically effective dose of at least one agent that inhibits CXXC5-DVL interface in a subject; wherein the subject has received at least one therapeutic regime comprising drug treatments, surgery, therapy, and wherein the subject experiences at least one side effect derived from the therapeutic regime. Consistent with these embodiments, side effects can include, but are not limited to, drug-resistance and/or relapse.

### Kits

Kits are disclosed by the present disclosure, such kits comprising: one or more pharmaceutical compositions, each composition sterilized within a container, means for administration of the pharmaceutical compositions to a subject, and instructions for use.

Some disclosures include kits for carrying out the compositions for use in methods disclosed herein. Such kits typically comprise two or more components required for treating a clinical condition. Components of the kit include, but are not limited to, one or more of agents/compositions disclosed herein, reagents, containers, equipment and/or instructions for using the kit. Accordingly, the compositions and compositions for use in methods described herein can be performed by utilizing pre-packaged kits disclosed herein.

### Examples

The following examples illustrate various aspects of the disclosure. It will be apparent to those skilled in the art that many modifications, both to materials and methods, can be practiced without departing from the scope of the disclosure.

### The involvement of CXXC5 in metabolic diseases including obesity and diabetes

Reports have shown that diabetes could be reversed by understanding molecular and cellular events that may control adipose tissue expansion. Understanding the molecular and cellular events can be a useful tool in identifying a therapeutic approach for the prevention and treatment of the overall features of metabolic diseases such as obesity-related insulin resistance and systemic inflammation.

The canonical Wnt/β-catenin pathway regulates cellular metabolism involving nutrient sensing by affecting the expression of metabolically relevant transcription factors such as *WISP1*, *c-MYC, CCND1*, *PPARδ,* and *TCF7L2.* The activation of the Wnt/β-catenin pathway suppresses *PPARγ* and *C*/*EBPα*, the adipogenic transcription factors, by the induction of *WISP1*, *c-MYC,* and *CCND1.* PPARδ improves the metabolic parameters and stimulates β-oxidation in metabolically active tissues including the liver and adipose tissues. The common variants of *TCF7L2* are associated with an increased risk of T2DM and impaired function of the incretin hormone, and its receptor in pancreatic β-cells. Moreover, the adipocyte-specific deletion of *TCF7L2* promotes adipocyte hypertrophy, hepatic insulin resistance, and whole-body glucose intolerance. The role of Wnt/β-catenin signaling inactivation in the pathogenesis of T2DM has been shown as elevated expression of Dickkopf-1 (DKK-1), the Wnt/β-catenin signaling antagonist, in the serum of a T2DM patient. Inactivation of Wnt/β-catenin signaling modifies the adipokine-secretion profile followed by obesity-induced adipose tissue inflammation, thereby, influencing the systemic insulin resistance. Overall, aberrant regulation of the direct or indirect Wnt/β-catenin pathway response genes is involved in metabolic disease. However, the development of drugs targeting the Wnt/β-catenin pathway is limited and has mainly focused on the level of the downstream transcription factors including *PPARγ.* Therefore, identification of a factor influencing the whole Wnt/β-catenin pathway, especially which drives metabolic diseases, is further needed for the systemic treatment of the multi-diverse metabolic diseases.

CXXC5-type zinc finger protein 5 (CXXC5) is a negative feedback regulator of the Wnt/β-catenin pathway that functions via Dishevelled (Dvl) binding. CXXC5 plays various pathophysiological roles involving regenerative tissue remodeling, especially at the specific pathophysiological status. However, the role of CXXC5 in the process of adipogenesis and obesity-related metabolic diseases has not been defined yet. In the present disclosure, it was found unexpectedly that *CXXC5* was highly expressed in visceral adipose tissues from obese and diabetic patients, and thereby, experiencing reduced the expression of Wnt/β-catenin target genes.

As disclosed herein, HFD-fed *Cxxc5*^{*-*/*-*} mice did not develop obesity and obesity-related insulin resistance. Further, the oral administration of at least one compound disclosed herein (e.g., 5-methoxyindirubin-3'-oxime, hereinafter A3334) activated Wnt/β-catenin signaling by interfering the Dvl-CXXC5 protein-protein interaction (Dvl-CXXC5 PPI) in HFD-fed mice and mimicked the results derived from HFD-fed *Cxxc5*^{*-*/*-*} mice. It was unexpected and surprising that the administration of A3334 resulted in prolonged effectiveness for the control of fasting glucose levels in HFD-fed mice when compared to the treatment with sitagliptin and metformin, which are the drugs regularly prescribed for T2DM patients. These effects of A3334 on metabolic diseases acquired through activation of the Wnt/β-catenin pathway by blocking the function of the aberrantly overexpressed Cxxc5 during HFD-induced obesity was found. Activation of the Wnt/β-catenin pathway by A3334 was followed by transcriptional regulation of the direct and indirect metabolic targets genes involving inflammation, lipogenesis, adipogenesis, and mitochondria biogenesis, leading to improvement of whole-body energy metabolism.

**Human visceral fat specimens.** To monitor the expression patterns of *β*-catenin and CXXC5 during the development of obesity-related diabetes, 5-mm biopsy specimens were obtained from liver or colon cancer patients who had undergone surgery. The age of the subjects ranged from 43 to 82 years, who had a body mass index (BMI) between 17 and 32 kg m⁻². Individuals were assigned by BMI or diabetes mellitus (DM) grade (lean, BMI < 25 or DM grade = 0, 1, 2) divided four cohorts. (i) lean, BMI < 25, DM = 0; (ii) obesity, BMI > 25, DM = 0 or 1; (iii) lean, BMI < 25, DM = 2; and (iv) obesity, BMI >25, DM = 2. Experiments using patient samples were approved by the Institutional Review Board of the Clinical Research Institute of Severance Hospital and were conducted according to the Declaration of Helsinki Principles.

**Animals.** The generation of *Cxxc5*^{*-*/*-*} mice has been described previously. *Cxxc5* heterozygous mice were intercrossed for four generations to obtain littermate wild-type and *Cxxc5*^{*-*/*-*} mice and were maintained on a C57BL/6 background. Six-week-old *Cxxc5*^{+/+} and *Cxxc5*^{*-*/*-*} mice were fed HFD for 8 weeks. Wild-type male C57BL/6 mice (KOATECH, Seoul, Korea) were fed HFD consisting of 60% calories from fat (Research Diet, D12492) for 8 weeks. To validate that the insulin resistance mouse model was successfully established, fasting glucose levels were assessed with a One Touch Ultra glucometer (LifeScan). Subsequently, each HFD-fed mouse with a fasting glucose level higher than 16.7 mmol/L was orally administered A3334 (25 mg kg⁻¹), sitagliptin (50 mg kg⁻¹), or metformin (100 mg kg⁻¹) each day for 5 days at weeks 8 and 12. After the removal of the drugs, mice were maintained for 3 weeks on the HFD. To monitor pancreas regeneration, six-week-old *Cxxc5*^{+/+} and *Cxxc5*^{*-*/*-*} mice were fed an HFD as above. After dietary treatment for 4 weeks, the mice were intraperitoneally injected with STZ (50 mg/kg/d) for 1 week and the control group were injected with saline. After 2 weeks, *Cxxc5*^{+/+} mice were administered A3334 (25 mg kg⁻¹) and sitagliptin (50 mg kg⁻¹) per day by oral gavage for 4 weeks. For GTT or ITT, mice were injected with D-glucose (1.5 g/kg body weight) after overnight starvation or human insulin (0.75 units/kg body weight) after 4 h starvation, respectively. Tail blood was drawn at 0, 15, 30, 60, 120, 180 min intervals and blood glucose level was measured with a One Touch Ultra glucometer. All mice were maintained under temperature-controlled and light-controlled (standard 12 h light/dark cycle) conditions and provided with food and water ad libitum. All protocols were reviewed and approved by the Institutional Review Board of Severance Hospital, Yonsei University College of Medicine (09-013).

**Blood chemistry.** Total blood of mice was collected by cardiac puncture after fasting. The blood was allowed to clot for 30 min and was then centrifuged for 10 min at 1,000 × g to obtain supernatant to measure metabolic parameters. ELISA assay kits were used to assess serum insulin (Millipore), serum FFA (Cayman Chemical), serum adiponectin (ABclonal). The insulin function test was evaluated by HOMA-IR and was calculated as fasting plasma glucose (m mol/l) × fasting serum insulin (mU/l)/22.5. Serum chemistry variables included total cholesterol, HDL-cholesterol, glucose, TG, ALT, AST, ALP, Ca, and Mg concentration. The calibration of serum parameters was performed using the quality control card supplied with the FUJI DRI-CHEM slides whenever slides from a new lot were used.

**Adipokine-related protein analysis.** Adipokines and hormones in mouse serum were measured using mouse adipokine array kits (Proteome Profiler and Human Cytokine; R&D System) to simultaneously detect the relative expression levels of 38 different obesity-related proteins. The array was performed according to the manufacturer's instructions. Blots were developed with enhanced chemiluminescence using a luminescent image analyzer, LAS-3000 (Fujifilm). All data were normalized by the intensity of reference spots in each membrane following the manufacturer's instruction.

**Hematoxylin and eosin (H&E) staining.** Dissected tissues were fixed in 4% neutral paraformaldehyde and embedded in paraffin. The paraffin sections were cut at a thickness of 4 µm and subjected to H&E staining. The adipocyte cell size was measured in 20 randomly chosen microscopic areas from 3 independent animals using a Nikon bright-field optical microscope (Nikon TE-2000U). The average adipocyte size was determined using Image J software.

**Immunohistochemistry (IHC).** Paraffin sections 4 µm in size were deparaffinized and rehydrated. For antigen retrieval, the slides were autoclaved in 10 mM sodium citrate buffer (pH 6.0). Sections were blocked in phosphate buffered saline (PBS) containing 10% BSA at room temperature for 30 min. The sections were incubated overnight at 4°C with the following dilution of primary antibodies: anti-β-catenin (1: 100; BD), anti-CXXC5 (1:50; Santa Cruz Biotechnology, Inc.), anti-F4/80 (1: 100; Cell Signaling Technology), and anti-CD11b (1:100; eBioscience). The slides were washed with PBS, incubated with Alexa Fluor 488- or Alexa Fluor 555-conjugated IgG secondary antibody (1:300; Molecular Probes) at room temperature for 1 h, and counterstained with DAPI (1:5,000; Boehringer Mannheim). The images were captured using a LSM700 META confocal microscope (Carl Zeiss) after excitation with 405-, 488-, or 543-nm laser lines. To block endogenous peroxidase activity before peroxidase IHC analysis, tissues were incubated with 0.345% H₂O₂ (Samchum Chemicals) for 30 min. Before incubating sections with mouse primary antibody, mouse IgG was blocked using a M.O.M Mouse IgG blocking kit (Vector Laboratories). Sections were incubated with primary antibody overnight at 4°C with the following dilution of primary antibodies: anti-UCP1 (1:500; Abcam). Then, sections were incubated with biotinylated anti-rabbit (1:300; Dako) secondary antibodies for 1 h at room temperature. The samples were stained with 3, 3'-diaminobenzidine (DAB; Dako) for 3-7 min and counter stained with Mayer's hematoxylin (Muto). All incubations were conducted in humid chambers. Signals were analyzed using a bright field microscope (Nikon TE-2000U).

**Metabolic monitoring and body composition.** Metabolic performance (energy intake and energy expenditure) was studied using a PHENOMASTER automated combined indirect calorimetry system (TSE system GmBH). The mice were first acclimated for 24 h in a metabolic chamber and were provided with food and water. They were then subsequently evaluated for 3 days to measure oxygen consumption (VO₂), carbon dioxide production (VCO₂), ambulatory counts, and respiratory exchange ratio. An LF50 body composition analyzer (Bruker) was used to determine body composition (lean body mass and total body fat) of the mice. The temperature for these studies remained at 22°C, with a 12 h light/dark cycle. Standard in-house software was used for energy expenditure.

**Bioinformatics data analysis.** Molecular pathway dysregulation in the human visceral adipose tissues was determined by gene set enrichment analysis, surveying the molecular pathway gene set in Molecular Signature Database (MsigDB). Cross-species comparison of transcriptomic dysregulation was performed in the space of molecular pathway gene sets from HALLMARK and KEGG databases and with statistically significant dysregulation defined as false discovery rate (FDR) < 0.01 in either of the two human visceral and subcutaneous adipose tissue transcriptome datasets: normal (n = 5) *vs.* T2DM (n = 5) subjects (GSE16415), normal glucose tolerance (n = 17) *vs.* T2DM (n = 17) subjects (hgu-133a), lean (n = 10) vs. obese (n = 10) subjects (GSE2508), normal glucose tolerance (NGT) (n = 4) vs. impaired glucose tolerance (IGT) (n = 4) vs. T2DM (n = 4) subjects (GSE27951), and insulin sensitive (n = 5) vs. resistance (n = 5) subjects (GSE15773).

**Dvl-CXXC5 *in vitro* binding assay.** For the Dvl-CXXC5 *in vitro* binding assay, 100 µl of 5 mg/ml purified Dvl PDZ domain was added into 96-well Maxibinding Immunoplate (SPL) and incubated overnight in a 4°C chamber. After washing with PBS, 10 µM PolyR-DBM³⁷ was added to each well and incubated for 3 h at room temperature. After washing with PBS, 100 µl of 1, 5, and 10 µM A3334 in PBS was added to each well and incubated for 1 h at room temperature. After washing with PBS three times, the fluorescence of each well was measured using a Fluorstar Optima microplate reader (BGM Lab Technologies). A3334 used in screening were designed and synthesized by Dr Gyoonhee Han (Yonsei University, Seoul, Korea).

**Luciferase reporter assay.** HEK293-TOP cells were seeded in 24-well plates. Cells were treated with A3334 at a concentration of 1, 5, and 10 µM for 24 h. Total cell lysates were extracted with 35 µl of 5X Reporter Lysis Buffer per well according to the manufacturer's instructions (Promega). A total of 35 µl of luciferin was added and luciferase activity was measured using a Fluorstar Optima microplate reader.

**Cell culture and adipocyte differentiation.** 3T3-L1 cells were seeded in six-well plates at a density of 3 × 10⁴ cells per well. The cells were grown in Dulbecco's modified Eagle medium (DMEM) with 10% bovine calf serum (Gibco) until confluent. After confluence, cells were induced to differentiate in DMEM containing 10% fetal bovine serum (FBS; Gibco) and MDI (520 µM methylisobutylxanthine (IBMX; Sigma-Aldrich), 1 µM dexamethasone (Sigma-Aldrich), and 167 nM insulin (Gibco)) with or without A3334. On day 4, the medium was replaced with DMEM containing 10% FBS and 167 nM insulin with or without A3334 and changed with fresh identical medium every 2 days up to day 14 post-induction. Cells were incubated at 37°C in a 5% CO₂ environment.

**Oil Red O staining.** 3T3-L1 cells and liver tissues were washed with PBS and 70% isopropanol (Duksan Pure Chemicals) and stained with Oil Red O solution (Sigma-Aldrich) at room temperature overnight. Samples were washed thoroughly with distilled water. Tissues were counterstained with Mayer's hematoxylin. Images of the Oil Red O staining were visualized with a bright field microscope (Nikon TE-2000U). For the quantification of lipid contents, the Oil Red O was eluted by the addition of 500 µl isopropanol containing 4% nonidet P-40 to each well and the absorbance was measured spectrophotometrically at 590 nm.

**Triglyceride (TG) assay.** Tissues were incubated on ice into 100 µl saline solution (2 M NaCl, 2 mM EDTA, 50 mM sodium phosphate, and pH 7.4). Cells and tissues suspensions were assayed for TG content using a TG assay kit (Cayman Chemical).

**Quantitative real-time polymerase chain reaction (PCR).** Total RNA was extracted from ground tissue powder using TRIzol reagent (Invitrogen) according to the manufacturer's instructions. Reverse transcription was performed with M-MLV reverse transcriptase (Invitrogen) using 2 µg of total RNA. Synthesized cDNA was diluted to a concentration of 100 ng/µl. Quantitative PCR analyses were performed in the Rotor-gene Q real-time PCR cycler (Qiagen) using SYBR green reagent (Qiagen) with conditions of 95°C for 10 min followed by 40 cycles at 95°C for 5 s and 60°C for 15 s. Relative quantification of mRNA levels was estimated using the comparative Ct method (ΔΔCt). All mRNA values were normalized with respect to *GAPDH.* The primer sequences are listed in **Table 1**.

**Table 1. List of Primers**

| Mouse | | | |
|---|---|---|---|
| Gene | Strand | Primer Sequences | |
| *CXXCS* | F | 5'-CAAGAAGAAGCGGAAACGCTGC-3' | **SEQ ID NO. 1** |
| | R | 5'-TCTCCAGAGCAGCGGAAGGCTT-3' | **SEQ ID NO. 2** |
| *CXXC4* | F | 5'-CAACCCAGCCAAGAAGAAGA-3' | **SEQ ID NO. 3** |
| | R | 5'-AGATCTGGTGTCCCGTTTTG-3' | **SEQ ID NO. 4** |
| *Tcf4* | F | 5'-TGTGTACCCAATCACGACAGGAG-3' | **SEQ ID NO. 5** |
| | R | 5'-GATTCCGGTCGTGTGCAGAG-3' | **SEQ ID NO. 6** |
| *Axin2* | F | 5'-TGGAGAGTGAGCGGCAGAGC-3' | **SEQ ID NO. 7** |
| | R | 5'-TGGAGACGAGCGGGCAGA-3' | **SEQ ID NO. 8** |
| *Dvl1* | F | 5'-CCTTCCATCCAAATGTTGC-3' | **SEQ ID NO. 9** |
| | R | 5'-GTGACTGACCATAGACTCTGTGC-3' | **SEQ ID NO. 10** |
| *Wisp1* | F | 5'-ATCGCCCGAGGTACGCAATAGG-3' | **SEQ ID NO. 11** |
| | R | 5'-CAGCCCACCGTGCCATCAATG-3' | **SEQ ID NO. 12** |
| *Fosl1* | F | 5'-AACCGGAGGAAGGAACTGAC-3' | **SEQ ID NO. 13** |
| | R | 5'-AACCGGAGGAAGGAACTGAC-3' | **SEQ ID NO. 14** |
| *PPARγ* | F | 5'-TGTGGGGATAAAGCATCAGGC-3' | **SEQ ID NO. 15** |
| | R | 5'-CCGGCAGTTAAGATCACACCTAT-3' | **SEQ ID NO. 16** |
| *C*/*EBPα* | F | 5'-GGTGGACAAGAACAGCAACGA-3' | **SEQ ID NO. 17** |
| | R | 5'-TGTCCAGTTCACGGCTCAGCT-3' | **SEQ ID NO. 18** |
| *SREBP1* | F | 5'-GGAGCCATGGATTGCACATT-3' | **SEQ ID NO. 19** |
| | R | 5'-GGCCCGGGAAGTCACTGT-3' | **SEQ ID NO. 20** |
| *FAS* | F | 5'-GCGATGAAGAGCATGGTTTAG-3' | **SEQ ID NO. 21** |
| | R | 5'-GGCTCAAGGGTTCCATGTT-3' | **SEQ ID NO. 22** |
| *SCD-1* | F | 5'-CTGTACGGGATCATACTGGTTC-3' | **SEQ ID NO. 23** |
| | R | 5'-GCCGTGCCTTGTAAGTTCTG-3' | **SEQ ID NO. 24** |
| *ACC* | F | 5'-CCTCCGTCAGCTCAGATACA-3' | **SEQ ID NO. 25** |
| | R | 5'-TTTACTAGGTGCAAGCCAGACA-3' | **SEQ ID NO. 26** |
| *Tnfα* | F | 5'-CGGAGTCCGGGCAGGT-3' | **SEQ ID NO. 27** |
| | R | 5'-GCTGGGTAGAGAATGGATCA-3' | **SEQ ID NO. 28** |
| *Tgfβ1* | F | 5'-TGACGTCACTGGAGTTGTACGG-3' | **SEQ ID NO. 29** |
| | R | 5'-GGTTCATGTCATGGATGGTGC-3' | **SEQ ID NO. 30** |
| *Ifnγ* | F | 5'-TCAAGTGGCATAGATGTGGAAGAA-3' | **SEQ ID NO. 31** |
| | R | 5'-TGGCTCTGCAGGATTTTCATG-3' | **SEQ ID NO. 32** |
| *F4*/*80* | F | 5'-CTTTGGCTATGGGCTTCCAGTC-3' | **SEQ ID NO. 33** |
| | R | 5'-GCAAGGAGGACAGAGTTTATCGTG-3' | **SEQ ID NO. 34** |
| *MCP1* | F | 5'-ACTGAAGCCAGCTCTCTCTTCCTC-3' | **SEQ ID NO. 35** |
| | R | 5'-TTCCTTCTTGGGGTCAGCACAGAC-3' | SEQ ID NO. 36 |
| *Arg1* | F | 5'-CTCCAAGCCAAAGTCCTTAGAG-3' | SEQ ID NO. 37 |
| | R | 5'-GGAGCTGTCATTAGGGACATCA-3' | SEQ ID NO. 38 |
| *Chi3l3* | F | 5'-CAGGTCTGGCAATTCTTCTGAA-3' | SEQ ID NO. 39 |
| | R | 5'-GTCTTGCTCATGTGTGTAAGTGA-3' | SEQ ID NO. 40 |
| *Retnla* | F | 5'-CCAATCCAGCTAACTATCCCTCC-3' | SEQ ID NO. 41 |
| | R | 5'-ACCCAGTAGCAGTCATCCCA-3' | SEQ ID NO. 42 |
| *Pdcd1lg2* | F | 5'-TTGTCGGTGTGATTGGCTTC-3' | SEQ ID NO. 43 |
| | R | 5'-AAAAGGCAGCACACAGTTGC-3' | SEQ ID NO. 44 |
| *IL-10* | F | 5'-GCTATGCTGCCTGCTCTTACT-3' | SEQ ID NO. 45 |
| | R | 5'-CCTGCTGATCCTCATGCCA-3' | SEQ ID NO. 46 |
| *Irs1* | F | 5'-GGACTTGAGCTATGACACGGG-3' | SEQ ID NO. 47 |
| | R | 5'-GCCAATCAGGTTCTTTGTCTGAC-3' | SEQ ID NO. 48 |
| *G6pc* | F | 5'-GTCGTGGCTGGAGTCTTG-3' | SEQ ID NO. 49 |
| | R | 5'-CGGAGGCTGGCATTGTAG-3' | SEQ ID NO. 50 |
| *PEPCK* | F | 5'-ATCTCCTTTGGAAGCGGATATG-3' | SEQ ID NO. 51 |
| | R | 5'-CGCAACGCAAAGCATTTCTT-3' | SEQ ID NO. 52 |
| *Pck1* | F | 5'-GGTATTGAACTGACAGACTC-3' | SEQ ID NO. 53 |
| | R | 5'-CCAGTTGTTGACCAAAGG-3' | SEQ ID NO. 54 |
| *Fbp1* | F | 5'-GTAACATCTACAGCCTTAATGAG-3' | SEQ ID NO. 55 |
| | R | 5'-CCAGAGTGCGGTGAATATC-3' | SEQ ID NO. 56 |
| *UCP1* | F | 5'-AGGCTTCCAGTACCATTAGGT-3' | **SEQ ID NO. 57** |
| | R | 5'-CTGAGTGAGGCAAAGCTGATTT-3' | **SEQ ID NO. 58** |
| *SIRT1* | F | 5'-TTGGCACCGATCCTCGAAC-3' | **SEQ ID NO. 59** |
| | R | 5'-CCCAGCTCCAGTCAGAACTAT-3' | **SEQ ID NO. 60** |
| *Dio2* | F | 5'-CAGTGTGGTGCACGTCTCCAATC-3' | **SEQ ID NO. 61** |
| | R | 5'-TGAACCAAAGTTGACCACCAG-3' | **SEQ ID NO. 62** |
| *PGC1α* | F | 5'-AGCCGTGACCACTGACAACGAG-3' | **SEQ ID NO. 63** |
| | R | 5'-GCTGCATGGTTCTGAGTGCTAAG-3' | **SEQ ID NO. 64** |
| *CPT1* | F | 5'-GCTGGAGGTGGCTTTGGT-3' | **SEQ ID NO. 65** |
| | R | 5'-GCTTGGCGGATGTGGTTC-3' | **SEQ ID NO. 66** |
| *CPT2* | F | 5'-GGATAAACAGAATAAGCACACCA-3' | **SEQ ID NO. 67** |
| | R | 5'-GAAGGAACAAAGCGGATGAG-3' | **SEQ ID NO. 68** |
| *α-SMA* | F | 5'-CCGACCGAATGCAGAAGGA-3' | **SEQ ID NO. 69** |
| | R | 5'-ACAGAGTATTTGCGCTCCGAA-3' | **SEQ ID NO. 70** |
| *Col1a1* | F | 5'-AAGGTATTGCTGGACAGCGT-3' | **SEQ ID NO. 71** |
| | R | 5'-TGTTTGCCAGGTTCACCAGA-3' | **SEQ ID NO. 72** |
| *MMP-3* | F | 5'-AACATCTGGCACTCCACACC-3' | **SEQ ID NO. 73** |
| | R | 5'-GCAGAAGTTCTTTGGCCTGC-3' | **SEQ ID NO. 74** |

**Western blot analysis.** Cells and tissues were lysed using radioimmunoprecipitation assay (RIPA) buffer (150 mM NaCl, 10 mM Tris, pH 7.2, 0.1% SDS, 1.0% Triton X-100, 1% sodium deoxycholate, and 5 mM EDTA). Samples were separated on 12% SDS polyacrylamide gels and transferred onto PROTRAN nitrocellulose membranes (Shleicher and Schuell Co.). After blocking with PBS containing 5% nonfat dry skim milk and 0.07% (vol/vol) Tween 20, the membranes were incubated with antibody specific for β-catenin (1:1,000; Santa Cruz Biotechnology, Inc.), CXXC5 (1:1,000), and Erk (1:5,000; Santa Cruz Biotechnology, Inc.) at 4°C overnight. Samples were then incubated with horseradish peroxidase-conjugated anti-rabbit (1:1,000; Bio-Rad) or anti-mouse (1:1,000; Cell Signaling Technology) IgG secondary antibody. Protein bands were visualized with enhanced chemiluminescence (GE Healthcare) using a luminescent image analyzer, LAS-3000.

**Statistical analysis.** Data are presented as means ± standard deviation (SD). Statistical analyses were performed using unpaired two-tailed Student's t-test. Asterisks denote statistically significant differences (*, P < 0.05; **, P < 0.01; ***, P < 0.005).

### Synthesis of 5, 6-dichloroindirubin-3'-methoxime (A3051)

Synthesis of intermediate product, 5',6'-dichloro-[2,3'-biindolinylidene]-2',3-dione.

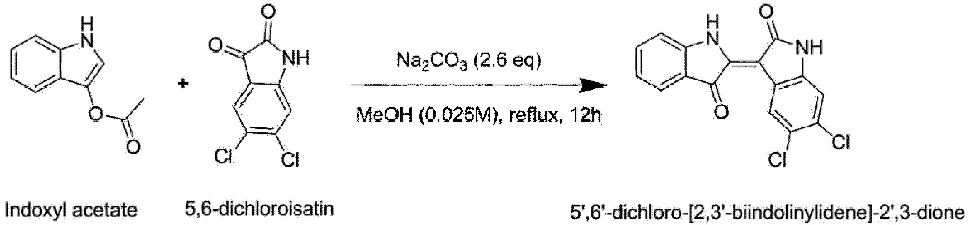

5,6-dichloroisatin (500 mg, 2.32 mmol) was added to a 250 mL round bottom flask and dissolved in methanol (MeOH) (92.80 mL) followed by the addition of indoxyl acetate (405.48 mg, 2.315 mmol) and sodium percarbonate (Na2CO3) (637.83 mg, 6.02 mmol), and the mixture was stirred at 65 °C for 12 hours. The reaction is terminated using TLC (Rf = 0.4, ethyl acetate / hexane = 1/2 (v/v)) and the product is allowed to cool down on ice until a lump of crystals is formed. After the crystals are formed, the solvent is removed by filtration. The filtrate is discarded and the product is washed several times with a solvent (ethanol / water = 1/1 (v / v)). The product was filtered and dried in a vacuum pump and used in the next step without further purification.

### Synthesis of A3051

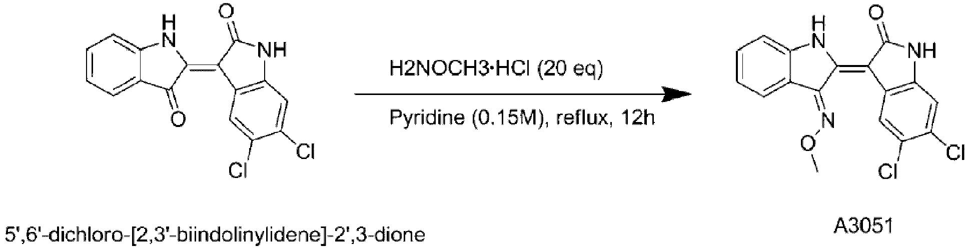

A 100 ml round-bottomed flask was charged with 5', 6'- dichloro- [2,3'-biindolinylidene ] -2 ', 3dione (600 mg, 1.81 mmol) and it was dissolved in pyridine (151 ml), and then H₂NOCH₃-HCl (3026.4 mg, 36.24 mmol) was added, and the mixture was stirred at 120 °C for 12 hours. The reaction is terminated using TLC (Rf = 0.4, ethyl acetate / hexane = 1/1 (v / v)) and the temperature of the reaction solution is lowered to room temperature. After evaporation of the pyridine solvent, the product was dissolved in water and ethylacetate for 30 minutes using ultrasonic waves. The product was extracted twice with ethyl acetate and washed with saturated NaHCO3 solution. The extracted solution is dehydrated with anhydrous magnesium sulfate, and the solvent is evaporated and recrystallized using methanol and nucleic acid. The product was dried in a vacuum pump and red solid A3051 (326 mg) can be obtained in 47.94% yield. 1H NMR(400 MHz, DMSO-d6) δ 11.36 (s, 2H), 8.80 (s, 1H), 8.08 (d, 1H, J = 7.7 Hz), 7.46 - 7.41 (m, 2H), 7.07 - 6.99 (m, 2H), 4.38 (s,3H).

### Synthesis of 5-methoxylindirubin-3'-oxim (A3334)

### Synthesis of intermediate product, 5'-methoxy-[2,3'-biindolinylidene]-2',3-dione

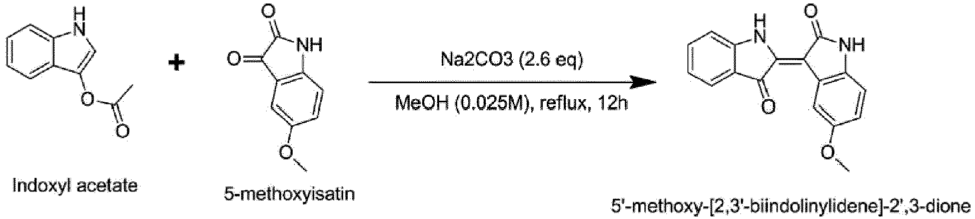

5-methoxyisatin (1000 mg, 5.65 mmol) was added to a 250-mL round bottom flask and dissolved in methanol (225 mL), followed by the addition of indoxyl acetate (989 mg, 5.65 mmol) and sodium carbonate (Na₂CO₃) (1496 mg, 14.11 mmol), and the mixture is stirred at 65 °C for 12 hours. The reaction is terminated using TLC (Rf = 0.4, ethyl acetate / hexane = 1/2 (v / v)) and the product is allowed to cool down on ice until a lump of crystals is formed. After the crystals are formed and the solvent is removed by filtration, the filtrate is discarded, and the product is washed several times with a solvent (ethanol / water = 1/1 (v / v)). The product was filtered and dried in a vacuum pump and used in the next step without further purification.

### Synthesis of A3334

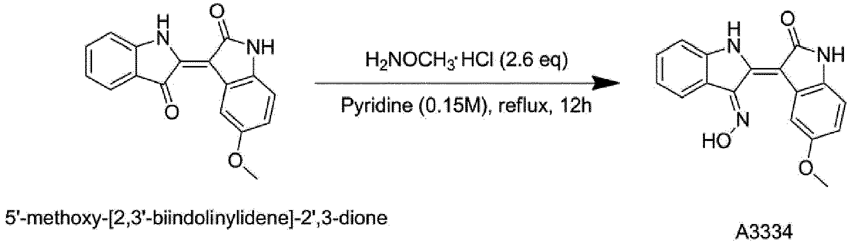

5'-methoxy-[2, 3'-biindolinylidene]-2', 3-dione) (670 mg, 2.29 mmol) was added to a 100-mL round bottom flask and dissolved in pyridine (27 ml), followed by addition of H₂NOCH₃-HCl (3186 mg, 45.85 mmol) and the mixture was stirred at 120°C for 12 hours. The reaction is terminated using TLC (Rf = 0.5, ethyl acetate / hexane = 1/1 (v / v)) and the temperature of the reaction solution is lowered to room temperature. After evaporation of the pyridine solvent, the product was dissolved in water and ethylacetate for 30 minutes using ultrasonic waves. The product was extracted twice with ethyl acetate and washed with saturated NaHCO₃ solution. The extracted solution is dehydrated with anhydrous magnesium sulfate, the solvent is evaporated and recrystallized using methanol and nucleic acid. The product was dried in a vacuum pump and red solid A3334 can be obtained (420 mg) in 59% yield.

### Indirubin-3'-oxime (I3O or IO)

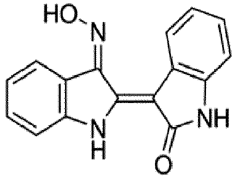

### (Z)-4-(5-((1-(4-nitrophenyl)-1H-pyrazol-4-yl)methylene)-4-oxo-2-thioxothiazolidin-3-yl) butanoic acid (A2763)

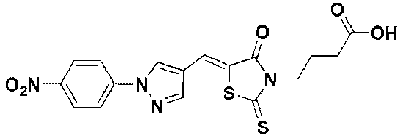

### Comparative example 3. (Z)-3-(5-((5-(4-cyanophenyl)furan-2-yl)methylene)-2,4-dioxothiazoli din-3-yl) propanoic acid (A2764)

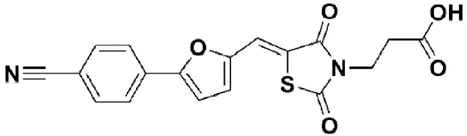

### 5,6-dichloroindirubin-3'-propyloxim (A3486)

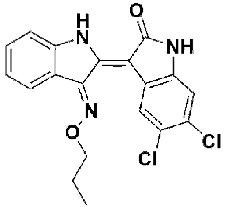

### 5. 6-chloroindirubin-3'-benzyloxime (A3538)

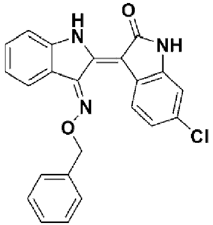

### (Z)-5'-bromo-6'-nitro-[2,3'-biindolinylidene]-2',3-dione (A2785)

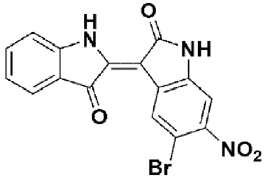

### 6-Nitro-5-trifuloromethoxyindirubin-3'-methoxime (A2794)

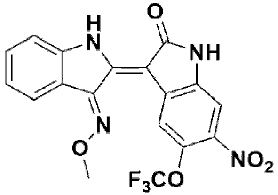

**Screening for compounds that inhibit the CXXC5-DVL interaction.** To initially identify small molecules that inhibited the CXXCS-DVI, interaction, chemical libraries (2,280 compounds: 1,000 from ChemDiv and 1,280 from SigmaLOPAC) were screened by *in vitro* binding assay that was previously described. See e.g., Kim et al. (2015) CXXC5 is a negative-feedback regulator of the Wnt/beta-catenin pathway involved in osteoblast differentiation. CELL. DEATH. DIFFER. 22, 912-920. Briefly, 5 mg/ml purified DVL-PDZ domain was incubated in each well of a 96-well Maxibinding Immunoplate (SPL, Seoul, Korea) at 4°C for 16 h. After the addition of 10 µM FITC-tagged PTD-DBMP to each well, each compound in the chemical library or control (DMSO) was treated to the well at a final concentration of 30 µM. The fluorescence intensity was measured using a Fluorstar Optima microplate reader (BGM Lab Technologie, Ortenberg, Germany) and normalized as follows: "('compounds-treated group'-'blank')/('DMSO-treated control'-'blank')*100".

Nineteen compounds were selected as initial hits which suppress the CXXC5-DVL (PDZ-DVL-PTD-DBMP (FITC) interaction more than 90%, and their capabilities of activation of the Wnt/β-catenin pathway was confirmed by using the HEK293 cells harboring the pTOFOplash reporter in its chromosome. Among these compounds, indirubin analogs including BIO and 130 were identified. A summary of the high-throughput screening results is provided in **Table 2**.

**Table 2. Summary of high-throughput screening results**

| Category | Parameter | Description |
|---|---|---|
| Assay | Type of assay | *In vitro* binding assay |
| | Target | CXXC5-DVL interaction |
| | Primary measurement | Fluorescence intensity |
| | Key reagents | FITC-tagged PTD-DBM peptide and DVL-PDZ domain protein |
| | Assay protocol | The protocol was provided in "Small molecule inhibitors of the Dishevelled-CXXC5 interaction are new drug candidates" |
| Library | Library size | 2280 compounds assayed in 96-well plates as single compounds at 10 mM in DMSO |
| | Library composition | Small molecules |
| | Source | ChemDiv and Sigma LOPAC 1280 |
| Screen | Format | 96-well black polystyrene plates |
| | Concentration(s) tested | Constant 30 µM concentration, 0.3% DMSO |
| | Plate controls | DMSO-treated group |
| | Reagent/compound dispensing system | Reagents and compounds were dispensed manually |
| | Detection instrument and software | FLUOstar OPTIMA (BMG LABTECH) |
| | Assay validation/QC | Z-factor > 0.7 |
| | Correction factors | N/A The sample result was normalized to positive |
| | Normalization | control and is represented as % CXXC5-DVL interaction |
| Post-HTS analysis | Hit criteria | < 10% inhibition |
| | Hit rate | 1% |

**Table 3. List of top-ranked compounds screened through an in vitro CXXC5-DVL PPI inhibition assay using chemical libraries that includes 2,280 small molecules**

| **Compound** | **Structure** | **Empirical Formula** | **CXXC5-DVL inhibitory activity (%)** |
|---|---|---|---|
| 1 | 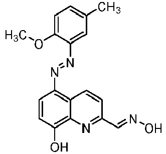 | C₁₈H₁₆N₄O₃ | 85.25 |
| 2 | 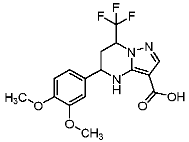 | C₁₆H₁₆F₃N₃O₄ | 90.63 |
| 3 | 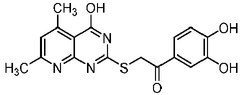 | C₁₇H₁₅N₃O₄S | 88.19 |
| 4 | 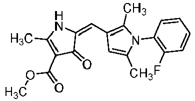 | C₂₀H₁₉FN₂O₃ | 87.58 |
| 5 | 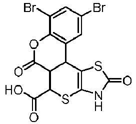 | C₁₄H₇Br₂NO₅S₂ | 90.48 |
| 6 | 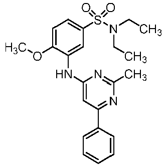 | C₂₂H₂₆N₄O₃S | 58.24 |
| 7 | 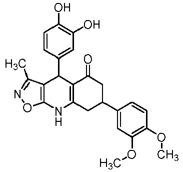 | C₂₅H₂₄N₂O₆ | 90.87 |
| 8 | 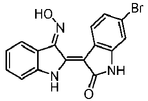 | C₁₆H₁₀BrN₃O₂ | 103 |
| 9 | 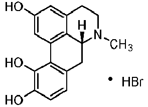 | C₁₇H₁₇NO₃·HBr | 89.41 |
| 10 | 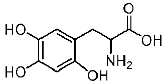 | C₉H₁₁NO₅ | 90.32 |
| 11 | 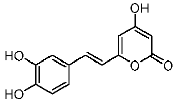 | C₁₃H₁₀O₅ | 90.05 |
| 12 | 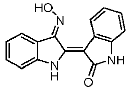 | C₁₆H₁₁N₃O₂ | 89 |
| 13 | 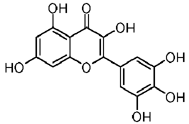 | C₁₅H₁₀O₈ | 85.22 |
| 14 | 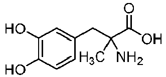 | C₁₀H₁₃NO₄ | 85.22 |
| 15 | 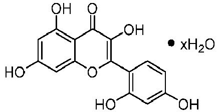 | C₁₅H₁₀O₇ · xH₂O | 85.41 |
| 16 | 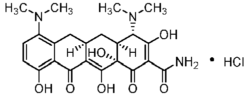 | C₂₃H₂₇N₃O₇ · HCl | 87.9 |
| 17 | 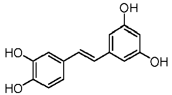 | C₁₄H₁₂O₄ | 85.25 |
| 18 | 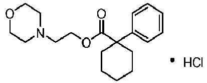 | C₁₉H₂₇NO₃ · HCl | 86.81 |
| 19 | 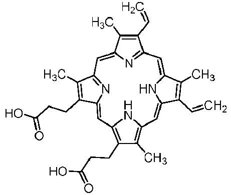 | C₃₄H₃₄N₄O₄ | 89.44 |

Indirubin analog compounds (#8 and #12; **Table 3**) were repeatedly identified as CXXC5-DVL inhibitors and showed effectiveness in the activation of Wnt/β-catenin pathway using reporter assay. To obtain functionally improved compound, about 60 indirubin derivatives were newly synthesized by replacing the functional groups at the R₁ and R₂ sites of the indirubin backbone based on the structure of indribin-3'-oxim (I3O) (#12; **Table 3**). Newly synthesized indirubin derivatives are described in **Tables 4-6** and the structures of these compounds are shown **FIG. 30** **and** **FIG. 49**.

**Table 4. List of chemically synthesized compounds shown to at least partially inhibit the activity of CXXC5-DVL. C: control**

| | Compound # | IUPAC name | R1 | | | | R2 |
|---|---|---|---|---|---|---|---|
| | | | 4 | 5 | 6 | 7 | 3' |
| C | Indirubin | Indirubin | H | H | H | H | O |
| 1 | A2735 | 6-Chloro-5-nitroindirubin | H | NOz | Cl | H | O |
| 2 | A2736 | 6-Chloro-5-nitroindirubin-3'-oxime | H | NOz | Cl | H | NOH |
| 3 | A2941 | 5,6-dichloroindirubin | H | Cl | Cl | H | O |
| 4 | A3050 | 5,6-dichloroindirubin-3'-oxime | H | Cl | Cl | H | NOH |
| 5 | A3051 | 5,6-dichloroindirubin-3'-methoxime | H | Cl | Cl | H | NOCH₃ |
| 6 | A3471 | 5,6-dichloroindirubin-3'-ethyloxime | H | Cl | Cl | H | NOCH₂CH₃ |
| 7 | A3486 | 5,6-dichloroindirubin-3'-propyloxime | H | Cl | Cl | H | NOCH₂CH₂CH₃ |
| 8 | A2813 | 6-Chloroindirubin | H | H | Cl | H | O |
| 9 | A2853 | 6-Chloroindirubin-3' -oxime | H | H | Cl | H | NOH |
| 10 | A2793 | 6-Chloroindirubin-3 '-methoxime | H | H | Cl | H | NOCH₃ |
| 11 | A3473 | 6-Chloroindirubin-3 '-ethyloxime | H | H | Cl | H | NOCH₂CH₃ |
| 12 | A3481 | 6-Chloroindirubin-3'-propyloxime | H | H | Cl | H | NOCH₂CH₂CH₃ |
| 13 | A3538 | 6-Chloroidirubin-3'-benzyloxime | H | H | Cl | H | NOCH₂Ph |
| 14 | A2851 | 5 -Chloroindirubin | H | Cl | H | H | O |
| 15 | A3439 | 5-Chloroindirubin-3' -oxime | H | Cl | H | H | NOH |
| 16 | A3440 | 5-Chloroindirubin-3 '-methoxime | H | Cl | H | H | NOCH₃ |
| 17 | A3470 | 5-Chloroindirubin-3 '-ethyloxime | H | Cl | H | H | NOCH₂CH₃ |
| 18 | A3485 | 5-Chloroindirubin-3 ' -propyloxime | H | Cl | H | H | NOCH₂CH₂CH₃ |
| 19 | A3536 | 5-Chloroindirubin-3'-benzyloxime | H | Cl | H | H | NOCH₂Ph |
| 20 | A3331 | 5-Methoxyindirubin | H | OCH₃ | H | H | O |
| 21 | A3334 | 5-Methoxyindirubin-3'-oxime | H | OCH₃ | H | H | NOH |
| 22 | A3441 | 5-Methoxyindirubin-3'-methoxime | H | OCH₃ | H | H | NOCH₃ |
| 23 | A3484 | 5-Methoxyindirubin-3'-ethyloxime | H | OCH₃ | H | H | NOCH₂CH₃ |
| 24 | A3483 | 5- Methoxyindirubin-3'-proyloxime | H | OCH₃ | H | H | NOCH₂CH₂CH₃ |
| 25 | A3330 | 5-Methylindirubin | H | CH₃ | H | H | O |
| 26 | A3335 | 5-Methylindirubin-3'-oxime | H | CH₃ | H | H | NOH |
| 27 | A3442 | 5 -Methylindirubin-3' -methoxime | H | CH₃ | H | H | NOCH₃ |
| 28 | A3533 | 5-Methylindirubin-3' -ethyloxime | H | CH₃ | H | H | NOCH₂CH₃ |
| 29 | A3534 | 5-Methylindirubin-3 '-propyloxime | H | CH₃ | H | H | NOCH₂CH₂CH₃ |
| 30 | A3535 | 5-Methylindirubin-3 '-benzyloxime | H | CH₃ | H | H | NOCH₂Ph |

**Table 5. List of chemically synthesized compounds shown to at least partially inhibit the activity of CXXC5-DVL. C: control**

| | Compound # | IUPAC name | R1 | | | | R2 |
|---|---|---|---|---|---|---|---|
| | | | 4 | 5 | 6 | 7 | 3' |
| C | 130 | Indirubin-3 ' -oxime | H | H | H | H | NOH |
| 31 | A3332 | 5 -Bromoindirubin | H | Br | H | H | O |
| 32 | A3390 | 5-bromoindirubin-3'-oxime | H | Br | H | H | NOH |
| 33 | A3391 | 5-bromoindirubin-3'-methoxime | H | Br | H | H | NOCH₃ |
| 34 | A3472 | 5-bromoindirubin-3'-ethyloxime | H | Br | H | H | NOCH₂CH₃ |
| 35 | A3482 | 5-bromoindirubin-3 '-propyloxime | H | Br | H | H | NOCH₂CH₂CH₃ |
| 36 | A3537 | 5-bromoindirubin-3'-benzyloxime | H | Br | H | H | NOCH₂Ph |
| 37 | A2784 | 5 -Chloro-6-nitroindirubin | H | Cl | NO₂ | H | O |
| 38 | A2848 | 5-Chloro-6-nitroindirubin-3' -oxime | H | Cl | NO₂ | H | NOH |
| 39 | A3049 | 5-Chloro-6-nitroindirubin-3'-methoxime | H | Cl | NO₂ | H | NOCH₃ |
| 40 | A2849 | 5-Nitroindirubin | H | H | NO₂ | H | O |
| 41 | A2854 | 5-Nitroindirubin-3'-oxime | H | H | NO₂ | H | NOH |
| 42 | A3333 | 5-Trifluoromethoxyindirubin | H | OCF₃ | H | H | O |
| 43 | A3392 | 5-Trifluoromethoxyindirubin | H | OCF₃ | H | H | NOH |
| 44 | A3393 | 5-Trifluoromethoxyindirubin-3'-methoxime | H | OCF₃ | H | H | NOCH₃ |
| 45 | A2942 | 6-Methylindirubin | H | H | CH₃ | H | O |
| 46 | A2943 | 6-Methylindirubin-3'-oxime | H | H | CH₃ | H | NOH |
| 47 | A2944 | 6-Methylindirubin-3 ' -methoxime | H | H | CH₃ | H | NOCH₃ |
| 48 | A2852 | 6-Methyl-5-nitroindirubin | H | NO₂ | CH₃ | H | O |
| 49 | A3336 | 6-Methyl-5-nitroindirubin-3 ' -oxime | H | NO₂ | CH₃ | H | NOH |
| 50 | A3337 | 6-Methyl-5 -nitroindirubin-3' -methoxime | H | NOz | CH₃ | H | NOCH₃ |
| 51 | A2802 | 6-Nitro-5-Trifluoromethoxyindirubin | H | OCF₃ | NO₂ | H | O |
| 52 | A2801 | 6-Nitro-5-trifluoromethoxylndirubin-3'-oxime | H | OCF₃ | NO₂ | H | NOH |
| 53 | A2794 | 6-Nitro-5-trifluoromethoxyindirubin-3'-methoxime | H | OCF₃ | NO₂ | H | NOCH₃ |
| 54 | A3307 | Indirubin-7-carboxylic acid | H | H | H | COOH | O |
| 55 | A3309 | Indirubin-7-carboxylic acid-3'-oxime | H | H | H | COOH | NOH |
| 56 | A3309 | 7-Trifluoromethylindirubin | H | H | H | CF₃ | O |
| 57 | A3310 | 7-Trifluoromethylindirubin-3'-oxime | H | H | H | CF₃ | NOH |
| 58 | A3311 | 4-Bromoindirubin | Br | H | H | H | O |
| 59 | A2783 | 4-Bromoindirubin-3' -oxime | Br | H | H | H | NOH |
| 60 | A3312 | 4-Chloroindirubin | H | H | H | H | O |

**Table 6. List of chemically synthesized compounds shown to at least partially inhibit the activity ofCXXC5-DVL**

| No. | Compound # | IUPAC Name | 3' moiety |
|---|---|---|---|
| 1 | A4664 | 5-Fluoroindirubin | O |
| 2 | A4665 | 5-Fluoroindirubin-3'-oxime | NOH |
| 3 | A4666 | 6-Bromoindirubin | O |
| 4 | A4667 | 6-Bromoindirubin-3 '-oxime | NOH |

Clinical characteristics of metabolic diseases including insulin resistance and obesity are related to complex interrelated pathological progressions. Understanding the molecular mechanism of the overall pathogenesis could provide a strategy for reversing the metabolic diseases. The Wnt/β-catenin pathway plays a role in the pathological process, and its response genes can be used as therapeutic targets for the metabolic diseases. CXXCS-type zinc finger protein 5 (CXXC5), a negative regulator of the Wnt/β-catenin pathway functioning via Dishevelled (Dvl) binding. As provided herein, the functional role of CXXCS in metabolic diseases and the relationship between CXXCS and Wnt/β-catenin signaling were investigated in human adipose tissue and a mouse model. CXXC5 is highly expressed in visceral adipose tissues of obese-diabetes patient tissues. *Cxxc5*^{*-*/*-*} mice fed a high-fat diet were abrogated hyperglycemia, inflammation, gluconeogenesis, lipogenesis, or insulin resistance. These results provided herein suggest CXXCS as a therapeutic target for treatment of obesity-related diabetes. A small molecule inhibiting the CXXC5-Dvl interaction restored the metabolic phenotypes as observed in HFD-fed *Cxxc5*^{*-*/*-*} mice. Administration of at least one of compounds and/or compositions described herein lowered the fasting blood glucose level of HFD-fed mice. Different from sitagliptin or metformin, the glucose controlling effect persisted for weeks. These long-lasting effects correlated with adipose tissue remodeling in adaptive energy homeostasis accompanying improvement of insulin resistance and inflammation. In addition, in a late stage of diabetes, the compounds and/or compositions described herein contributed to the regeneration of both mass and functions of pancreatic β-cells. Overall, inhibition of CXXCS activity by a small molecule-mediated interference of Dvl binding can be a potential therapeutic approach for the treatment of metabolic diseases including obesity and diabetes.

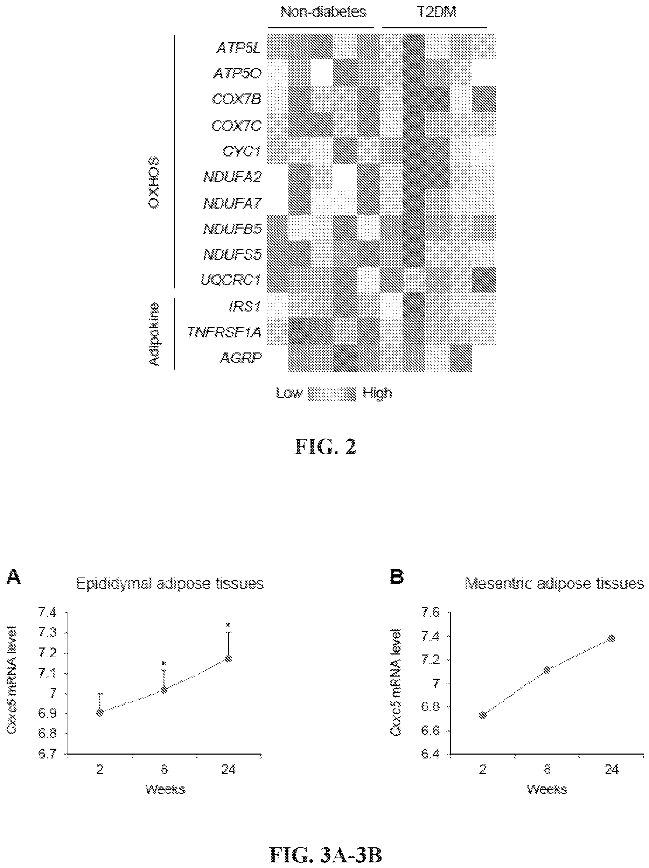

**Elevated CXXC5 levels in visceral fat tissues from obese diabetic patients**. The clinical implication of the functional roles of CXXCS in metabolic diseases and the relationship between CXXCS and Wnt/β-catenin signaling was investigated in human adipose tissues. Referring to **FIG. 1A-1I**, the results from the microarray analyses showed that the mRNA levels of the Wnt/β-catenin signaling target genes including *TCF7L2*, *WISP-1*, *IGF-1*, and *PPARδ* were higher in the visceral adipose tissues from non-diabetes subjects than those from T2DM patients (**FIG. 1A-1B**). T2DM status was confirmed by downregulated oxidative phosphorylation and upregulated adipokine genes in the same subjects (FIG. 2). The mRNA level of *WISP-1,* a major adipogenic transcription factor that suppresses *PPARγ* and *C*/*EBPα,* was also lower in T2DM patients (**FIG. 1A**). Contrarily, the mRNA levels of *CXXCS* and *DKK-1,* the negative regulators of Wnt/β-catenin signaling, were high in the visceral adipose tissues of obese-T2DM patients (**FIG. 1A**, **1C**). The involvement of CXXCS in the pathogenesis of metabolic diseases especially T2DM and obesity was indicated by the increment of mRNA level of *CXXCS* in the adipose tissues of insulin resistant, T2DM, and obese patients (**FIG. 1D-1F**). The *CXXC4,* an analog of *CXXC5* that also interacts with Dvl using an identical motif⁴⁰, was not expressed in the visceral adipose tissues regardless of pathological status **(****FIG. 1A****)**. CXXC5 was not expressed in the visceral adipose tissues of the lean non-diabetes subjects, whereas it was highly expressed in the obesity, diabetes, or obese-diabetes patients, especially in the cytosol **(****FIG. 1G****)**. Contrarily, β-catenin was expressed in both the nucleus and cytosol of the cells for the lean non-diabetes adipose tissue but was mostly abolished in cells from the obese-diabetes patients **(****FIG. 1G****)**. Quantitative analyses confirmed inverse expression patterns of β-catenin and CXXCS in visceral adipose tissues **(****FIG. 1G**; right panels). Moreover, the CXXCS expression level was correlated with the body mass index (BMI) **(****FIG. 1H****)**, whereas the β-catenin expression level was inversely correlated with the BMI **(****FIG. 1I**) in visceral fat tissues. Overall, CXXC5 was specifically induced in the subcutaneous and visceral adipose tissues of T2DM patients.

***Cxxc5*^{*-*/*-*} mice resists diet-induced obesity and metabolic diseases**. Referring to FIG. 3A-3B, the mRNA level of Cxxc5 was increased in epididymal white adipose tissue (epiWAT) and mesenteric WAT from HFD-fed mice **(****FIG. 3A-3B****)**. The systemic roles of CXXC5 in obesity-related metabolic diseases were investigated using *Cxxc5*^{+/+} and Cxxc5^{-/-} mice fed either HFD or NCD for 8 weeks, respectively.

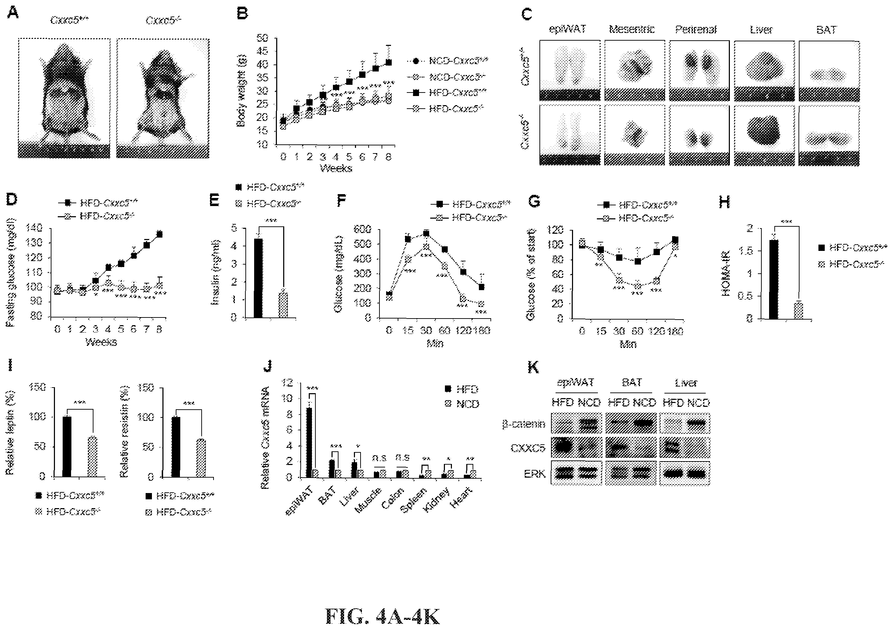

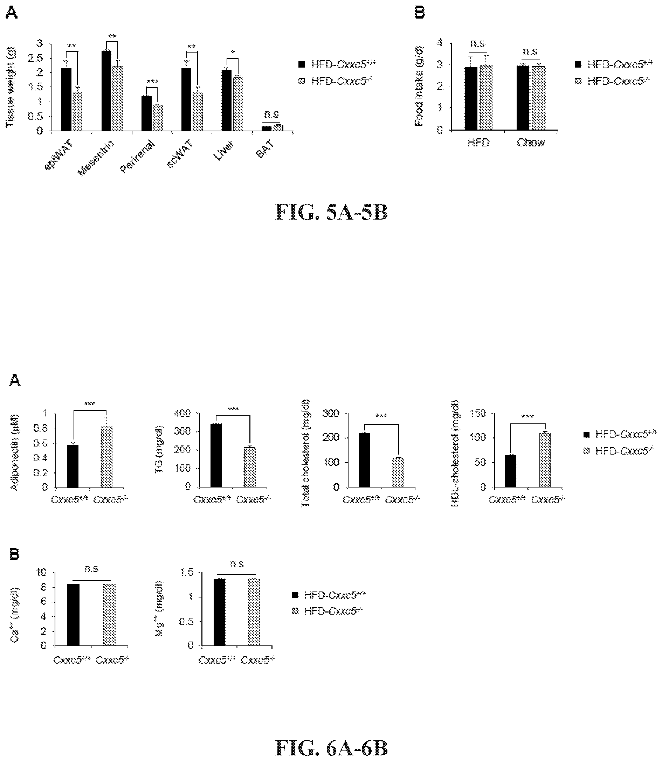

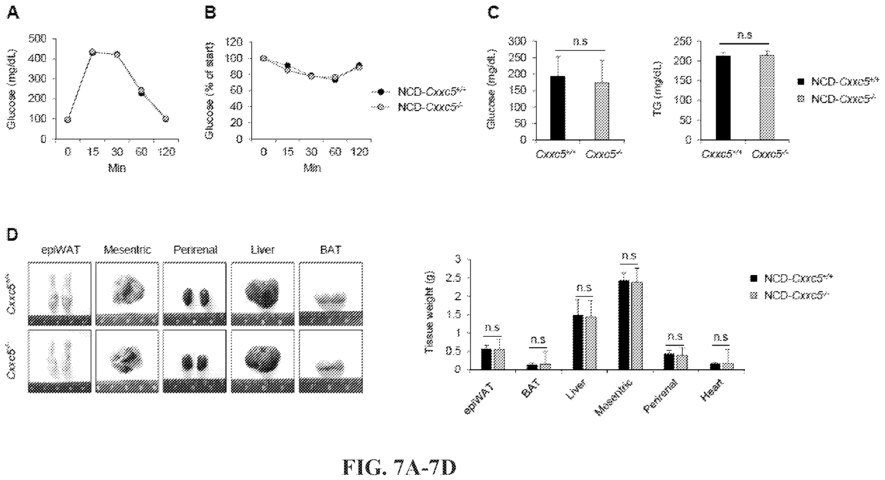

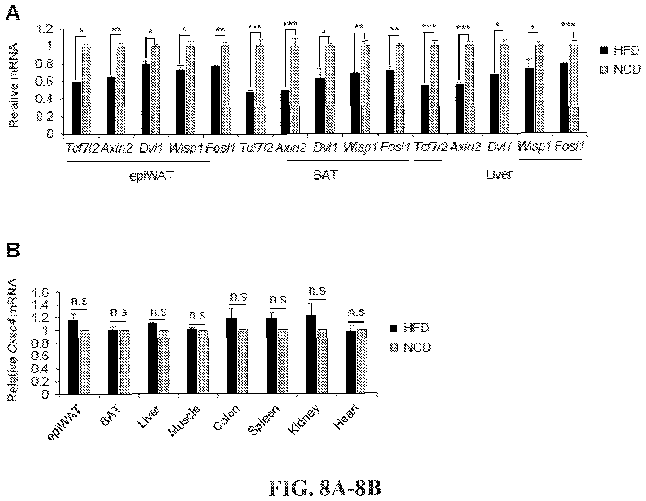

HFD-induced obesity did not occur in Cxxc5^{-/-} mice **(****FIG. 4A****)** and their body weights were similar to those of *Cxxc5*^{+/+} mice fed the NCD **(****FIG. 4B****)**. The lower body weights of HFD-fed Cxxc5^{-/-} mice were accounted for by the lower weights of the visceral adipose and liver tissues (**FIG. 4C** and **FIG. 5A**) without alteration in food intake compared with the HFD-fed *Cxxc5*^{+/+} mice **(****FIG. 5B****)**. Consistent with the reduced obesity, HFD-fed Cxxc5^{-/-} mice exhibited markedly lower levels of fasting glucose and insulin (**FIG. 4D-4E**). Moreover, HFD-fed Cxxc5^{-/-} mice showed significantly improved glucose tolerance and insulin sensitivity (**FIG. 4F-4I**) with improved metabolic parameters including leptin, resistin, adiponectin, triglyceride (TG), total cholesterol, and high density lipoprotein (HDL)-cholesterol levels **(****FIG. 4I** **and** **FIG. 6A**), whereas vital minerals such as calcium (Ca⁺⁺) and magnesium (Mg⁺⁺) had similar levels between groups (FIG. 6B). In contrast, on an NCD, *Cxxc5*^{*+*/*+*} and Cxxc5^{-/-} mice did not show any differences in insulin sensitivity, serum levels of glucose and TG, and visceral fat weights (**FIG. 7A-7D**). We also monitored the levels of Cxxc5 and Wnt/β-catenin signaling target genes in various tissues. The mRNA expression levels of Cxxc5 were significantly and selectively upregulated in metabolic tissues such as epiWAT, brown adipose tissue (BAT), and liver tissue in HFD-fed mice compared to NCD-fed mice **(****FIG. 4J****)**. In contrast, mRNA expression levels of the Wnt/β-catenin signaling target genes, including Tcf7l2, Axin2, Dvl1, Wisp1, and Fosl1, were downregulated in epiWAT, BAT, and liver tissues in HFD-fed mice **(****FIG. 8A****)**. These inverse regulation patterns were confirmed by immunoblot analyses of β-catenin and Cxxc5 in epiWAT, BAT, and liver tissues in HFD-fed mice (**FIG. 4K**). The specificity of the Cxxc5 expression was further supported by the absence of Cxxc4 induction in the adipose and liver tissues of HFD-fed mice **(****FIG. 4J** vs **FIG. 8B****)**. Overall, Cxxc5 was specifically induced in the epiWAT, BAT, and liver tissues of HFD-fed Cxxc5 mice.

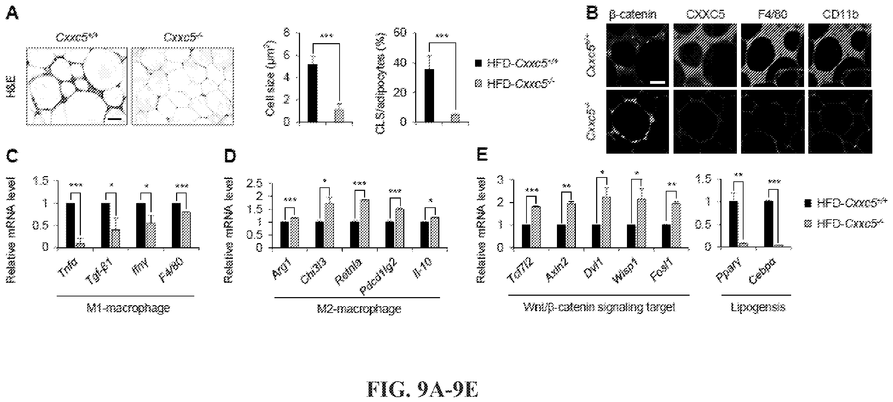

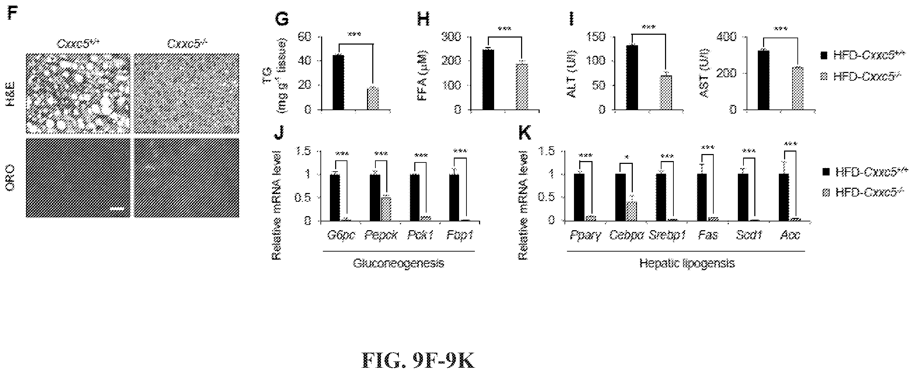

**Ablation of *Cxxc5* attenuates adipocyte hypertrophy and improves hepatic glucose homeostasis in HFD-fed mice**. HFD-fed *Cxxc5*^{*-*/*-*} mice had smaller adipocytes compared to HFD-fed *Cxxc5*^{*+*/*+*} mice **(****FIG. 9A****)**. HFD-fed *Cxxc5*^{*-*/*-*} mice also exhibited fewer crown-like structures (CLSs) and F4/80⁺ and CD11b⁺ proinflammatory macrophages with repressed expression of M1 macrophage markers such as *Tnf-α, Tgf-β1, Ifnγ,* and *F4*/*80* **(****FIG. 9A-9C****)**. In contrast, expression levels of the M2 macrophage markers including *Arg, Chi3l3, Retnla, Pdcd1lg2,* and *Il-10* were increased in HFD-fed *Cxxc5*^{*-*/*-*} mice **(****FIG. 9D****)**. The expression levels of the Wnt/β-catenin signaling target genes such as *Tcj712, Axin2, Dvl1, Wisp1,* and *Fosl1* were increased, whereas the late adipocyte differentiation markers such as *Ppary* and *Cebpα* were decreased in the epiWAT of HFD-fed *Cxxc5*^{*-*/*-*} mice **(****FIG. 9E****)**. Compared with HFD-fed *Cxxc5*^{+/+} mice, HFD-fed *Cxxc5*^{*-*/*-*} mice showed decreased fat deposition in the liver and reduced TG contents **(****FIG. 9F** and **9G****)**. Moreover, serum levels of free fatty acids (FFA), alanine aminotransferase (ALT), and aspartate aminotransferase (AST) were decreased in HFD-fed *Cxxc5*^{*-*/*-*} mice **(****FIG. 9H-9I****)**. The expression levels of gluconeogenic and hepatic lipogenic genes were markedly reduced in the livers of HFD-fed *Cxxc5*^{*-*/*-*} mice **(****FIG. 9J-9K****)**. Thus, HFD-induced adipocyte hypertrophy with inflammation and hepatic lipid homeostasis were abrogated in *Cxxc5*^{*-*/*-*} mice.

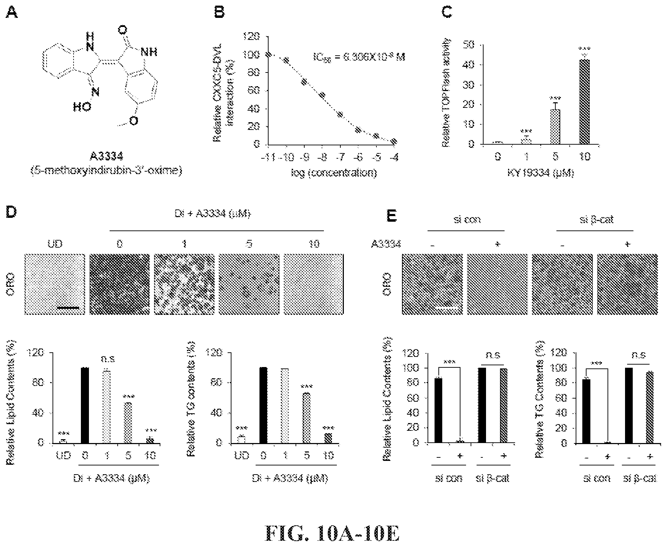

**A3334, a small molecule inhibits CXXC5-Dvl interaction, inhibits HFD-induced metabolic diseases with a long-lasting glucose controlling effect.** To test whether the blockade of CXXC5, especially its Dvl binding function, restored Wnt/β-catenin signaling and exerted anti-diabetes properties, we used a small molecule obtained by the *in vitro* screening system to monitor the interaction between the PDZ domain of Dvl and the protein transduction domain-fused Dvl binding motif peptide (PTD-DBMP). The small molecules inhibiting CXXC5-Dvl protein-protein interaction (PPI) were obtained by initial screening of a small-molecule library composed of 5,000 compounds. Several of the candidates that were used in the study were obtained by selection from 60 chemically synthesized analogs of indirubin 3'-oxime, a PTD-DBMP PPI inhibiting compound that was screened two times at the initial screening (*see* supra). Among the positive candidates, 5-methoxyindirubin-3'-oxime (A3334) (FIG. 10A) were selected after consideration of its high capabilities of CXXC5-Dvl PPI inhibition (IC₅₀ value = 6.306 × 10⁻⁸ M) with a dose-dependent increment of the TOPFlash reporter activity (FIG. 10B-10C). A3334 efficiently inhibited the adipogenic differentiation of 3T3-L1 preadipocytes via the Wnt/β-catenin signaling pathway as shown by the abolishment of its inhibitory effect by β-catenin knock down (FIG. 10D-10E).

To investigate the systemic effects of the CXXC5-Dvl PPI inhibitor, A3334 (25 mg kg⁻¹), sitagliptin (50 mg kg⁻¹), or metformin (100 mg kg⁻¹) was orally administered daily for 5 days at weeks 8 and 12 on HFD mice **(****FIG. 11A****)**. After a week of initial treatment, the fasting glucose level was lower in the A3334 treatment than in the sitagliptin and metformin treatment groups **(****FIG. 11B** and **12A****)**. The fasting glucose level started to increase after a week; however, the increment in the A3334 treatment group was much lower than that in the sitagliptin and metformin treatment groups **(****FIG. 11B** and **12A****)**. The persistent effectiveness of fasting glucose control by A3334 was critical during the second application at 4 weeks after the initial application **(****FIG. 11B** and **12A****)**. The glucose-lowering effect was constantly maintained for 4 more weeks after treatment with A3334, whereas this prolonged effect was not shown in the sitagliptin and metformin treatment groups; therefore, fasting glucose levels were increased in a pattern similar to the initial termination of the drugs **(****FIG. 11B** and **12A****)**. The A3334 treatment significantly improved the systemic glucose tolerance and insulin resistance compared with the sitagliptin and metformin treatments in HFD-fed mice **(****FIG. 11C- 11D** and **12B-12C)**. Mice administered A3334 showed improved insulin resistance as estimated by HOMA of insulin resistance (HOMA-IR) analyses **(****FIG. 11E-11F** and **12D-12F)**. Consistent with the enhanced insulin sensitivity by A3334 treatment in HFD-fed mice, the body weights were decreased without significant changes in food intake **(****FIG. 11G** and **13A****)**. The suppression of body weight gain by the A3334 treatment was attributed to the reduction of overall fat mass, especially in visceral tissues, subcutaneous adipose depots, and the liver **(****FIG. 11H** and **13B****)**. In addition, A3334-treated mice showed much better effects than sitagliptin- and metformin-treated mice regarding improvements of their endocrine and metabolic parameters including total cholesterol, HDL-cholesterol, TG, and adiponectin (**FIG. 11I** and **13C****-13D**) without any effect on Ca⁺⁺ and Mg⁺⁺ imbalance **(****FIG. 13E****)**. As confirmed by protein chip analyses, A3334 decreased the expression levels of various secretory proteins, including adipokines such as angiopoietin-3, DPPIV, IGFBP-5, leptin, resistin, and PAI-1 that are implicated in obesity-related insulin resistance **(****FIG. 11J****)**. Overall, A3334 had a long-lasting glucose controlling effect with an improvement in obesity-related insulin resistance and overall metabolic parameters.

**A3334 reduces inflammation and increases lipolysis in the epiWAT of HFD-fed mice.** Referring now to **FIG. 14** and **15**, the decreased obesity of HFD-fed mice after A3334 treatment could result from a reduction in inflammatory responses in the epiWAT. A3334 treatment significantly decreased the number of CLSs as well as the size of adipocytes in the epiWAT compared with that of the vehicle- and sitagliptin-treated mice **(****FIG. 14A-14B****)**. The expression and localization of the Cxxc5 protein overlapped with those of F4/80⁺ and CD11b⁺ CLSs in the epiWAT of vehicle- and sitagliptin-treated mice **(****FIG. 14B****)**. Compared to the vehicle- and sitagliptin-treated mice, Cxxc5 expression was mostly abolished and only a small amount remained in the nucleus with the elevation of nuclear β-catenin in the epiWAT of A3334-treated mice **(****FIG. 14B****)**. From the histology data, the populations of F4/80⁺ and CD11b⁺ cells decreased in the A3334-treated mice **(****FIG. 14C****)**. The mRNA expression levels of all M1 and M2 macrophage marker genes were decreased and increased in the epiWAT of A3334-treated mice, respectively **(****FIG. 14D****)**. In addition, the Wnt/β-catenin signaling target genes were upregulated with the regression of the lipogenesis genes in the epiWAT of A3334-treated mice **(****FIG. 15A-15B****)**. Different from A3334, sitagliptin did not significantly change the expression of inflammation or the Wnt/β-catenin signaling target genes **(****FIG. 14D** and **FIG. 15B****)**.

**A3334 reduces hepatic steatosis and improves glucose homeostasis.** Referring now to **FIG. 16**, the hypertrophic adipose tissue releases excess FFAs, which are taken up by hepatocytes and stored as TG, resulting in hepatic steatosis and insulin resistance. The liver tissues from HFD-fed A3334-treated mice did not form lipid droplets with a reduction in the hepatic TG levels (FIG. 16E-16F). The HFD-induced increment of factors indicating liver damage such as ALT, AST, and FFA was mostly suppressed by the A3334 treatment and these protective effects were more significant than those shown from the sitagliptin treatment (FIG. 16G-16H). Consistent with the hepatic glucose homeostasis effects, the expression of lipogenesis and gluconeogenesis genes were repressed in the liver of A3334-treated mice with the induction of Wnt/β-catenin signaling target genes **(****FIG. 16A-16C****)**. Overall, A3334 improved the general metabolic phenotypes as well as suppressed inflammation, which was not obtained from the use of sitagliptin.

**A3334 promotes energy expenditure through the enhancement of thermogenic activity of brown- and beige-fat tissues.** Referring now to **FIG. 17**, the reduction in adipose tissue mass without any alteration in food intake indicated a potential enhancement in energy expenditure in HFD-fed A3334-treated mice. HFD-fed A3334-treated mice consumed higher oxygen (VO₂) and exhaled more carbon dioxide (VCO₂) during both light and dark periods than the vehicle-treated mice **(****FIG. 17A-17B****)**. HFD-fed A3334-treated mice had higher energy expenditure and more active behavior than vehicle-treated mice, especially during the day time **(****FIG. 17C-17D****)**. The lower respiratory exchange ratio in the HFD-fed A3334-treated mice revealed a higher fat usage **(****FIG. 17E****)**. Consistent with the increased energy expenditure, the protein level increment of the major thermogenin uncoupling protein 1 (UCP1) was confirmed in BAT and scWAT of HFD-fed mice administered A3334 **(****FIG. 17F****)**. In addition, scWAT from A3334-treated DIO mice exhibited elevated thermogenesis and beige-fat markers including *Cd137, Tmem26,* and *Tbx1* **(****FIG. 17G****)**. HFD-fed A3334-treated mice had significantly elevated expression levels of the key thermogenesis markers such as *Ucp1, Pgc1α, Prdm16, Elovl3,* and *Cox8b* in BAT **(****FIG. 17H****)**. These results correlate with the suppression of lipid accumulation in BAT from *Cxxc5*^{*-*/*-*} mice compared with *Cxxc5*^{+/+} mice fed the HFD **(****FIG. 18A****)**. The inhibitory effect of Cxxc5 on the transition of brown fat to white fat was further indicated by the induction of expression levels of the mitochondrial biogenesis and fatty acid oxidation genes and a decrease in the lipogenesis genes in BAT of HFD-fed *Cxxc5*^{*-*/*-*} mice **(****FIG. 18B****-A8-D)**. Therefore, A3334 promoted energy expenditure by the enhancement of the thermogenic activity of both brown and beige fat.

**A3334 reverses diabetes phenotypes and promotes pancreatic β-cell regeneration in HFD and streptozotocin (STZ)-treated diabetic mice.** Referring now to **FIG. 19-20**, the loss of functional β-cells is a crucial event in the development of diabetes. To investigate whether A3334 reverses diabetes by promoting pancreatic β-cell regeneration, we administered A3334 or sitagliptin to multiple low-dose STZ-induced diabetic mice. After 2 weeks of STZ injection, the blood glucose levels were significantly increased **(****FIG. 19A****)**. Subsequently, the application of A3334 lowered the glucose level and improved the results from the glucose tolerance test (GTT) and insulin tolerance test (ITT) of the diabetic mice, with the effects being more significant than those acquired by the application of the same amount of sitagliptin **(****FIG. 19A-19C****)**. The vehicle-treated diabetic mice had a severe loss of pancreatic β-cells with a reduction of insulin secretion **(****FIG. 19D****)**. However, diabetic mice administered A3334 showed normal islets with insulin secretion and increased expression levels of β-catenin, PCNA, and Pdx-1 **(****FIG. 19D-19H****)**. This recovery of the pathologic phenotypes was confirmed by similar positive effects in the *Cxxc5*^{*-*/*-*} diabetes mice **(****FIG. 20A-20H****)**. Therefore, the activation of Wnt/β-catenin signaling by blockade of the CXXCS function could be a strategy for pancreatic β-cell regeneration.

As disclosed herein, an approach was provided for improving overall metabolic disease phenotypes by utilizing the obese-diabetes model related to inflammation, steatosis, gluconeogenesis, lipogenesis, energy metabolism, and β-cell dysfunction. Illustrating the role of Cxxc5 in inhibiting the Wnt/β-catenin pathway related to metabolic diseases and a small molecule-mediated interference of the Cxxc5 function, an unexpected long-lasting glucose controlling effect was identified, which might lead to improvement of metabolic diseases in general.

Diabetes is one of the major metabolic diseases and the current clinically available drugs including sulfonylureas, SGLT2 inhibitors, PPARy agonists, DPP4 inhibitors, and biguanides can control blood glucose levels by acting on peripheral insulin target tissues such as the pancreas, intestine, muscle, and liver. The glucose controlling effect of these drugs is acquired by different mechanisms and is transient; thus, patients need to be prescribed these drugs throughout their lifetimes.

One example of drug candidates, A3334, which restores the suppressed Wnt/β-catenin pathway in the diet-induced obesity and diabetes models, showed an initial temporal effect similar to that of the major anti-diabetes drugs, the DPP4 inhibitor sitagliptin and the biguanide medication metformin. Sitagliptin and metformin showed an increased fasting glucose level after their second application, whereas A3334 revealed an unexpected long-lasting glucose controlling effect in HFD-fed mice. Notably, the fasting glucose level persisted for over 4 weeks after the second application. Without bound by any theory, these long-lasting effects of A3334 on glucose control could be acquired by adipose tissue remodeling, which inhibits the initial event of metabolic diseases, accompanying the suppression of inflammation, insulin resistance, and FFA and adipokine production with body weight loss of the HFD-fed mice. In accordance with the differences, the systemic immune effect or direct effect on macrophages during adipocyte hypertrophy in HFD-fed mice did not occur by administration of the peripheral tissue targeting drug, sitagliptin. The metabolic improvement effects of A3334-treated mice were acquired by the induction of metabolic genes including *Tcj7l2, Wisp1, c-Myc, Ccnd1,* and *Pparδ* by the activation of Wnt/β-catenin signaling via blockade of the CXXC5-Dvl interaction by the negative feedback mechanism. The pathological significance of the blockade of CXXC5-Dvl PPI was shown by the high induction of CXXCS in obese-diabetes patients and its reverse correlation with β-catenin, which controls the major metabolic genes such as *TCL7L2, WISP1, c-MYC, CCND1,* and *PPARδ.* The role of CXXC5 in the suppression of Wnt/β-catenin signaling and metabolic disease was correlated with high induction and its correlation with inflammation markers in the cytosol of adipocyte cells of obese-diabetes patients as well as the HFD-induced obese-diabetes mice. In the present disclosure, the role of CXXCS as a target for metabolic diseases was confirmed by observing the suppression of HFD-induced obesity with the repression of cytosolic Cxxc5 and inflammation markers with the activation of β-catenin in adipocytes. CXXC5-Dvl PPI was found to be a target for the improvement of metabolic diseases with similar phenotypes showing systemic improvement of metabolic abnormalities in both *Cxxc5*^{*-*/*-*} and A3334-treated *Cxxc5*^{+/*+*} mice fed the HFD. One of the effects of restoring the suppressed Wnt/β-catenin signaling by A3334 was the enhancement of energy metabolism, as shown by enhanced energy expenditure in the A3334-treated mice fed the HFD. Enhanced energy expenditure by the A3334-treated mice was also consistent with a substantial upregulation in the expression of genes that regulate thermogenesis and mitochondrial biogenesis in both scWAT and BAT. Furthermore, ectopic expression of UCP1 in scWAT from A3334-treated mice was linked to protecting diet-induced obesity by increased fatty acid oxidation of scWAT.

The instant approach of activating Wnt/β-catenin signaling via the inhibition of Dvl binding function of CXXCS could be highly advantageous owing to its potential usage in multiple metabolic diseases including obesity, diabetes, and potentially non-alcoholic steatohepatitis. The small molecules that targeted CXXC5-Dvl could be specific to the increased CXXCS level of the diseases as proven by no observation of metabolic disease phenotypes after A3334 administration for the NCD-fed mice, which did not induce CXXCS **(****FIG. 21****)**. We also confirmed that the CXXCS controlling effects of A3334 were specific to the metabolic diseases as CXXC4, an alternative Dvl binding protein, was not expressed in either T2DM patients or HFD-mice that exhibited increased CXXCS. As such, CXXC5, which is overexpressed in obesity and diabetes patients, could be a biomarker for detecting or diagnosing metabolic diseases.

As disclosed herein, CXXC5 overexpression plays a role as a major driver in the pathogenesis of multiple obese-related metabolic diseases. An approach for restoring the suppressed Wnt/β-catenin signaling via blockade of CXXC5-Dvl interaction is a therapeutic approach for the treatment of multiple metabolic diseases induced by CXXCS. Orally administered A3334, which can restore the multiple metabolic disease phenotypes, provides a novel approach for treating chronic metabolic diseases.

### The involvement of CXXCS in metabolic diseases and NASH

As disclosed herein, CXXC5 expression were progressively increased in the white adipocytes and the liver tissues of patients diagnosed with NASH and diabetes. Further, Wnt/β-catenin pathway target genes such as *TCF7L2,* and *FOSL1* were found to be suppressed in patients diagnosed with NASH and/or Type II diabetes. *Cxxc5*^{*-*/*-*} mice did not develop any phenotypes of metabolic diseases including obesity, diabetes, and/or NASH. The results disclosed herein suggest that CXXC5 contributes to the development of metabolic diseases. Thus, the instant disclosure provides a novel function of CXXC5-DVL interface that may lead to the treatment of metabolic diseases including, but are not limited to, obesity, diabetes, and/or NASH.

**Gene set enrichment analysis (GSEA) and heat map gene expression analyses.** The gene expression profile results were obtained by analyses of the data deposited in NCBI's Gene Expression Omnibus database (GEO) (http://www.ncbi.nlm.nih.gov/geo/), and NASH and Type II diabetes data are accessible through GEO accession numbers GSE16415 and GSE48452, respectively.

**Animals and diets**. *Cxxc5*^{*-*/*-*} mice were established in a previous study. See e.g., Kim H.Y. et al. (2015), CXXC5 is a negative-feedback regulator of the Wnt/beta-catenin pathway involved in osteoblast differentiation. CELL DEATH DIFFER 22: 912-920. *CXXCS* heterozygous mice were intercrossed for four generations to obtain littermate wild-type and CXXCS KO mice and were maintained on a C57BL/6J background. Six-week-old CXXC5 WT and CXXCS KO mice were fed an HFD for 8 weeks.

**NASH study**. Eight-week-old wild-type male C57BL/6N mice (KOATECH, Seoul, Korea) were fed on the methionine-choline deficient (MCD) diet for 7 weeks, followed by treatment with vehicle, A3334, A3051, or I3O of (25 mg kg⁻¹) sitagliptin (25 mg kg⁻¹) for another 3 weeks via daily oral gavage.

**Obesity-induced diabetes study**. Six-week-old wild-type male C57BL/6N mice were fed a HFD consisting of 60% calories (Research Diet, D12492) for 8 weeks. After then, each mouse administered on average A3334 (25 mg kg⁻¹) or sitagliptin (50 mg kg⁻¹) per day by oral gavage for 5 days on HFD. After removal of the drugs, mice were maintained on a HFD for 2 weeks. All animal protocols were approved by the Institutional Review Board of Severance Hospital, Yonsei University College of Medicine.

**Immunohistochemistry.** Paraffin-embedded tissue sections (4 µm each) were deparaffinized and rehydrated. For antigen retrieval, the slides were autoclaved in 10 mM sodium citrate buffer (pH 6.0). Sections were blocked in PBS containing 10% BSA at room temperature for 30 min. The sections were incubated overnight at 4°C with the following dilution of primary antibodies: anti-β-catenin (1:100; BD), anti-CXXC5 (1:50; Santa Cruz Biotechnology, Inc.), anti-F4/80 (1:100; Cell Signaling Technology), anti-CD11b (1:100; eBioscience), anti-PCNA (1:100; Santa Cruz Biotechnology, Inc.), anti-insulin (1: 1,000; Sigma-Aldrich), and anti-glucagon (1:2,000; Sigma-Aldrich). The slides were washed with PBS, incubated with Alexa Fluor 488- or Alexa Fluor 555-conjugated IgG secondary antibody (1:300; Molecular Probes) at room temperature for 1 h, and counterstained with DAPI (1:5,000; Boehringer Mannheim).

The images were captured using a LSM700 META confocal microscope (Carl Zeiss) after excitation with 405-, 488-, or 543-nm laser lines. To block endogenous peroxidase activity before peroxidase IHC analysis, tissues were incubated with 0.345% H₂O₂ (Samchum Chemicals) for 30 min. Before incubating sections with mouse primary antibody, mouse IgG was blocked using a M.O.M Mouse IgG blocking kit (Vector Laboratories). Sections were incubated with primary antibody overnight at 4°C with the following dilution of primary antibodies: anti-β-catenin (1: 100), anti-CXXC5 (1:50), anti-IRS1 (1: 100; Santa Cruz Biotechnology, Inc.), anti-UCP1 (1:500; Abcam), anti-Glut4 (1:100; Cell Signaling Technology), anti-PPARy (1:100; Santa Cruz Biotechnology, Inc.), anti-fibronectin (1:100; Abam), and anti-α-SMA (1: 100; Abcam). Then, sections were incubated with biotinylated anti-mouse (1:300; Dako) or biotinylated anti-rabbit (1:300; Dako) secondary antibodies for 1 h at room temperature. The samples were stained with 3, 3'-diaminobenzidine (DAB; Dako) for 3-7 min and counter stained with Mayer's hematoxylin (Muto). All incubations were conducted in humid chambers. Signals were analyzed using a bright field microscope (Nikon TE-2000U). β-Cell mass from the insulin antibody-stained and α-cell mass from the glucagon antibody-stained sections were visualized using a LSM700 META confocal microscope (Carl Zeiss).

**Immunoblot analysis**. Cells and tissues were lysed using radioimmunoprecipitation assay (RIPA) buffer (150 mM NaCl, 10 mM Tris, pH 7.2, 0.1% SDS, 1.0% Triton X-100, 1% sodium deoxycholate, and 5 mM EDTA). Samples were separated on 6-12% SDS polyacrylamide gels and transferred onto PROTRAN nitrocellulose membranes (Shleicher and Schuell Co.). After blocking with PBS containing 5% nonfat dry skim milk and 0.07% (vol/vol) Tween 20, the membranes were incubated with antibody specific for β-catenin (1: 1,000; Santa Cruz Biotechnology, Inc.), CXXC5 (1: 1,000), Erk (1:5,000; Santa Cruz Biotechnology, Inc.) at 4°C overnight. Horseradish peroxidase-conjugated anti-mouse (Cell Signaling) and anti-rabbit (Bio-Rad, Hercules, CA) secondary antibodies were used at 1:3,000 dilutions for 1 hour at room temperature. Protein bands were visualized with enhanced chemiluminescence (Amersham Bioscience, Buckinghamshire, UK) using a luminescent image analyzer (LAS-3000; Fujifilm, Tokyo, Japan).

**Hematoxylin and eosin (H&E) staining**. Dissected tissues were fixed in 4% neutral paraformaldehyde and embedded in paraffin. The paraffin sections were cut at a thickness of 4 µm and subjected to the H&E staining. Adipocyte cell size was measured in seven randomly chosen microscopic areas from three independent animals using a Nikon bright-field optical microscope (Nikon TE-2000U, Tokyo, Japan). The average adipocyte size was determined using Image J Software.

**Reverse transcription and quantitative real-time PCR.** Total RNA was extracted using Trizol reagent (Invitrogen) according to the manufacturer's instructions. 2 µg of RNA was reverse-transcribed using 200 units of reverse transcriptase (Invitrogen) in a 40-µl reaction carried out at 37 °C for 1 h. For conventional PCR analyses, the resulting cDNA (2 µl) was amplified in a 20 µl reaction mixture containing 10 mM dNTP (Takara, Shiga, Japan), 10 pmol of the primer set (Bioneer, Daejeon, Korea), and 1 unit of Taq DNA polymerase (Invitrogen). For quantitative real-time PCR analyses (qRT-PCR), the resulting cDNA (1 µl) was amplified in 10 µl reaction mixture containing iQ SYBR Green Supermix (Qiagen, Germantown, MD), 10 pmol of the primer set (Bioneer). The comparative cycle-threshold (CT) method was used, and *GAPDH* served as an endogenous control. The primer sets are listed in Table 1.

**Blood chemistry / Enzyme-linked immunosorbent assay (ELISA)**. Whole blood of mice was collected by cardiac puncture. The blood was allowed to clot for 30 min and then centrifuged for 10 min at 1,000 xg to obtain supernatant. The supernatant was subjected to measure metabolic parameters. Plasma insulin concentration was measured with an ELISA kit (Millipore). FFA concentrations in plasma were measured with an ELISA kit (Cayman Chemical), respectively. Serum chemistry variables included total cholesterol, HDL-cholesterol, glucose, triglyceride, ALT, AST, ALP, Ca⁺⁺, and Mg⁺⁺ concentration. Calibration was done using the quality control (QC) card supplied with the FUJI DRI-CHEM slides whenever slides from a new lot were used.

**Adipokine-related protein analysis**. Adipokines and hormones in mouse serum were measured using Mouse adipokine array kits (Proteome Profiler and Human Cytokine; R&D System). These assays were used to simultaneously detect the relative expression levels of 38 different obesity-related proteins. The array analysis was performed according to the manufacture's instructions. Blots were developed with enhanced chemiluminescence using a luminescent image analyzer, LAS-3000 (Fujifilm). All of data were normalized by intensity of reference spots in each membrane, as followed by manufacture's instruction.

**Glucose tolerance, insulin tolerance tests, HOMA-IR analyses**. For glucose tolerance tests (GTTs) or insulin tolerance tests (ITTs), mice were injected with D-glucose (1.5 g/kg body weight) after overnight starvation or human insulin (0.75 units/kg body weight) after 4h starvation. Tail blood was drawn at indicated time intervals, and blood glucose level was measured with a One Touch Ultra glucometer (LifeScan). Insulin function test was evaluated by HOMA-IR and was calculated as fasting plasma glucose (mmol/l) × fasting serum insulin (mU/l) /22.5.

**β-Cell and α-cell mass analyses**. All of the β-cell, α-cell and the cross-sectional area of all pancreatic tissue were quantified. Total β-cell and α-cell area and total pancreas mass for each animal was calculated as the sum of the determinations from each of the 8-10 segments of pancreas. A total of 10-15 beta cells was counted for each pancreas.

**ORO staining.** 3T3-L1 preadipocytes were seeded in a 6-well plate at a density of 3 × 10⁴ cells per well. After reaching confluence, the cells were induced to differentiate as described above. At 14 days post differentiation, the plates were washed with PBS and stained with ORO overnight. In the morning each well was washed thoroughly with water and images of the ORO staining were recorded with a Nikon bright-field optical microscope (Nikon TE-2000U, Tokyo, Japan) and quantified for further statistical analysis. For quantify lipid contents, the ORO was eluted by addition of 500 µl isopropanol containing 4% nonidet P-40 to each well and the absorbance was measured spectrophotometrically at 590 nm.

**Collagen staining.** 4 µm-liver tissues sections were stained using two common collagen staining methods. For Masson's trichrome staining, slides were fixed in Bouin's solution for 1 h. After incubation in Weigert's iron hematoxylin solution for 10 min, the slides were stained with Biebrich Scarlet-Acid Fuchsin and Aniline blue for 5 min. The collagen fibers were stained blue and the nuclei were stained black. For picrosirius red staining, sections were stained with Weigert's solution for 8 min and picrosirius red for 1 h. The collagen fibers were stained red with blue nuclei.

Nineteen compounds were selected as initial hits which suppress the CXXC5-DVL (PDZ-DVL-PTD-DBMP (FITC) interaction more than 90%, and their capabilities of activation of the Wnt/β-catenin pathway was confirmed by using the HEK293 cells harboring the pTOFOplash reporter in its chromosome. Among these compounds, indirubin analogs including BIO and 130 were identified. A summary of the high-throughput screening results is provided in **Table 2**. As disclosed herein, indirubin analog compounds (#8 and #12; **Table 3**) were repeatedly identified as CXXC5-DVL inhibitors and showed effectiveness in the activation of Wnt/β-catenin pathway using reporter assay. To obtain functionally improved compound, about 60 indirubin derivatives were newly synthesized by replacing the functional groups at the R₁ and R₂ sites of the indirubin backbone based on the structure of indribin-3'-oxim (130) (#12; **Table 3)**. Newly synthesized indirubin derivatives are described in **Tables 4-6** and the structures of these compounds are shown **FIG. 30** **and** **FIG. 49**.

**Wnt/β-catenin signaling reporter luciferase assay**. HEK293-TOP cells were seeded were seeded in 96-well plates at a density of 2.5 × 10⁴ cells per well. Cells were treated with individual chemicals at a concentration of 1, 5, 10 µM for 24 h. Total cell lysates were extracted with 25 µl of 5 × Reporter Lysis Buffer per well according to the manufacturer's instruction (Promega, Madison, WI). Luciferin (25 µl) was added and luciferase activity was measured using FLUOSTAR (BMG labtech, Offenburg, Germany).

**In vitro screening of compounds that induce adipocyte differentiation** of **3T3-L1 preadipocytes**. 3T3-L1 preadipocyte cells were seeded in six-well plates at a density of 3 × 10⁴ cells per well. The cells were grown in Dulbecco's modified Eagle medium (DMEM) with 10% bovine calf serum (BCS; Gibco) until confluent. After confluence, cells were induced to differentiate in DMEM containing 10% fetal bovine serum (FBS; Gibco) and MDI (520 µM methylisobutylxanthine (IBMX; Sigma-Aldrich), 1 µM dexamethasone (Sigma-Aldrich) and 167 nM insulin (Gibco)) with or without small molecules. Each small molecule was used at a concentration of 10 µM. On day 4, the medium was replaced with DMEM containing 10% FBS with or without 130 or A3334. The medium changed with fresh identical medium every 2 days to day 14 of post-induction. Cells were incubated at 37°C in a 5% CO₂ environment.

**Cell viability assay**. HEK293-TOP cells were seeded in 96-well plates at a density of 2.5 × 10⁴ cells per well and treated with the respective chemicals at a concentration of 20 µM. After 24 h, 50 µl Cell Titer (Promega) was added to each well and incubated for 10 min at room temperature (RT). The luciferase reporter assay was performed as described above.

In the instant disclosure, it was found that a negative feedback regulator of the Wnt/β-catenin pathway, CXXC5, gradually increased with suppression of β-catenin in white adipocytes and liver tissues of NASH and Type II diabetes patients **(****FIG. 22** and **FIG. 23****)**.

**CXXC5 overexpression as a biomarker for diagnosis of NASH and Type II diabetes patients**. Referring now to **FIG. 22A**, CXXC5 gene was highly overexpressed with suppression of Wnt/β-catenin signaling in human liver tissues of NASH patents. The CXXC5 gene expression was analyzed using gene set enrichment analysis (GSEA) of microarray transcriptome data from human liver tissues of normal (*n* = 12) and NASH patents (*n* = 12) (GEO: GSE48452). CXXC5 overexpression and suppression of Wnt/β-catenin was also observed in the GSEA of microarray transcriptome data from human liver tissues of different phases of normal (*n* = 4) and Type II diabetes patient (*n* = 4) (GEO: GSE16415) **(****FIG. 23A****)**.

Referring now to **FIG. 22** and **23**, the expression of Wnt/β-catenin pathway target genes (also labelled, "Wnt activator" genes) such as *TCF7L2,* and *FOSL1* in liver tissues of NASH and Type II diabetes patients was significantly reduced; whereas, the expression of the fibrosis marker genes such as *MMP7, CD36, Col1a* was upregulated in NASH and Type II diabetes patients. **FIG. 22B** and **23B** shows heat map analyses of gene expression for the Wnt/β-catenin pathway target genes; normal (*n* = 4) and NASH (*n* = 4) subjects (dataset; GSE48452). The results disclosed herein demonstrate suppressive expressions of factors including *TCF7L1* in NASH patient tissues **(****FIG. 22B**, right panel). CXXC5 protein levels were also highly increased with reduction of β-catenin levels in liver tissues of mice having NASH induced by methionine-choline deficient (MCD)-diet **(****FIG. 22C****)** and mice having diabetes induced either by HFD or streptozotosin (STZ) **(****FIG. 23C**; upper, HFD-induced: lower, STZ-induced).

**Phenotype of MCD-induced NASH mice and HFD-induced Type II diabetes mice**. Referring now to **FIG. 24**, the pathological features of the MCD diet-induced NASH mice were confirmed by steato/fatty liver, inflammation (detected by PPARy and F4/80 markers), and fibrosis, apoptosis, and fatty acid oxidation. Referring now to **FIG. 25**, the pathological features of the high fat diet (HFD)-induced Type II diabetes mice were also confirmed by immunohistochemistry (IHC), high fasting glucose level, insulin tolerance, inflammation (as well as crown like structure (CRC) formation), glucose tolerance (GTT) and insulin tolerance (ITT), and various other factors involving lipogenesis and gluconeogenesis **(****FIG. 25E-25I****)**.

*CXXC4*, a structural and functional analog of CXXCS that can also function as a negative regulator of Wnt/β-catenin signaling, was not expressed in liver tissues of NASH and Type II patients **(****FIG. 22A-22B** and **23A-23B)**. In addition, mRNA level of CXXC4 was not changed by HFD although both mRNA and protein levels of CXXCS were highly upregulated in white adipocytes, brown adipocytes, and liver tissues of mice fed with HFD for 8 weeks **(****FIG. 26A-26C****)**. These results show specificity and involvement of CXXCS in NASH and diabetes. This provides a novel therapeutic strategy for treating metabolic diseases such as NASH and diabetes that exhibit overexpression of CXXC5, but not CXXC4.

To further define the role of CXXC5 in metabolic diseases, the effect of HFD in *Cxxc5*^{+/+} and *Cxxc5*^{*-*/*-*} mice were assessed. Referring now to **FIG. 27A-27E**, *CXXC5*^{*-*/*-*} mice fed with HFD did not induce obesity phenotypes as shown by observations of visceral or organ fats, glucose and insulin tolerance (GTT, ITT) as well as reduction of inflammation and various serum factors involving obesity and metabolic diseases phenotypes. *CXXC5*^{*-*/*-*} mice showed suppression of glucose tolerance (GTT), insulin tolerance (ITT) with reductions of body weight and fat accumulation in various organs including white adipocytes, brown adipocytes, mesenteric and prerienal tissues as well as liver **(****FIG. 27A-27C****)**. *CXXC5*^{*-*/*-*} mice did not increase the HFD-induced size increment of adipocyte cells without forming the crown like structures (CRC) representing inflammation status. Further, *CXXC5*^{*-*/*-*} mice suppressed fasting insulin level and showed reduction of plasma levels of glucose, total cholesterol, triglyceride, ALT, AST and ALP without changing Ca⁺ and Mg⁺ levels **(****FIG. 27E****)**.

Referring now to **FIG. 28**, *CXXC5*^{*-*/*-*} mice also suppressed the HFD-induced accumulations of adipokines (leptin and resistin) in serum. However, mRNA level of adiponectin was decreased **(****FIG. 28A****)**. *CXXC5*^{*-*/*-*} mice also suppressed the HFD-induced expressions of markers for the gluconeogenesis (G6pc, PEPCK, PCK1, Fbp1) and lipogenesis (SREBP1, FAS, SCD1, ACC) in white fats **(****FIG. 28B****)**. *CXXC5*^{*-*/*-*} mice further suppressed inflammation by reducing M1 (Tnfα, Tgf-β1, INFy, F4/80) and increasing M2 (Retnla, Pdcd1lg2) macrophage markers **(****FIG. 28C****)**. Moreover, *CXXC5*^{*-*/*-*} mice suppressed mRNA expression of PPARy and C/EBPα with activation of the Wnt/β-catenin signaling target genes including *Wisp1* and *Fosl1* **(****FIG. 28D****)**.

In part due to the overexpression of CXXCS observed in NASH patients and MCD-diet induced NASH mice **(****FIG. 22** and **24****)**, as well as the effects observed from knock out phenotypes of mice **(****FIG. 27****)**, CXXC5 overexpression in liver and/or adipocyte can be used as a potent biomarker for detecting metabolic diseases such as obesity, diabetes, and/or NASH in a subject.

**The CXXC5-DVL protein-protein interaction (PPI) as a target for development of 1^{st}-in-class drugs for treating NASH and/or Type II diabetes patients**.

CXXC5 overexpression in human liver or adipocyte tissues of obesity, diabetes, and/or NASH patients, and the effect HFD in *Cxxc5*^{*-*/*-*} mice disclosed herein provide that CXXCS can be a target for treating metabolic diseases. As disclosed herein, inhibition of the CXXCS-DVL interface can provide selective and/or specific activation of Wnt/β-catenin pathway in a subject having or diagnosed with at least one metabolic diseases including, but are not limited to, obesity, diabetes, and/or NASH. This is further supported by the fact that CXXC4, a CXXC5 analog which can also function as a negative regulator of Wnt/β-catenin pathway, was not overexpressed in NASH and/or Type II diabetes patients. In addition, targeting cytosolic CXXC5, which functions via the interaction with DVL, can provide additional benefits as this approach will likely reduce any undesirable side effects that can rise from targeting nuclear CXXC5. Studies have reported that nuclear CXXCS can activate or induce genes such as Flk-1 and/or myelin genes (Kim et al., 2014; Kim et al., 2016).

CXXC5 can function as a negative regulator of Wnt/β-catenin pathway by binding to DVL. A protein transduction domain fused DVL binding motif peptide (PTD-DBMP), which interferes with the CXXC5-DVL interaction were developed (Kim *et al.*, 2015). To identify small molecules that mimic the function of the PTD-DBMP and inhibit or reduce the activity of the CXXC5-DVL interface, 2,280 compounds were screened from chemical libraries (1,000 from ChemDiv and 1,280 from SigmaLOPAC) with an *in vitro* assay system that monitors the CXXC5-DVL interaction (Kim et al, 2016) **(****FIG. 29****)**.

Indirubin analog compounds (#8 and #12; **Table 3)** were repeatedly identified as CXXC5-DVL inhibitors and showed effectiveness in the activation of Wnt/β-catenin pathway using reporter assay. To obtain functionally improved compound(s), about 60 indirubin derivatives were newly synthesized by replacing the functional groups at the R₁ and R₂ sites of the indirubin backbone based on the structure of indribin-3'-oxim (130) (#12; **Table 3)**. Newly synthesized indirubin derivatives are described in **Tables 4-6** and their structures are shown **FIG. 30** **and** **FIG. 49**.

**Characterization of the indirubin analogs**. To select compounds that can control and/or treat metabolic diseases by the activation of the Wnt/β-catenin signaling through CXXCS-DVL PPI inhibition, following tests were performed; 1) *in vitro* CXXC5-DVL PPI inhibition assay; 2) cell-based assays monitoring the Wnt/β-catenin signaling through the pTOFPPLASH reporter system; and 3) an adipocyte differentiation assay using 3T3L1 pre-adipocyte cells.

Referring now to **FIG. 31**, about 42 representative indirubin analogs were tested their capabilities to inhibit (PTD-DBMP)-(PDZ-DVL) interaction *in vitro,* and their effects on activation of the Wnt/β-catenin reporter activity. Blue bars in **FIG. 31A** represents inhibition levels that were observed to be higher than that of indirubin-3'-oxime (I3O; a positive control) and red bars in **FIG. 31B** represents levels of the Wnt/β-catenin reporter activity that were observed to be higher than that of I3O. Several compounds (e.g., A2735, A2853, A2736, A3439, A3050, A3334, and A3441) were selected and tested further for dose-dependency **(****FIG. 31C****)**. Toxicity and solubility of the compounds were also tested.

Referring now to **FIG. 32**, about 25 compounds were selected and tested for inhibitory effects on adipocyte differentiation by using 3T3L1 pre-adipocyte cells. A3334 and A3051 which show significant anti-differentiation effects were selected for further analyses. Referring now to **FIG. 33A-33D**, PDZ-DVL binding of the several candidates (e.g., A3334, A3051, and 130) were further characterized using the *in vitro* (PTD-DBMP)-(PDZ-DVL) binding analyses. The results are as follows: A3334 IC₅₀=2.584 × 10⁻⁸, A3051 IC₅₀=7.708 × 10⁻⁹, Indirubin 3'-oxime (130) IC₅₀=9.890 × 10⁻⁸. After consideration of its solubility for formulation, A3334 (5-methoxyindirubin-3'-oxime) and A3051 (5, 6-dichloroindirubin-3'-methoxime) were selected for further investigation.

**Functional characterization of candidate compounds.** The efficacy of representative compounds (e.g., A3334, A3051, and I3O) were investigated in the improvement of metabolic diseases including obesity, diabetes, NASH.

**Anti-obesity effects of A3334, a small molecule CXXC5-DVL PPI inhibitor.** Referring now to **FIG. 34**, C57BL/6N mice were fed HFD for 16 weeks with or without oral administration of 25 mpk A3334 or Orlistat. Orlistat is an anti-obesity drug that is thought to act by limiting the absorption of fats from the diet (e.g., a lipase inhibitor). Referring now to **FIG. 34A-34B****,** the HFD-induced body weight gain and abdominal obesity were not observed in the HFD mice treated with A3334; these group of mice exhibited phenotypes that are similar to the mice fed with normal diet. In addition, the inhibitory effect of A3334 on weight gain was much more significant than the effects observed in the HFD mice treated with orlistat at identical concentration (25 mpk) for 16 weeks **(****FIG. 34B****)**. Treatment of A3334 significantly reduced the level of triglyceride and total cholesterol and increased the level of HDL-cholesterol in the HFD mice **(****FIG. 34C****)**. The glucose tolerance (GTT) and insulin tolerance (ITT) were also significantly improved by the A3334 treatment **(****FIG. 34D****)**.

Referring now to **FIG. 35**, The C57BL/6N mice were grown with HFD for 16 weeks with or without oral administration of 25 mpk of A3334, A3051, and I3O. The anti-obesity effect of A3334 observed were correlated with suppression of fatty liver and white adipocyte tissue in the HFD mice **(****FIG. 35A-35B****)**. The increase in adipocyte cell sizes and enhancement of inflammation (evaluated by crown like structures; blue; **FIG. 35B**) were suppressed by treatment with A3334 in the HFD mice. The A3051 and 130 treatments also showed reduction in adipocyte size and anti-inflammatory effects, but those effects were lower when compared to that of the A3334 treatment.

**No effect of A3334 on mice fed with normal diet.** To evaluate whether the A3334 treatment would have any effect on normal diet fed mice, C57BL/6N mice were fed normal diet for 16 weeks with or without oral administration of 25 mpk A3334. As shown in **FIG. 26A-26C**, normal diet did not induce CXXC5 upregulation in mice. Referring now to **FIG. 36**, the normal diet fed mice with or without A3334 treatment did not show any differences on size of fats **(****FIG. 36A-36B****)**, weight of organs **(****FIG. 36D****)**, and level of ALT and AST in blood serum (FIG. 36F). Also, no histological differences were observed between the normal diet fed mice and the normal diet fed mice with the A3334 treatment **(****FIG. 36E****)**.

**Anti-diabetic effects by A3334, a small molecule CXXC5-DVL PPI inhibitor.** Referring now to **FIG. 37A-37E**, C57BL/6N mice were fed HFD for 8 weeks to induce diabetes. Then, the mice were maintained on HFD and treated with either 25 mpk A3334 or 25 mpk rosiglitazone (RSG) once per day for next 5 days. Then, the mice were maintained HFD condition without any drug treatments (e.g., A3334 or RSG) for the next several weeks. Rosiglitazone (RSG) is a diabetes drug in thiazolidinedione (TZD) class which works as an insulin sensitizer functioning via binding PPAR in fat cells.

A3334 improved the HFD-induced major diabetes phenotypes (e.g., inflammation, insulin resistance, β-cell destruction with fast fasting glucose reduction, and long-term effect). A3334 also showed advantageous effects compared to RSG including suppression of the HFD-induced ALT/AST and GTT/ITT enhancement in blood, suppression of weight gain, as well as anti-inflammatory effects. Referring now to **FIG. 37A**, after the termination of drug treatment, the fasting glucose level was quickly increased in mice treated with RSG. However, in mice treated with A3334, the fasting glucose level was maintained at low level for several weeks. Further, the A3334 treatment suppressed the body weight gain induced by HFD; whereas, the RSG treatment showed increased body weight gain, a result consistent with known side effect of RSG (A, right panel; see e.g., Ahmadian et al., 2013. PPARγ signaling and metabolism: the good, the bad and the future. NAT. MED. 19, 557-566). The levels of ALT and AST in blood serum were increased in HFD-induced diabetes mice. The increased levels of ALT and AST were not evident in HFD mice treated with A3334. In contrast, the increased levels of ALT and AST were further enhanced by the RSG treatment **(****FIG. 37B****)**. A significant reduction of both GTT and ITT were observed with the A3334 treatment, but not with the RSG treatment, in the HFD-induced diabetes mice **(****FIG. 37C****)**. Referring now to **FIG. 37D-37E**, the treatment with A3334 suppressed overexpression of CXXC5, increased expressions of β-catenin, suppressed formation of crown like structures (CRC), and suppressed the expression of PPARy and macrophage markers (e.g., F4/80 and CD11b) in HFD-induced diabetes mice. In contrast, the treatment with RSG did not induce any of these effects.

Referring now to **FIG. 38A-38J**, C57BL/6N mice were induced diabetes by HFD for 8 weeks, and then A3334 (25 mpk), metformin (100 mpk) or sitagliptin (SITA) (50 mpk) was orally administered for 5 days once per a day starting at week 9 and at week 12. The mice were maintained at HFD condition throughout the experiment. The fasting glucose level was reduced similarly by treatment with all of these compounds at initial applications. A3334 exhibited long lasting effect in suppression of fast glucose level, and similar patterns of fasting glucose suppression were also observed after the second application of drugs at week 12 **(****FIG. 38A****)**. The levels of GTT/ITT **(****FIG. 38B****)**, total cholesterol **(****FIG. 38C****)**, fasting glucose level **(****FIG. 38D****)**, triglyceride **(****FIG. 38E****)**, FFAs **(****FIG. 38F****)** in serum was most significantly reduced by A3334, and their levels are almost equivalent to those fed with normal diet (Chow). In contrast, serum adiponectin level was increased by A3334 **(****FIG. 38G****)**. The effects of metformin and sitaglipitin in suppression of ITT/GTT, total cholesterol, glucose, TG, FFAs levels and increment of adiponection were weaker than those of A3333. The ALT and AST levels and Homeostatic Model Assessment for Insulin Resistance (HOMA-IR) analysis, which represents insulin resistance, were reduced by A3334. However, ALT and AST levels were increased by metformin **(****FIG. 38H-38I****)**. The levels of Ca⁺⁺ and Mg⁺⁺ in the serum were not significantly changed by the administration of either A3334 or other drugs **(****FIG. 38J-38K****)**.

Referring now to **FIG. 39A-39D**, C57BL/6N mice were induced diabetes by HFD for 8 weeks, and then A3334 (25 mpk), A3051 (25 mpk), 130 (25 mpk), metformin (100 mpk) or sitagliptin (SITA) (50 mpk) was orally adiministered once a day for 5 days starting at week 9 and at week 12 under the HFD condition. Referring now to **FIG. 39A**, the fasting glucose level was reduced similarly by treatment of all these compounds. By 5 days, A3334 administration revealed long lasting effect in the suppression of fast glucose level, and similar patterns of fasting glucose suppression patterns were shown by secondary application after 5 additional weeks. 130 and A3051 also reduced fasting glucose. However, effects of A3051 and 130 is similar to or little bit weaker than SITA. A3334 showed most significant and long last effects on fasting glucose control effect. Referring now to **FIG. 39B**, the levels of GTT and ITT in serum were most significantly reduced by A3334, and their levels are almost equivalent to those feed with normal diet (Chow) The insulin resistance monitored by fasting insulin level and HOMA-IR (Homeostatic Model Assessment for Insulin Resistance) analysis mostly resolved by A3334 administration **(****FIG. 39C-39D****)**.

**A3334 suppressed diabetes induced by multiple low-dose STZ-induced diabetes**. Referring now to **FIG. 40A-40G**, C57BL/6N mice were induced diabetes by HFD for 8 weeks, and induced diabetes by injection of 50 mpk streptozotosin (STZ) for 1 week. After 2 weeks, A3334 (25 mpk) or SITA (25 mpk) was applied for 4 more weeks, histological analyses of pancreas were done. Referring now to **FIG. 40A-40B**, the STZ-induced β-cell destruction islet of Langerhans was mostly protected with the A3334 treatment. The β-cells exhibited normal proliferative status as evaluated by histological analyses (estimation of islet area with insulin production with increment of β-catenin. Referring now to **FIG. 40C**, the fasting glucose level which was increased by STZ injection was more significantly lowered by A3334 compared to that by SITA. The body weight of mice in the treatment of A3334 were slightly increased in HFD+STZ mice which may indicate recovery of body weights which were lowered by diabetes **(****FIG. 40D****)**. The β-cell and α-cell mass monitored by insulin and glucagon, respectively, in IHC analyses of STZ-induced pancreas tissues were increased and decreased, respectively, by the A3334 treatment **(****FIG. 40E-40F****)**. The STZ-induced β-cells exhibited normal proliferative status after the A3334 treatment **(****FIG. 40G****)**.

**Effects of A3334, a small molecule CXXC5-DVL PPI inhibitor, on NASH mice model.** For a NASH study, eight-week-old wild-type male C57BL/6N mice were fed with the methionine-choline deficient (MCD) diet for 7 weeks, followed by treatment with vehicle, A3334, A3051, indirubin 3'-oxim (130) of (25 mg kg⁻¹) sitagliptin (25 mg kg⁻¹) for another 3 weeks via daily oral gavage. Referring now to **FIG. 41A-41C**, body weights as well as liver weights were significantly reduced in the MCD-dieted mice. However, liver weight considered together with body weight (liver Wt. /body Wt.) did not significantly change. The MCD-dieted mice were significantly induced the fatty liver phenotype, and the hepatosteatosis observed by H&E analyses was significantly abolished by A3334 co-treatment **(****FIG. 41C****)**. A3051, 130, and SITA also reduced hepatosteatosis, but revealed only marginal effects compared to A3334 **(****FIG. 41D****)**. The increased levels of ALT and AST induced by MCD-diets were mostly abolished by A3334 treatment **(****FIG. 41E-41F****)**. The increased level of CXXC5 and decreased level of Wnt/β-catenin signaling (shown by β-catenin detection) were confirmed in IHC analyses of liver tissues, and those were critically reversed with abolishment of steatosis **(****FIG. 41G****)**.

Referring now to **FIG. 42A-42G**, eight-week-old wild-type male C57BL/6N mice were fed with the methionine-choline deficient (MCD) diet for 7 weeks, followed by treatment with vehicle, A3334 (25 mg kg⁻¹), A3051 (25 mg kg⁻¹), 130 (25 mg kg⁻¹), and sitagliptin (25 mg kg⁻¹) for another 3 weeks via daily oral gavage. The FFAs and TG increments in serum by MCD-diet were mostly abolished with A3334 treatment **(****FIG. 42A-42B****)**. The pathological features of NASH in liver which were investigated by measurement of mRNA levels of pro-inflammation **(****FIG. 42C****)**, FA oxidation **(****FIG. 42D****)**, apoptosis **(****FIG. 42E****)**, and fibrosis **(****FIG. 42F****)** markers were significantly reduced by A3334. The improvement of the MCD-diet induced fibrosis by A3334 was also shown by several different histological analyses **(****FIG. 42G****)**.

**A3334 improved metabolic disease markers induced by HFD**. C57BL/6N mice were induced diabetes by HFD for 8 weeks, and then treated with A3334 (25 mpk) once per day for 5 days starting at week 9 and at week 12 under HFD condition. Animals were sacrificed at week 16. Referring now to **FIG. 43A-43C**, the H&E and IHC analyses of white adipose tissues (WAT) showed that A3334 suppressed HFD-induced increase in cell size and the expression of PPARγ and C/EBPα, which is involved in adipocyte differentiation and obesity. HFD-induced inflammation as indicated by crown like structures (CLCs) as shown by measurement of F4/80 and CD11 was also suppressed by A3334. The ALT and AST levels were also lowered by A3334 **(****FIG. 43D****)**. The serum of mice fed HFD with or without A3334 were blotted for the protein chips including antibodies for various metabolic disease factors **(****FIG. 43E****)**. The mRNA levels of indicated proteins were also reduced serums **(****FIG. 43F****)** and white adipose tissues **(****FIG. 43G****)**.

**A3334 improved diabetic phenotypes induced by HFD**. Referring now to **FIG. 44A-44G**, C57BL/6N mice were induced diabetes by HFD for 8 weeks, and then treated with A3334 (25 mpk) or SITA (50 mpk) once per day for 5 days starting at week 9 and at week 12 under HFD condition. Animals were sacrificed at week 16. The HFD-induced fatty liver and steatosis of liver analyzed by H&E and Oil red O (ORO) staining were totally abolished by the A3334 treatment **(****FIG. 44A**)**.** The cell size increment and CLS formation of adipocytes tissues were inhibited by the A3334 treatment **(****FIG. 44B****)**. The transition of brown adipocytes to white adipocytes were suppressed by the A3334 treatment together with recovery of the levels of UCP1 and GLUT4 **(****FIG. 44C-44D****)**. The increased mRNA levels of the lipogenesis and inflammation markers observed in white adipocyte tissues and increased mRNA levels of the lipogenesis and gluconeogenesis markers observed in liver tissues of HFD mice were significantly lowered by the A3334 treatment **(****FIG. 44E-44F****)**. In contrast, mRNA levels of the mitochondria biogenesis markers detected in brown adipocyte tissues of HFD mice were all increased by the A3334 treatment **(****FIG. 44G****)**.

Referring now to **FIG. 45A-45F**, C57BL/6N mice were induced diabetes by HFD for 8 weeks, and then treated with A3334 (25 mpk) or RSG (25 mpk) once per day for 5 days starting at week 8 under HFD condition. Animals were sacrificed at week 11. The liver steatosis induced by HFD was suppressed by A3334, but not by rosiglitazone (RSG; 25 mpk) **(****FIG. 45A****)**. The insulin and total cholesterol levels increased in serum of diabetes mice induced by HFD were abolished by A3334 **(****FIG. 45B-45C****)**. The mRNA levels of the lipogenesis markers, which were increased in liver tissues of mice treated with A3334, suppressed by A3334, but those levels were largely increased by RSG **(****FIG. 45D****)**. The mRNA level of the Wnt/β-catenin signaling target genes including *Dvl1, Wisp1* and *Fost1* were increased by A3334, but not by RSG. However, PPARy mRNA level was not increased by A3334 while it was increased by RSG **(****FIG. 45E****)**. The mRNA levels of M1 and M2 macrophage markers, which represent pro- and anti-inflammatory effects on inflammation were decreased and increased by A3334, respectively. However, RSG did not result in these effects **(****FIG. 45F****)**.

Referring now to **FIG. 46A-46F**, C57BL/6N mice were induced diabetes by HFD for 8 weeks, and then treated with A3334 (25 mpk) or SITA (50 mpk) once per day for 5 days starting at week 9 and at week 12 under HFD condition. Animals were sacrificed at week 16. The fatty liver and liver steatosis induced by HFD was suppressed by A3334 while SITA exhibited only a marginal effect **(****FIG. 46A****)**. The serum TG, FFAs, and ALT/ AST levels increased by HFD-mice diabetes model were significantly lowered by A3334, but these were weakly reduced by SITA even at a higher concentration (FIG. 46B-46C). In liver tissues of the HFD-induced diabetes model system, mRNA levels of Wnt/β-catenin pathway target genes were significantly increased by A3334 treatment, but not by SITA **(****FIG. 46D****)**. mRNA levels of lipogenesis and gluconeogenesis markers increased by HFD were reduced by both A3334 and SITA, but A3334 showed better effectiveness **(****FIG. 46E-46F****)**.

Referring now to **FIG. 47A-47B**, treatment of A3334 also reduces fasting glucose levels in a distinct diabetes mice genetic model (db/db). See e.g., Wang et al., Diabetologia (2002) 45: 1263-1273.

Considering the roles of the Wnt/β-catenin signaling in stem cell activation and the involvement of its aberrant activation in human cancers, drugs targeting Wnt/β-catenin pathway can face potential side effects including cancer. However, these side effects will likely be avoided as the instant approach provides methods of inhibiting or reducing the CXXC5-mediated negative feedback loop rather than targeting direct activation of Wnt/β-catenin pathway. Potential problems of developing cancer by direct activation of Wnt/β-catenin signaling may not be a critical issue when using the methods disclosed herein. This was validated by absence of any cancerous phenotype in various organs of *CXXC5*^{*-*/*-*} mice which were grown more than 2 years. In addition, topical application of the peptide(s) interfering the CXXC5-DVL interface onto the skin of the mice did not result in any aberrant phenotypes (data not shown). Further, the addition of these peptide(s) did not have any effect on transformation or the DMBA/TPA-induced skin carcinogenesis (data not shown). The approach of targeting a negative feedback regulation in a drug discovery is also supported by the development of drugs targeting sclerostin (encoded by *SOST* gene), another negative feedback regulator of the Wnt/β-catenin pathway that functions by binding to LGPS/6 co-receptor.

### Emulsion Solution

To determine emulsion solution, solubility of A3334, for example, is measured in the several oil and surfactants. A3334 is mixed with oil or surfactants (1ml) and vortexed for 30 min. The equilibrium state was reached by shaking for 72 hours at 37°C with 50 rpm speed in an incubator, then centrifuged at 16,100 × g for 5 min. The supernatant was diluted with methanol and analyzed by LC/MS/MS (see e.g., **Table 7**).

'Waters xevo TQ MS-ACQUITY UPLC System' is used as an analyses equipment. ACQUITY UPLC^{®} BEH(C18, 1.7 µm, 2.1×50 mm) column was used for analyses at room temperature. Mobile phase is used after filtration of deionized distilled water including 0.1% formic acid and acetonitrile including 0.1% formic acid (30 : 70, v/v) with 0.2 µl membrane filter. The flow rate is 0.25 ml/min and it is analyzed by injection of 0.5 µl sample. There are LC/MS/MS conditions for Decursin analysis; Cone 38 V, Collison 32 V. Precursor ion and daughter ion are monitored 329 and 229 m/z, respectively.

**Table 7. Solubility of A3334 in various type of oil or surfactants**

| Type | Type | Solubility (mg/ml) |
|---|---|---|
| Oil | Olive oil | 0.83 |
| | Sunflower oil | 1.35 |
| | KOLLIPHOR EL | 5.4 |
| Surfactants | Tween 20 | 4.58 |
| | Tween 80 | 4.92 |
| Co-Surfactant | Transcutol P | 1.15 |
| | Propylene glycol | 0.25 |
| | PEG 400 | 3.1 |

As shown **FIG. 59A** and **Table 2**, compositions using KOLLIPHOR^{®} EL, Tween 20, Tween 80, PEG 400 showed highest solubility among the emulsified solutions. Above mentioned KOLLIPHOR^{®} EL, Tween 20 or Tween 80 and PEG 400 are known to be harmless and safe. These emulsions can be used for human including application onto skin application.

### Ternary diagram analysis

Based on solubility experiment results, KOLLIPHOR^{®} EL was selected as an oil phase for emulsions and mixture of Tween 80 and PEG 400 are selected as surfactants. To confirm area of emulsion forming range, ternary phase diagram was completed by using H₂O titration method the room temperature. Surfactant mixtures were prepared by mixing surfactant Tween 80 and PEG 400 at each 1:1, 2:1 and 1:2 ratio. And, emulsion solution was made by mixing surfactant at a various ratio (0.5:9.5, 1:9. 2:8, 3:7, 4:6, 5:5, 6:4, 7:3, 8:2, 9:1, 9.5:0.5) with oil phase, KOLLIPHOR^{®} EL. While stirring above mentioned emulsion solution and loading water with a 1 ml/min rate of speed, area maintaining uniformly mixed state observed with naked eye is marked in the phase diagram and above ternary diagrams are presented **FIG. 59B****,** **59C,** and **59D****.**

**FIG. 59B** shows a ternary diagram for emulsion solution made of surfactant mixed 2:1 ratio (Tween 80: PEG 400). **FIG. 59C** shows a ternary diagram for emulsion solution made of surfactant mixed 1:1 ratio (Tween 80 : PEG 400). **FIG. 59D** shows ternary diagram for emulsion solution made of surfactant mixed 1:2 ratio (Tween 80 : PEG 400). The area forming stable emulsion are represented by red dots.

Referring now to **FIG. 59A-59D**, emulsion solution using surfactants mixed Tween 80 and PEG 400 at a 1:2 ratio showed most wide range of numbers, therefore, this ratio was selected. Then, for final selection of ratio between above mentioned surfactants and emulsion, emulsion solution, droplet size, viscosity, zeta potential of emulsion solution produced with various ratio are compared.

Emulsion solution is manufactured with surfactant, polyethylene glycol and oil selected in various ratios, 10:30:60 or 90:3:7 (Table 7). To manufacture emulsion including active ingredient on the present disclosure, emulsion was manufactured by adding active ingredient (e.g., A3334) to make final 10% weight concentration for the total weight of entire composition and stirred overnight. (For example, if weight of total composition is 100 g, then 20 g of A3334 was mixed with above mentioned emulsion solution, 80 g)

The solubilizer Cyclodextrin can be included in the above mentioned composition. Indirubin derivative (active ingredient) (100 part by weight) with cyclodextrin (100 part by weight) was used in this experiment to increase solubilization of indirubin derivative, A3334.

**Table 8. Formulations F1-F9**

| class | Emulsion solution(Kollipore^{®} EL:Tween 80:PEG400) Part by weight | Solubility (mg/ml) | Droplet size (nm) | Viscosity | Zeta-potential (mV) |
|---|---|---|---|---|---|
| F1 | 10:30:60 | 2.99 | 1525.7 | 23.33 | -5.14 |
| F2 | 20:26:54 | 3.58 | 921.3 | 4446 | -6.87 |
| F3 | 30:23:47 | 3.81 | 624.9 | 59.12 | -7.23 |
| F4 | 40:20:40 | 4.11 | 479.4 | 65.07 | -6.94 |
| F5 | 50:16:34 | 5.67 | 332.3 | 72.72 | -7.99 |
| F6 | 60:13:27 | 6.86 | 264.9 | 88.85 | -9.72 |
| F7 | 70:10:20 | 7.59 | 183.8 | 91.39 | -10.83 |
| F8 | 80:6:14 | 7.69 | 41.5 | 98.3 | -20.38 |
| F9 | 90:3:7 | 6.88 | 29.3 | 99,52 | -20.99 |

Emulsion solution manufactured reveals as opaque red colored as seen by naked eyes. Above mentioned solubility, droplet size, viscosity, zeta potential for above mentioned emulsion solutions are analyzed and presented in **Table 8**.

As shown on Table 8, droplet size decreased as oil contents increased and it is identified that all of them have appropriate physical properties to use for pharmaceutical or cosmetic compositions. However, Formulation F8 exhibited the most desirable size, viscosity, zeta-potential and solubility.

### Assessment for safety evaluation of the formulation for the emulsion solution

To confirm stability of manufactured emulsion solution, F8, F10-F12 emulsion solutions were made with a composition in **Table 9**. Stability of above mentioned emulsion solutions are measured based on relative solubility (%) change at the low temperature (4°C) and room temperature (25°C). Relative solubility (%) are recorded up to 3 months and presented in **FIG. 60A** and **FIG. 60B**.

**FIG. 60A** represents a graph showing relative solubility (%) change of F8, F10~12 emulsion solutions at room temperature (25 °C). **FIG. 60B** is a graph showing relative solubility (%) change of F8, F10~12 emulsion solution at a low temperature (4 °C)

As shown in the **FIG. 60A**, **FIG. 60B** and **Table 9**, after leaving 3 months at a low temperature and room temperature, changes in relative solubility were observed. For F8, the change in solubility (%) was minimal within 3 months from manufacture at 4 °C or 25 °C. In contrast, for F10, F11, and F12, there was over 40% solubility change within the same period. Specifically, solubility dropped rapidly over 80% at the low temperature condition.

### Assessment of stability of emulsion solution

F8 and F10-F12 emulsion formulations were manufactured with composition of above mentioned **Table 9** to confirm whether activity of active ingredient of indirubin derivative, A3334, is remained stable in the emulsion formulation. Relative Wnt reporter activity (%) within 3 months from manufacture can be seen **FIG. 60C** and **FIG. 60D**.

As shown in FIG. 60C and FIG. 60D, relative Wnt reporter activity (%)were observed after leaving emulsion solution for 3 months at the low temperature and room temperature. Wnt reporter activity (%) change difference was minimal in case of emulsion solution, F8. Whereas, the Wnt reporter activity (%) was decreased over 40% in case of emulsion solutions, F11, F10, and F12. Moreover, Wnt reporter activity (%) was dropped rapidly over 80% at low temperature.

**Table 9. Formulations F8, F10, F11, and F12.**

| Class | Emulsion solution | | | Active ingredient Indirubin derivative of embodiment 2 | Solubilizer (2-hydroxy-cyclodextrin) | Aqua state |
|---|---|---|---|---|---|---|
| DMSO Control | Oil | surfactants | Polyethylene glycol | 12.5g | 12.5g | Distilled water 100g |
| F8 | Kollipore^{®} EL 80 g | Tween 80 6.7 g | PEG400 13.3 g | 12.5g | 12.5g | Distilled water 100g |
| F10 | - | - | | 12.5g | 12.5g | Distilled water 100g |
| F11 | - | Tween 80 100 g | - | 12.5g | 12.5g | Distilled water 100g |
| F12 | Kollipore EL 100 g | - | - | 12.5g | 12.5g | Distilled water 100g |

Emulsions disclosed herein can include at least one agent comprising at least one compound according to any one of the first to the twenty fifth embodiments and/or at least one composition according to the twenty sixth embodiment. For the above mentioned emulsions, the mixing weight ratio of oil : surfactants : polyethylene glycol may be 0.3-30 : 1 : 2-2.5, and more preferably 10-20 : 1 : 2-2.5. The composition of the emulsion formulation can be determined by a pseudo three-phase diagram created in accordance with conventional methods for determining the composition of the emulsion system. Specifically, after thoroughly mixing in an oil phase (polyethoxylated castor oil (Kolliphor^{®}EL)) and a surfactant (for example, a mixture of tween 80 and polyethylene glycol) in different weight ratios in a certain ratio range, a pseudo three-phase diagram can be created by adding water to each percentage of the oil and the surfactant mixture and marking the points corresponding to the emulsion forming regions. The emulsion region can be determined in the created pseudo three-phase diagram, and a specific composition among the compositions contained in the region can be selected to determine the composition of the emulsion formulation to dissolve the active ingredient. It is desirable that active ingredient be present in 1-20 weight % of the total weight of the composition. The active ingredient can include at least one compound according to any one of the first to the twenty fifth embodiments and/or at least one composition according to the twenty sixth embodiment.

Above mentioned surfactant is a twin-type nonionic surfactant and serves as an auxiliary solubilizer. In pharmaceuticals, it is used as an emulsifying agent or wetting agent in oral or parenteral formulations, and is also used as an additive in cosmetics and foods. It is also used as a substance for the inhibition of p-glycoprotein to increase the bioavailability of the drug. Tween series of surfactants include polyoxyethylene sorbitan monolaurate (Tween 20), polyoxyethylene sorbitan monopalmitate (Tween 40), polyoxyethylene sorbitan monostearate (Tween 60) and polyoxyethylene sorbitan oleate (Tween 80). However, the present disclosure is not limited thereto.

The polyethylene glycol is an amphoteric polymer which have hydrophilicity and hydrophobicity, and the polyethylene glycol is a liquid in the case of a low molecular weight but becomes a solid with an increase in molecular weight. The polyethylene glycol may be selected from the group consisting of PEG 150, 300, 400, 1000, 6000, 8000, 10000, 20000, 30000 and 40000. Here, the PEG 300 refers to polyethylene glycol having a molecular weight of 300, and the polyethylene glycol having a molecular weight exceeding 10,000 may be also referred to as polyethylene oxide (PEO). Of these, PEG 400 exists in the form of a liquid and is frequently used for the solubilization of various insoluble drugs.

As disclosed herein, emulsion formulations can include cyclodextrin for the higher solubilization. Cyclodextrin can be included with 100-1000 part by weight based on active ingredient 100 part by weight existing in the composition.

Above mentioned emulsion formulation is stable formulation that there is no change of Wnt activity and solubility in the distilled water at a temperature of 25 °C or 4 °C. Therefore, it was confirmed that activity of the active ingredient can be maintained for a long period of time, and the absorption into the living body can be effectively improved.

Water was added to the emulsion formulation of the present disclosure and a microphotograph was taken. As a result, the emulsion of the present disclosure was completely dissolved to form an emulsion formulation (F8) in a solution state. Further, as a result of observing the stability of the emulsion of the present disclosure, it was found that the droplet was in a spherical shape, the average diameter was 20 to 1,500 nm and the preferable diameter was 30 to 50 nm to form nanosized droplets, indicating a narrow range of size distribution. Also, it is confirmed that there is no change in activity and solubility of active ingredients with maintenance of solubility for 3 months at room temperature.

While the novel technology has been illustrated and described in detail in the figures and foregoing description, the same is to be considered as illustrative and not restrictive in character, it being understood that only the preferred embodiments have been shown and described and that the scope of protection is defined by the claims.

## Claims

1. A composition comprising at least one compound and/or a pharmaceutically acceptable hydrate, salt, or carrier thereof, wherein the compound is: 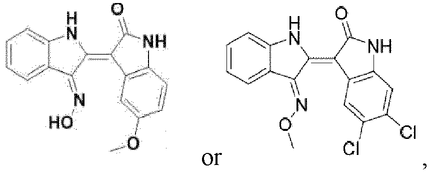 for use in a method of treating or preventing a metabolic disease in a subject.

2. The composition for use according to claim 1 wherein the subject has upregulated expression of CXXC5.

3. The composition for use according to claims 1 or 2, wherein the subject exhibits an abnormal lipid profile and/or blood glucose levels.

4. The composition for use according to any one of the claims 1 to 3, wherein the metabolic disease is obesity, diabetes, non-alcoholic fatty liver disease (NAFLD), and/or non-alcoholic steatohepatitis (NASH).

5. The composition for use according to any one of claims 1 to 4, wherein the subject is a human or an animal.

6. The composition for use according to any one of claims 1 to 5, wherein the composition is an oral preparation or intravenous preparation.

## Patentansprüche

1. Zusammensetzung, umfassend mindestens eine Verbindung und/oder ein pharmazeutisch unbedenkliches Hydrat, ein pharmazeutisch unbedenkliches Salz oder einen pharmazeutisch unbedenklichen Träger davon, wobei es sich bei der Verbindung um: 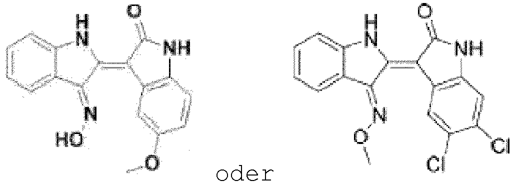 handelt,
zur Verwendung bei einem Verfahren zur Behandlung oder Prävention einer Stoffwechselerkrankung bei einem Individuum.

2. Zusammensetzung zur Verwendung nach Anspruch 1, wobei das Individuum eine heraufregulierte Expression von CXXC5 aufweist.

3. Zusammensetzung zur Verwendung nach Anspruch 1 oder 2, wobei das Individuum ein abnormales Lipidprofil und/oder abnormale Blutzuckerspiegel zeigt.

4. Zusammensetzung zur Verwendung nach einem der Ansprüche 1 bis 3, wobei es sich bei der Stoffwechselerkrankung um Obesitas, Diabetes, nichtalkoholische Fettlebererkrankung (NAFLD) und/oder nichtalkoholische Steatohepatitis (NASH) handelt.

5. Zusammensetzung zur Verwendung nach einem der Ansprüche 1 bis 4, wobei es sich bei dem Individuum um einen Menschen oder ein Tier handelt.

6. Zusammensetzung zur Verwendung nach einem der Ansprüche 1 bis 5, wobei es sich bei der Zusammensetzung um eine orale Zubereitung oder intravenöse Zubereitung handelt.

## Revendications

1. Composition comprenant au moins un composé et/ou un hydrate, un sel ou un support pharmaceutiquement acceptable correspondant, le composé étant : 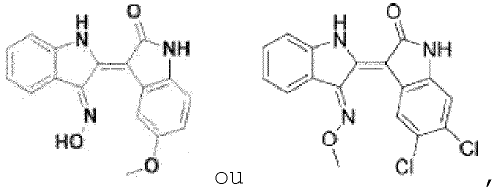 pour une utilisation dans un procédé de traitement ou de prévention d'une maladie métabolique chez un sujet.

2. Composition pour une utilisation selon la revendication 1, le sujet ayant une expression régulée à la hausse de CXXC5.

3. Composition pour une utilisation selon les revendications 1 ou 2, le sujet présentant un profil lipidique anormal et/ou des taux de glucose sanguin anormaux.

4. Composition pour une utilisation selon l'une quelconque des revendications 1 à 3, la maladie métabolique étant l'obésité, le diabète, une maladie du foie gras non alcoolique (NAFLD) et/ou une stéatohépatite non alcoolique (NASH).

5. Composition pour une utilisation selon l'une quelconque des revendications 1 à 4, le sujet étant un humain ou un animal.

6. Composition pour une utilisation selon l'une quelconque des revendications 1 à 5, la composition étant une préparation orale ou une préparation intraveineuse.
